# EUROPEAN PATENT SPECIFICATION

(11) **EP 1 385 844 B1**
(45) Date of publication and mention of the grant of the patent: **05.07.2006**
(21) Application number: 02720195.3
(22) Date of filing: 03.04.2002
(51) Int. Cl.: C07D 413/14, A61K 31/422, A61P 31/04, C07D 417/14

(54) **OXAZOLIDINONES CONTAINING A SULFONIMID GROUP AS ANTIBIOTICS**
EINE SULFONIMID-GRUPPE ENTHALTENDE OXAZOLIDINONE ALS ANTIBIOTIKA
OXAZOLIDINONES CONTENANT UN GROUPE SULFONIMIDE EN TANT QU'ANTIBIOTIQUE

(30) Priority: 07.04.2001 GB 0108765; 25.10.2001 US 330588 P
(43) Date of publication of application: 04.02.2004
(73) Proprietor: AstraZeneca AB, 151 85 (SE)
(72) Inventor: BETTS, Michael, John, Macclesfield, Cheshire SK10 4TG (GB); SWAIN, Michael, Lingard, Macclesfield, Cheshire SK10 4TG (GB); HALES, Neil, James, Waltham, MA 02451 (US); HUYNH, Hoan, Khai, Waltham, MA 02451 (US)
(74) Representative: Phillips, Neil Godfrey Alasdair
(86) International application number: PCT/GB2002/001644
(87) International publication number: WO 2002/081470

(56) References cited:
- WO-A-95/07271
- WO-A-96/15130
- WO-A-97/09328
- WO-A-97/37980
- WO-A-98/54161
- DE-A- 19 707 628
- GREGORY W A ET AL: "ANTIBACTERIALS. SYNTHESIS AND STRUCTURE-ACTIVITY STUDIES OF 3-ARYL-2-OXOOXAZOLIDINES. I THE B GROUP" JOURNAL OF MEDICINAL CHEMISTRY, AMERICAN CHEMICAL SOCIETY. WASHINGTON, US, vol. 32, no. 8, 1 August 1989 (1989-08-01), pages 1673-1681, XP000573960 ISSN: 0022-2623 cited in the application

## Description

The present invention relates to antibiotic compounds and in particular to antibiotic compounds containing a substituted oxazolidinone ring. This invention further relates to processes for their preparation, to intermediates useful in their preparation, to their use as therapeutic agents and to pharmaceutical compositions containing them.

The international microbiological community continues to express serious concern that the evolution of antibiotic resistance could result in strains against which currently available antibacterial agents will be ineffective. In general, bacterial pathogens may be classified as either Gram-positive or Gram-negative pathogens. Antibiotic compounds with effective activity against both Gram-positive and Gram-negative pathogens are generally regarded as having a broad spectrum of activity. The compounds of the present invention are regarded as effective against both Gram-positive and certain Gram-negative pathogens.

Gram-positive pathogens, for example Staphylococci, Enterococci, and Streptococci are particularly important because of the development of resistant strains which are both difficult to treat and difficult to eradicate from the hospital environment once established. Examples of such strains are methicillin resistant staphylococcus (MRSA), methicillin resistant coagulase negative staphylococci (MRCNS), penicillin resistant Streptococcus pneumoniae and multiply resistant Enterococcus faecium.

The major clinically effective antibiotic for treatment of such resistant Gram-positive pathogens is yancomycin. Vancomycin is a glycopeptide and is associated with nephrotoxicity and ototoxicity. Furthermore, and most importantly, antibacterial resistance to vancomycin and other glycopeptides is also appearing. This resistance is increasing at a steady rate rendering these agents less and less effective in the treatment of Gram-positive pathogens. There is also now increasing resistance appearing towards agents such as β-lactams, quinolones and macrolides used for the treatment of upper respiratory tract infections, also caused by certain Gram negative strains including H.influenzae and M.catarrhalis.

Certain antibacterial compounds containing an oxazolidinone ring have been described in the art (for example, Walter A. Gregory et al in J.Med.Chem. 1990, 33, 2569-2578 and Chung-Ho Park et al in J.Med.Chem. 1992, 35, 1156-1165). Such antibacterial oxazolidinone compounds with a 5-acetamidomethyl sidechain may be subject to mammalian peptidase metabolism. Furthermore, bacterial resistance to known antibacterial agents may develop, for example, by (i) the evolution of active binding sites in the bacteria rendering a previously active pharmacophore less effective or redundant, (ii) the evolution of means to chemically deactivate a given pharmacophore and/or (iii) the development and/or up-regulation of efflux mechanisms. Therefore, there remains an ongoing need to find new antibacterial agents with a favourable pharmacological profile, in particular for compounds containing new pharmacophores.

We have discovered a new class of antibiotic compounds containing an aryl substituted oxazolidinone ring in which the aryl ring is itself substituted by certain novel sulfilimine and sulfoximine-containing rings. These compounds have useful activity against Gram-positive pathogens including MRSA and MRCNS and, in particular, against various strains exhibiting resistance to vancomycin and against E. faecium strains resistant to both aminoglycosides and clinically used β-lactams, but also to fastidious Gram negative strains such as H.influenzae, M.catarrhalis, mycoplasma spp. and chlamydial strains.

Accordingly the present invention provides a compound of the formula (IA), or a pharmaceutically-acceptable salt, or in-vivo hydrolysable ester thereof 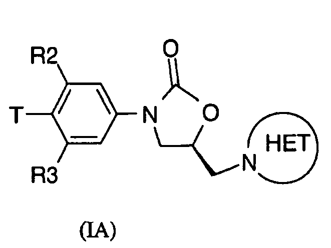 wherein HET is 1,2,3-triazole, 1,2,4-triazole or tetrazole; or HET is a di-hydro version of pyrimidine, pyridazine, pyrazine, 1,2,3-triazine, 1,2,4-triazine, 1,3,5-triazine and pyridine;
wherein HET is optionally substituted on any available C atom, other than a C atom adjacent to the linking N atom, by a substituent selected from (1-4C)alkyl, (2-4C)alkenyl, (3-6C)cycloalkyl, amino, (1-4C)alkylamino, di-(1-4C)alkylamino, (1-4C)alkylthio, (1-4C)alkoxy, (1-4C)alkoxycarbonyl, halogen, cyano and trifluoromethyl and/or on an available nitrogen atom (provided that the ring is not thereby quatemised) by (1-4C)alkyl; and wherein at each occurrence of alkyl, alkenyl and cycloalkyl HET substituents, each is optionally substituted with one or more F, Cl or CN;
R² and R³ are independently hydrogen or fluoro; and
T is selected from (TA1), (TA2) and (TB1) to (TB3), wherein (TA1), (TA2) and (TB1) to (TB3);
(TA) T is selected from the following groups (TA1) and (TA2):- 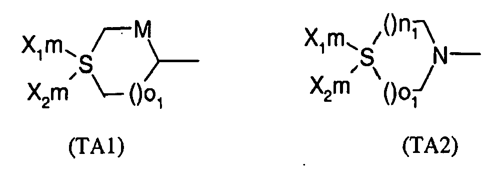 wherein :
in (TA1), ()o₁ is 0 or 1 and represents a chain of carbon atoms (optionally substituted as defined for AR1) of length o₁ and M is a bond joining the adjacent carbon atoms, or M represents one or two carbon atoms, and defines a 4- to 7-membered monocyclic ring, which ring may optionally have one of
(i) one double bond between any two ring carbon atoms; or
(ii) a C1-C3 bridge connecting any two appropriate, non-adjacent ring carbon atoms, which bridge may optionally contain one heteroatom selected from oxygen or >NRc; or
(iii) a C2-C5 cyclic moiety including a ring carbon atom to define a spiro C2-C5 ring system, which ring may optionally contain one heteroatom selected from oxygen or >NRc; or
(iv) a C1-C4 bridge connecting adjacent carbon atoms to define a fused ring, wherein a C2-C4 bridge may optionally contain one heteroatom selected from oxygen or >NRc; wherein Rc is as defined hereinafter;
wherein in (TA2), ()n₁ and ()o₁ are independently 0, 1or 2 and represent chains of carbon atoms (optionally substituted as defined for AR1) of length n₁ and o₁ respectively, and define a 4- to 8-membered monocyclic ring, which ring may optionally have one of
(i) a C1-C3 bridge connecting any two appropriate, non-adjacent ring carbon atoms, which bridge contains one heteroatom selected from oxygen or >NRc; or
(ii) a C2-C5 cyclic moiety including a ring carbon atom to define a spiro C2-C5 ring system, which ring may optionally contain one heteroatom selected from oxygen or >NRc; or
(iii) a C1-C4 bridge connecting adjacent carbon atoms to define a fused ring, wherein a C2-C4 bridge may optionally contain one heteroatom selected from oxygen or >NRc; wherein Rc is as defined hereinafter; and
wherein in (TA1) and (TA2), X₁ₘ and X₂ₘ taken together represent R₂ₛ-(E)ₘₛ-N=; or X₁ₘ is O= and X₂ₘ is R₂ₛ-(E)ₘₛ-N-, and vice versa;
wherein E is an electron withdrawing group selected from -SO₂-, -CO-, -O-CO-, -CO-O-, -CS-, -CON(Rₛ)-, -SO₂N(Rₛ)-, or E may represent a group of the formula R₃ₛ-C(=N-O-R₃ₛ)-C(=O)-, wherein R₃ₛ is H or as defined in R₂ₛ at (i) below;
or, when E is -CON(Rₛ)- or -SO₂N(Rₛ)-, R₂ₛ and Rₛ may link together to form a carbon chain which defines a 5- or 6-membered saturated, unsaturated or partially unsaturated ring linked via the N atom in E, which ring is optionally further substituted by an oxo substituent, and
which ring may be optionally fused with a phenyl group to form a benzo-fused system,
wherein the phenyl group is optionally substituted by up to three substituents independently selected from halo, cyano, (1-4C)alkyl and (1-4C)alkoxy;
msis 0 or 1;
R₂ₛ and Rₛ are independently selected from :
(i) hydrogen (except where E is -SO₂-or -O-CO-), or
   (1-6C)alkyl {optionally substituted by one or more (1-4C)alkanoyl groups (including geminal disubstitution) and/or optionally monosubstituted by cyano, cyano-imino, (1-4C)alkoxy, trifluoromethyl, (1-4C)alkoxycarbonyl, phenyl (optionally substituted as defined for AR1 herein), optionally substituted heteroaryl group of the formula AR2, AR2a, AR2b, AR3, AR3a, AR3b, AR4, AR4a or CY all as defined (and optionally substituted as defined) herein, (1-4C)alkylS(O)_{q}- (q is 0, 1 or 2); and/or (with the proviso that where R₂ₛ is -SO₂ or -O-CO-not on the first carbon atom of the (1-6C) alkyl chain) optionally substituted by one or more groups (including geminal disubstitution) each independently selected from hydroxy and fluoro, and/or optionally further substituted, by no more than one of each of, oxo, -NRvRw [wherein Rv is hydrogen or (1-4C)alkyl; Rw is hydrogen or (1-4C)alkyl], (1-6C)alkanoylamino, (1-4C)alkoxycarbonylamino, N-(1-4C)alkyl-N-(1-6C)alkanoylamino, (1-4C)alkylS(O)ₚNH- or (1-4C)alkylS(O)ₚ₋((1-4C)alkyl)N- (p is 1 or 2)}; or
(ii) an optionally substituted aryl or optionally substituted heteroaryl group of the formula AR1, AR2, AR2a, AR2b, AR3, AR3a, AR3b, AR4, AR4a or CY all as defined (and optionally substituted as defined) herein;
   or (where ms is 0 only);
(iii) cyano, -CO-NRvRw, -CO-NRv Rw', -SO₂-NRvRw, -SO₂-NRv Rw' [wherein Rv is hydrogen or (1-4C)alkyl; Rw is hydrogen or (1-4C)alkyl; Rw' is phenyl (optionally substituted as defined for AR1 herein), or a heteroaryl group selected from AR2, AR2a, AR2b, AR3, AR3a, AR3b, AR4, AR4a (optionally substituted as defined herein)],
   (1-4C)alkoxycarbonyl, trifluoromethyl, ethenyl, 2-(1-4C)alkylethenyl, 2-cyanoethenyl, 2-cyano-2-((1-4C)alkyl)ethenyl, 2-nitroethenyl,2-nitro-2-((1-4C)alkyl)ethenyl, 2-((1-4C)alkylaminocarbonyl)ethenyl, 2-((1-4C)alkoxycarbonyl)ethenyl, 2-(AR1)ethenyl, 2-(AR2)ethenyl, or 2-(AR2a)ethenyl; or
(TB) T is selected from the following groups (TB1) to (TB3) :- 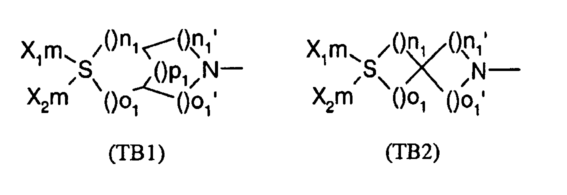 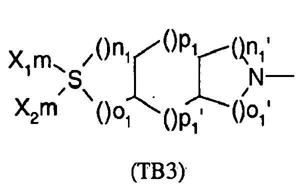 wherein:
X₁ₘ and X₂ₘ taken together represent R₂ₛ-(E)ₘₛ-N=; or
X₁ₘ is O= and X₂ₘ is R₂ₛ-(E)ₘₛ-N-, and vice versa;
wherein E is an electron withdrawing group selected from -SO₂-, -CO-, -O-CO-, -CO-O-, -CS-, -CON(Rₛ)-, -SO₂N(Rₛ)-, or E may represent a group of the formula R₃ₛ-C(=N-O-R₃ₛ)-C(=O)-, wherein R₃ₛ is H or as defined in R₂ₛ at (i) below;
or, when E is -CON(Rₛ)- or -SO₂N(Rₛ)-, R₂ₛ and Rₛ may link together to form a carbon chain which defines a 5- or 6-membered saturated, unsaturated or partially unsaturated ring linked via the N atom in E, which ring is optionally further substituted by an oxo substituent, and
which ring may be optionally fused with a phenyl group to form a benzo-fused system,
wherein the phenyl group is optionally substituted by up to three substituents independently selected from halo, cyano, (1-4C)alkyl and (1-4C)alkoxy;
ms is 0 or 1;
R₂ₛ and Rₛ are independently selected from :

(i) hydrogen (except where E is -SO₂-or -O-CO-), or
   (1-6C)alkyl {optionally substituted by one or more (1-4C)alkanoyl groups (including geminal disubstitution) and/or optionally monosubstituted by cyano, cyano-imino, (1-4C)alkoxy, trifluoromethyl, (1-4C)alkoxycarbonyl, phenyl (optionally substituted as for AR1), optionally substituted heteroaryl group of the formula AR2, AR2a, AR2b, AR3, AR3a, AR3b, AR4, AR4a or CY all as defined hereinafter, (1-4C)alkylS(O)_{q}- (q is 0, 1 or 2); and/or (with the proviso that where R₂ₛ is -SO₂ or -O-CO- not on the first carbon atom of the (1-6C) alkyl chain) optionally substituted by one or more groups (including geminal disubstitution) each independently selected from hydroxy and fluoro, and/or optionally further substituted, by no more than one of each of, oxo, -NRvRw [wherein Rv is hydrogen or (1-4C)alkyl; Rw is hydrogen or (1-4C)alkyl], (1-6C)alkanoylamino, (1-4C)alkoxycaibonylamino, N-(1-4C)alkyl-N-(1-6C)alkanoylamino, (1-4C)alkyls(O)pNH- or (1-4C)alkylS(O)ₚ-((1-4C)alky)N- (p is 1 or 2)} ; or
(ii) an optionally substituted aryl or optionally substituted heteroaryl group of the formula AR1, AR2, AR2a, AR2b, AR3, AR3a, AR3b, AR4, AR4a or CY all as defined herein;
   or (where ms is 0 only);
(iii) cyano, -CO-NRvRw, -CO-NRv Rw', -SO₂-NRvRw, -SO₂-NRv Rw' [wherein Rv is hydrogen or (1-4C)alkyl; Rw is hydrogen or (1-4C)alkyl; Rw' is phenyl (optionally substituted as for AR1), or a heteroaryl group selected from AR2, AR2a, AR2b, AR3, AR3a, AR3b, AR4, AR4a (as defined herein)],
   (1-4C)alkoxycarbonyl, trifluoromethyl, ethenyl, 2-(1-4C)alkylethenyl, 2-cyanoethenyl, 2-cyano-2-((1-4C)alkyl)ethenyl, 2-nitroethenyl, 2-nitro-2-((1-4C)alkyl)ethenyl, 2-((1-4C)alkylaminocarbonyl)ethenyl, 2-((1-4C)alkoxycarbonyl)ethenyl, 2-(AR1)ethenyl, 2-(AR2)ethenyl, or 2-(AR2a)ethenyl; and
wherein ()n₁, ()o₁, ()n₁., ()o₁, ()p₁ and ()p₁, represent chains of carbon atoms (optionally substituted as for AR1) of length n₁, o₁, n₁, o_{1'}, p₁ and p_{1'} respectively, and are independently 0-2, with the proviso that in (TB1) and (TB2) the sum of n₁, o₁, n₁, and o_{1,} does not exceed 8 (giving a maximum ring size of 14 in (TB1) and 11 in (TB2)), and in (TB3) the sum of n₁, o₁, n₁, o_{1'}, p₁ and p_{1'} does not exceed 6 (giving a maximum ring size of 12);
wherein Rc is selected from groups (Rc1) to (Rc5) :-
*(Rc1)* optionally substituted (1-6C)alkyl;
*(Rc2)* R¹³CO- , R¹³SO₂- or R¹³CS-
wherein R¹³ is selected from (Rc2a) to (Rc2e) :-
*(Rc2a)* AR1, AR2, AR2a, AR2b, AR3, AR3a, AR3b, AR4, AR4a, CY;
*(Rc2b)* hydrogen, (1-4C)alkoxycarbonyl, trifluoromethyl, -NRvRw [wherein Rv is hydrogen or (1-4C)alkyl; Rw is hydrogen or (1-4C)alkyl], ethenyl, 2-(1-4C)alkylethenyl, 2-cyanoethenyl, 2-cyano-2-((1-4C)alkyl)ethenyl, 2-nitroethenyl, 2-nitro-2-((1-4C)alkyl)ethenyl, 2-((1-4C)alkylaminocarbonyl)ethenyl, 2-((1-4C)alkoxycarbonyl)ethenyl, 2-(AR1)ethenyl, 2-(AR2)ethenyl, 2-(AR2a)ethenyl;
*(Rc2c)* optionally substituted (1-10C)alkyl;
*(Rc2d)* R¹⁴C(O)O(1-6C)alkyl wherein R¹⁴ is AR1, AR2, (1-4C)alkylamino (the (1-4C)alkyl group being optionally substituted by (1-4C)alkoxycarbonyl or by carboxy), benzyloxy-(1-4C)alkyl or (1-10C)alkyl {optionally substituted as defined for (Rc2c)};
*(Rc2e)* R¹⁵O- wherein R¹⁵ is benzyl, (1-6C)alkyl {optionally substituted as defined for (Rc2c)}, CY, or AR2b;
(*Rc3*) hydrogen, cyano, 2-cyanoethenyl, 2-cyano-2-((1-4C)alkyl)ethenyl, 2-((1-4C)alkylaminocarbonyl)ethenyl, 2-((1-4C)alkoxycarbonyl)ethenyl, 2-nitroethenyl, 2-nitro-2-((1-4C)alkyl)ethenyl, 2-(AR1)ethenyl, 2-(AR2)ethenyl, or of the formula (Rc3a) 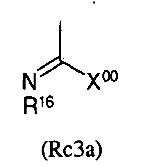 wherein X⁰⁰ is -OR¹⁷, -SR¹⁷, -NHR¹⁷ and -N(R¹⁷)₂;
   wherein R¹⁷ is hydrogen (when X⁰⁰ is -NHR¹⁷ and -N(R¹⁷)₂), and R¹⁷ is (1-4C)alkyl, phenyl or AR2 (when X⁰⁰ is -OR¹⁷, -SR¹⁷ and -NHR¹⁷); and R¹⁶ is cyano, nitro, (1-4C)alkylsulfonyl, (4-7C)cycloalkylsulfonyl, phenylsulfonyl, (1-4C)alkanoyl and (1-4C)alkoxycarbonyl;
*(Rc4)* trityl, AR1, AR2, AR2a, AR2b, AR3, AR3a, AR3b;
*(Rc5)* RdOC(Re)=CH(C=O)-, RfC(=O)C(=O)-, RgN=C(Rh)C(=O)- or RiNHC(Rj)=CHC(=O)- wherein Rd is (1-6C)alkyl; Re is hydrogen or (1-6C)alkyl, or Rd and Re together form a (3-4C)alkylene chain; Rf is hydrogen, (1-6C)alkyl, hydroxy(1-6C)alkyl, (1-6C)alkoxy(1-6C)alkyl, -NRvRw [wherein Rv is hydrogen or (1-4C)alkyl; Rw is hydrogen or (1-4C)alkyl], (1-6C)alkoxy, (1-6C)alkoxy(1-6C)alkoxy, hydroxy(2-6C)alkoxy, (1-4C)alkylamino(2-6C)alkoxy, di-(1-4C)alkylamino(2-6C)alkoxy; Rg is (1-6C)alkyl, hydroxy or (1-6C)alkoxy; Rh is hydrogen or (1-6C)alkyl; Ri is hydrogen, (1-6C)alkyl, AR1, AR2, AR2a, AR2b and Rj is hydrogen or (1-6C)alkyl;
wherein
CY is an optionally substituted cyclobutyl, cyclopentyl, cyclohexyl, cyclopentenyl or cyclohexenyl ring;
**AR1** is an optionally substituted phenyl or optionally substituted naphthyl;
**AR2** is an optionally substituted 5- or 6-membered, fully unsaturated (i.e with the maximum degree of unsaturation) monocyclic heteroaryl ring containing up to four heteroatoms independently selected from O, N and S (but not containing any O-O, O-S or S-S bonds), and linked via a ring carbon atom, or a ring nitrogen atom if the ring is not thereby quatemised;
**AR2a** is a partially hydrogenated version of AR2 (i.e. AR2 systems retaining some, but not the full, degree of unsaturation), linked via a ring carbon atom or linked via a ring nitrogen atom if the ring is not thereby quatemised;
**AR2b** is a fully hydrogenated version of AR2 (i.e. AR2 systems having no unsaturation), linked via a ring carbon atom or linked via a ring nitrogen atom;
**AR3** is an optionally substituted 8-, 9- or 10-membered, fully unsaturated (i.e with the maximum degree of unsaturation) bicyclic heteroaryl ring containing up to four heteroatoms independently selected from O, N and S (but not containing any O-O, O-S or S-S bonds), and linked via a ring carbon atom in either of the rings comprising the bicyclic system;
**AR3a** is a partially hydrogenated version of AR3 (i.e. AR3 systems retaining some, but not the full, degree of unsaturation), linked via a ring carbon atom, or linked via a ring nitrogen atom if the ring is not thereby quaternised, in either of the rings comprising the bicyclic system;
**AR3b** is a fully hydrogenated version of AR3 (i.e. AR3 systems having no unsaturation), linked via a ring carbon atom, or linked via a ring nitrogen atom, in either of the rings comprising the bicyclic system;
**AR4** is an optionally substituted 13- or 14-membered, fully unsaturated (i.e with the maximum degree of unsaturation) tricyclic heteroaryl ring containing up to four heteroatoms independently selected from O, N and S (but not containing any O-O, O-S or S-S bonds), and linked via a ring carbon atom in any of the rings comprising the tricyclic system;
**AR4a** is a partially hydrogenated version of AR4 (i.e. AR4 systems retaining some, but not the full, degree of unsaturation), linked via a ring carbon atom, or linked via a ring nitrogen atom if the ring is not thereby quatemised, in any of the rings comprising the tricyclic system;
wherein substituents on AR1, AR2, AR2a, AR2b, AR3, AR3a, AR3b, AR4, AR4a and CY are (on an available carbon atom) up to three substituents independently selected from (1-4C)alkyl {optionally substituted by (preferably one) substituents selected independently from hydroxy, trifluoromethyl, (1-4C)alkyl S(O)_{q}- (q is 0, 1 or 2) (this last substituent preferably on AR1 only), (1-4C)alkoxy, (1-4C)alkoxycarbonyl, cyano, nitro, (1-4C)alkanoylamino, - CONRvRw or -NRvRw}, trifluoromethyl, hydroxy, halo, nitro, cyano, thiol, (1-4C)alkoxy, (1-4C)alkanoyloxy, dimethylaminomethyleneaminocarbonyl, di(N-(1-4C)alkyl)aminomethylimino, carboxy, (1-4C)alkoxycarbonyl, (1-4C)alkanoyl, (1-4C)alkylSO₂amino, (2-4C)alkenyl·{optionally substituted by carboxy or (1-4C)alkoxycarbonyl}, (2-4C)alkynyl, (1-4C)alkanoylamino, oxo (=O), thioxo (=S), (1-4C)alkanoylamino {the (1-4C)alkanoyl group being optionally substituted by hydroxy}, (1-4C)alkyl S(O)_{q}- (q is 0, 1 or 2) {the (1-4C)alkyl group being optionally substituted by one or more groups independently selected from cyano, hydroxy and (1-4C)alkoxy}, -CONRvRw or -NRvRw [wherein Rv is hydrogen or (1-4C)alkyl; Rw is hydrogen or (1-4C)alkyl];
and wherein further suitable substituents on AR1, AR2, AR2a, AR2b, AR3, AR3a, AR3b, AR4, AR4a, and CY (on an available carbon atom), and also on alkyl groups (unless indicated otherwise) are up to three substituents independently selected from trifluoromethoxy, benzoylamino, benzoyl, phenyl {optionally substituted by up to three substituents independently selected from halo, (1-4C)alkoxy or cyano}, furan, pyrrole, pyrazole, imidazole, triazole, pyrimidine, pyridazine, pyridine, isoxazole, oxazole, isothiazole, thiazole, thiophene, hydroxyimino(1-4C)alkyl, (1-4C)alkoxyimino(1-4C)alkyl, halo-(1-4C)alkyl, (1-4C)alkanesulfonamido, -SO₂NRvRw [wherein Rv is hydrogen or (1-4C)alkyl; Rw is hydrogen or (1-4C)alkyl].

For the avoidance of doubt in the definition of (TA1) & (TA2) and (TB), it is to be understood that when R₂ₛ and Rₛ are independently selected from
(ii) (1-6C)alkyl {optionally substituted, for example, by no more than one of each of oxo and -NRvRw [wherein Rv is hydrogen or (1-4C)alkyl; Rw is hydrogen or (1-4C)alkyl]}, to avoid duplication with the substituent -CO-NRvRw provided in section (iii) of the definition for R₂ₛ and Rₛ, then oxo and -NRvRw are not to be both selected together when (1-6C)alkyl is methyl.

For the avoidance of doubt, in the above definitions of TA1, TA2 and TB, ()n₁, ()o₁, ()n₁, ()o₁, ()p₁ and ()P₁ indicate (-CH2-)n₁, (-CH2-)o₁, (-CH2-)n_{1'}, (-CH2)o_{1'}, (-CH2-)_{P1} and (-CH2-)p_{1'} respectively.

In this specification, where it is stated that a ring may be linked via an sp² carbon atom, which ring is fully saturated other than (where appropriate) at a linking sp² carbon atom, it is to be understood that the ring is linked via one of the carbon atoms in a C=C double bond.

In this specification the term'alkyl' includes straight chained and branched structures. For example, (1-6C)alkyl includes propyl, isopropyl and tert-butyl. However, references to individual alkyl groups such as "propyl" are specific for the straight chained version only, and references to individual branched chain alkyl groups such as "isopropyl" are specific for the branched chain version only. A similar convention applies to other radicals, for example halo(1-4C)alkyl includes 1-bromoethyl and 2-bromoethyl.

In general "halogen" when present as an aromatic ring substituent is selected from any one of bromine, chlorine or fluorine, as an aliphatic substituent from chlorine or fluorine.

There follow particular and suitable values for certain substituents and groups referred to in this specification. These values may be used where appropriate with any of the definitions and embodiments disclosed hereinbefore, or hereinafter. For the avoidance of doubt each stated species represents a particular and independent aspect of this invention.

Examples of **(1-4C)alkyl** and **(1-5C)alkyl** include methyl, ethyl, propyl, isopropyl and t-butyl; examples of **(1-6C)alkyl** include methyl, ethyl, propyl, isopropyl, t-butyl, pentyl and hexyl; examples of **(1-10C)alkyl** include methyl, ethyl, propyl, isopropyl, pentyl, hexyl, heptyl, octyl and nonyl; examples of **(1-4C)alkanoylamino-(1-4C)alkyl** include formamidomethyl, acetamidomethyl and acetamidoethyl; examples of **hydroxy(1-4C)alkyl** and **hydroxy(1-6C)alkyl** include hydroxymethyl, 1-hydroxyethyl, 2-hydroxyethyl and 3-hydroxypropyl; examples of **(1-4C)alkoxycarbonyl** include methoxycarbonyl, ethoxycarbonyl and propoxycarbonyl; examples of **2-((1-4C)alkoxycarbonyl)ethenyl** include 2-(methoxycarbonyl)ethenyl and 2-(ethoxycarbonyl)ethenyl; examples of **2-cyano-2-((1-4C)alkyl)ethenyl** include 2-cyano-2-methylethenyl and 2-cyano-2-ethylethenyl; examples of **2-nitro-2-((1-4C)alkyl)ethenyl** include 2-nitro-2-methylethenyl and 2-nitro-2-ethylethenyl; examples of **2-((1-4C)alkylaminocarbonyl)ethenyl** include 2-(methylaminocarbonyl)ethenyl and 2-(ethylaminocarbonyl)ethenyl; examples of **(2-4C)alkenyl** include allyl and vinyl; examples of **(2-4C)alkynyl** include ethynyl and 2-propynyl; examples of **(1-4C)alkanoyl** include formyl, acetyl and propionyl; examples of **(1-4C)alkoxy** include methoxy, ethoxy and propoxy; examples of **(1-6C)alkoxy** and **(1-10C)alkoxy** include methoxy, ethoxy, propoxy and pentoxy; examples of **(1-4C)alkylthio** include methylthio and ethylthio; examples of (1-**4C)alkylamino** include methylamino, ethylamino and propylamino; examples of **di-((1-4C)alkyl)amino** include dimethylamino, N-ethyl-N-methylamino, diethylamino, N-methyl-N-propylamino and dipropylamino; examples of halo groups include fluoro, chloro and bromo; examples of **(1-4C)alkylsulfonyl** include methylsulfonyl and ethylsulfonyl; examples of (1-**4C)alkoxy-(1-4C)alkoxy** and **(1-6C)alkoxy-(1-6C)alkoxy** include methoxymethoxy, 2-methoxyethoxy, 2-ethoxyethoxy and 3-methoxypropoxy; examples of **(1-4C)alkoxy-(1-4C)alkoxy-(1-4C)alkoxy** include 2-(methoxymethoxy)ethoxy, 2-(2-methoxyethoxy)ethoxy; 3-(2-methoxyethoxy)propoxy and 2-(2-ethoxyethoxy)ethoxy; examples of **(1-4C)alkylS(O)**_{**2**}**amino** include methylsulfonylamino and ethylsulfonylamino; examples of **(1-4C)alkanoylamino** and **(1-6C)alkanoylamino** include formamido, acetamido and propionylamino; examples of **(1-4C)alkoxycarbonylamino** include methoxycarbonylamino and ethoxycarbonylamino; examples of **N-(1-4C)alkyl-N-(1-**6C)alkanoylamino include N-methylacetamido, N-ethylacetamido and N-methylpropionamido; examples of **(1-4C)alkylS(O)pNH-** wherein p is 1 or 2 include methylsulfinylamino, methylsulfonylamino, ethylsulfinylamino and ethylsulfonylamino; examples of **(1-4C)alkylS(O)p((1-4C)alkyl)N-** wherein p is 1 or 2 include methylsulfinylmethylamino, methylsulfonylmethylamino, 2-(ethylsulfinyl)ethylamino and 2-(ethylsulfonyl)ethylamino; examples of **fluoro(1-4C)alkylS(O)**_{**p**}**NH-** wherein p is 1 or 2 include trifluoromethylsulfinylamino and trifluoromethylsulfonylamino; examples of **fluoro(1-4C)alkylS(O)**_{**p**}**((1-4C)alkyl)NH-** wherein p is 1 or 2 include trifluoromethylsulfinylmethylamino and trifluoromethylsulfonylmethylamino examples of (1-**4C)alkoxy(hydroxy)phosphoryl** include methoxy(hydroxy)phosphoryl and ethoxy(hydroxy)phosphoryl; examples of **di-(1-4C)alkoxyphosphoryl** include di-methoxyphosphoryl, di-ethoxyphosphoryl and ethoxy(methoxy)phosphoryl; examples of **(1-4C)alkylS(O)**_{**q**}**-** wherein q is 0, 1 or 2 include methylthio, ethylthio, methylsulfinyl, ethylsulfinyl, methylsulfonyl and ethylsulfonyl; examples of **phenylS(O)q** and **naphthylS(O)**_{**q-**} wherein q is 0, 1 or 2 are phenylthio, phenylsulfinyl, phenylsulfonyl and naphthylthio, naphthylsulfinyl and naphthylsulfonyl respectively; examples of **benzyloxy-(1-4C)alkyl** include benzyloxymethyl and benzyloxyethyl; examples of a **(3-4C)alkylene** chain are trimethylene or tetramethylene; examples of **(1-6C)alkoxy-(1-6C)alkyl** include methoxymethyl, ethoxymethyl and 2-methoxyethyl; examples of hydroxy-(2-6C)alkoxy include 2-hydroxyethoxy and 3-hydroxypropoxy; examples of **(1-4C)alkylamino-(2-6C)alkoxy** include 2-methylaminoethoxy and 2-ethylaminoethoxy; examples of **di-(1-4C)alkylamino-(2-6C)alkoxy** include 2-dimethylaminoethoxy and 2-diethylaminoethoxy; examples of **phenyl(1-4C)alkyl** include benzyl and phenethyl; examples of **(1-4C)alkylcarbamoyl** include methylcarbamoyl and ethylcarbamoyl; examples of **di((1-4C)alkyl)carbamoyl** include di(methyl)carbamoyl and di(ethyl)carbamoyl; examples of **hydroxyimino(1-4C)alkyl** include hydroxyiminomethyl, 2-(hydroxyimino)ethyl and 1-(hydroxyimino)ethyl; examples of **(1-4C)alkoxyimino-(1-4C)alkyl** include methoxyiminomethyl, ethoxyiminomethyl, 1-(methoxyimino)ethyl and 2-(methoxyimino)ethyl; examples of **halo(1-4C)alkyl** include, halomethyl, 1-haloethyl, 2-haloethyl, and 3-halopropyl; examples of **nitro(1-4C)alkyl** include nitromethyl, 1-nitroethyl, 2-nitroethyl and 3-nitropropyl; examples of **amino(1-4C)alkyl** include aminomethyl, 1-aminoethyl, 2-aminoethyl and 3-aminopropyl; examples of cyano(1-4C)alkyl include cyanomethyl, 1-cyanoethyl, 2-cyanoethyl and 3-cyanopropyl; examples of **(1-4C)alkanesulfonamido** include methanesulfonamido and ethanesulfonamido; examples of **(1-4C)alkylaminosulfonyl** include methylaminosulfonyl and ethylaminosulfonyl; examples of **di-(1-4C)alkylaminosulfonyl** include dimethylaminosulfonyl, diethylaminosulfonyl and N-methyl-N-ethylaminosulfonyl; examples of **(1-4C)alkanesulfonyloxy** include methylsulfonyloxy, ethylsulfonyloxy and propylsulfonyloxy; examples of **(1-4C)alkanoyloxy** include acetoxy; examples of **(1-4C)alkylaminocarbonyl** include methylaminocarbonyl and ethylaminocarbonyl; examples of **di((1-4C)alkyl)aminocarbonyl** include dimethylaminocarbonyl and diethylaminocarbonyl; examples of **(3-8C)cycloalkyl** include cyclopropyl, cyclobutyl, cyclopentyl and cyclohexyl; examples of **(4-7C)cycloalkyl** include cyclobutyl, cyclopentyl and cyclohexyl; examples of **di(N-(1-4C)alkyl)aminomethylimino** include dimethylaminomethylimino and diethylaminomethylimino.

Particular values for AR2 include, for example, for those AR2 containing one heteroatom, furan, pyrrole, thiophene; for those AR2 containing one to four N atoms, pyrazole, imidazole, pyridine, pyrimidine, pyrazine, pyridazine, 1,2,3- & 1,2,4-triazole and tetrazole; for those AR2 containing one N and one O atom, oxazole, isoxazole and oxazine; for those AR2 containing one N and one S atom, thiazole and isothiazole; for those AR2 containing two N atoms and one S atom, 1,2,4- and 1,3,4-thiadiazole.

Particular examples of AR2a include, for example, dihydropynole (especially 2,5-dihydropyrrol-4-yl) and tetrahydropyridine (especially 1,2,5,6-tetrahydropyrid-4-yl). Particular examples of AR2b include, for example, tetrahydrofuran, pyrrolidine, morpholine (preferably morpholino), thiomorpholine (preferably thiomorpholino), piperazine (preferably piperazino), imidazoline and piperidine, 1,3-dioxolan-4-yl, 1,3-dioxan-4-yl, 1,3-dioxan-5-yl and 1,4-dioxan-2-yl.

Particular values for AR3 include, for example, bicyclic benzo-fused systems containing a 5- or 6-membered heteroaryl ring containing one nitrogen atom and optionally 1-3 further heteroatoms chosen from oxygen, sulfur and nitrogen. Specific examples of such ring systems include, for example, indole, benzofuran, benzothiophene, benzimidazole, benzothiazole, benzisothiazole, benzoxazole, benzisoxazole, quinoline, quinoxaline, quinazoline, phthalazine and cinnoline.

Other particular examples of AR3 include 5/5-, 5/6 and 6/6 bicyclic ring systems containing heteroatoms in both of the rings. Specific examples of such ring systems include, for example, purine and naphthyridine.

Further particular examples of AR3 include bicyclic heteroaryl ring systems with at least one bridgehead nitrogen and optionally a further 1-3 heteroatoms chosen from oxygen, sulfur and nitrogen. Specific examples of such ring systems include, for example, 3H-pyrrolo[1,2-a]pyrrole, pyrrolo[2,1-b]thiazole, 1H-imidazo[1,2-a]pyrrole, 1H-imidazo[1,2-a]imidazole, 1H,3H-pyrrolo[1,2-c]oxazole, 1H-imidazo[1,5-a]pyrrole, pyrrolo[1,2-b]isoxazole, imidazo[5,1-b]thiazole, imidazo[2,1-b]thiazole, indolizine, imidazo[1,2-a]pyridine, imidazo[1,5-a]pyridine, pyrazolo[1,5-a]pyridine, pyrrolo[1,2-b]pyridazine, pyrrolo[1,2-c]pyrimidine, pyrrolo[1,2-a]pyrazine, pyrrolo[1,2-a]pyrimidine, pyrido[2,1-c]-s-triazole, s-triazole[1,5-a]pyridine, imidazo[1,2-c]pyrimidine, imidazo[1,2-a]pyrazine, imidazo[1,2-a]pyrimidine, imidazo[1,5-a]pyrazine, imidazo[1,5-a]pyrimidine, imidazo[1,2-b]-pyridazine, s-triazolo[4,3-a]pyrimidine, imidazo[5,1-b]oxazole and imidazo[2,1-b]oxazole. Other specific examples of such ring systems include, for example, [1H]-pyrrolo[2,1-c]oxazine, [3H]-oxazolo[3,4-a]pyridine, [6H]-pyrrolo[2,1-c]oxazine and pyrido[2,1-c][1,4]oxazine. Other specific examples of 5/5- bicyclic ring systems are imidazooxazole or imidazothiazole, in particular imidazo[5,1-b]thiazole, imidazo[2,1-b]thiazole, imidazo[5,1-b]oxazole or imidazo[2,1-b]oxazole.

Particular examples of AR3a and AR3b include, for example, indoline, 1,3,4,6,9,9a-hexahydropyrido[2,1c][1,4]oxazin-8-yl, 1,2,3,5,8,8a-hexahydroimidazo[1,5a]pyridin-7-yl, 1,5,8,8a-tetrahydrooxazolo[3,4a]pyridin-7-yl, 1,5,6,7,8,8a-hexahydrooxazolo[3,4a]pyridin-7-yl, (7aS)[3H,5H]-1,7a-dihydropyrrolo[1,2c]oxazol-6-yl, (7aS)[5H]-1,2,3,7a-tetrahydropyrrolo[1,2c]imidazol-6-yl, (7aR)[3H,5H]-1,7a-dihydropyrrolo[1,2c]oxa2ol-6-yl, [3H,5H]-pyrrolo[1,2-c]oxazol-6-yl, [5H]-2,3-dihydropyrrolo[1,2-c]imidazol-6-yl, [3H,5H]-pyrrolo[1,2-c]thiazol-6-yl, [3H,5H]-1,7a-dihydropynolo[1,2-c]thiazol-6-yl, [5H]-pyrrolo[1,2-c]imidazol-6-yl, [1H]-3,4,8,8a-tetrahydropyrrolo[2,1-c]oxazin-7-yl, [3H]-1,5,8,8a-tetrahydrooxazolo[3,4-a]pyrid-7-yl, [3H]-5,8-dihydroxazolo[3,4-alpyrid-7-yl and 5,8-dihydroimidazo[1,5-a]pyrid-7-yl.

Particular values for AR4 include, for example, pyrrolo[a]quinoline, 2,3-pyrroloisoquinoline, pytolo[a]isoquinoline, 1H-pyrrolo[1,2-a]benzimidazole, 9H-imidazo[1,2-a]indole, 5H-imidazo[2,1-a]isoindole, 1H-imidazo[3,4-a]indole, imidazo[1,2-a]quinoline, imidazo[2,1-a]isoquinoline, imidazo[1,5-a]quinoline and imidazo[5,1-a]isoquinoline.

The nomenclature used is that found in, for example, "Heterocyclic Compounds (Systems with bridgehead nitrogen), W.L.Mosby (Intercsience Publishers Inc., New York), 1961, Parts 1 and 2.

Where optional substituents are listed such substitution is preferably not geminal disubstitution unless stated otherwise. If not stated elsewhere suitable optional substituents for a particular group are those as stated for similar groups herein.

Suitable substituents on AR1, AR2, AR2a, AR2b, AR3, AR3a, AR3b, AR4, AR4a and CY are (on an available carbon atom) up to three substituents independently selected from (1-4C)alkyl {optionally substituted by (preferably one) substituents selected independently from hydroxy, trifluoromethyl, (1-4C)alkyl S(O)_{q}- (q is 0, 1 or 2) (this last substituent preferably on AR1 only), (1-4C)alkoxy, (1-4C)alkoxycarbonyl, cyano, nitro, (1-4C)alkanoylamino, - CONRvRw or-NRvRw}, trifluoromethyl, hydroxy, halo, nitro, cyano, thiol, (1-4C)alkoxy, (1-4C)alkanoyloxy, dimethylaminomethyleneaminocarbonyl, di(N-(1-4C)alkyl)aminomethylimino, carboxy, (1-4C)alkoxycarbonyl, (1-4C)alkanoyl, (1-4C)alkyISO₂amino, (2-4C)alkenyl {optionally substituted by carboxy or (1-4C)alkoxycarbonyl }, (2-4C)alkynyl, (1-4C)alkanoylamino, oxo (=O), thioxo (=S), (1-4C)alkanoylamino {the (1-4C)alkanoyl group being optionally substituted by hydroxy}, (1-4C)alkyl S(O)_{q}- (q is 0, 1 or 2) {the (1-4C)alkyl group being optionally substituted by one or more groups independently selected from cyano, hydroxy and (1-4C)alkoxy}, -CONRvRw or -NRvRw [wherein Rv is hydrogen or (1-4C)alkyl; Rw is hydrogen or (1-4C)alkyl].

Further suitable substituents on AR1, AR2, AR2a, AR2b, AR3, AR3a, AR3b, AR4, AR4a, and CY (on an available carbon atom), and also on alkyl groups (unless indicated otherwise) are up to three substituents independently selected from trifluoromethoxy, benzoylamino, benzoyl, phenyl {optionally substituted by up to three substituents independently selected from halo, (1-4C)alkoxy or cyano}, furan, pyrrole, pyrazole, imidazole, triazole, pyrimidine, pyridazine, pyridine, isoxazole, oxazole, isothiazole, thiazole, thiophene, hydroxyimino(1-4C)alkyl, (1-4C)alkoxyimino(1-4C)alkyl, halo-(1-4C)alkyl, (1-4C)alkanesulfonamido, -SO₂NRvRw [wherein Rv is hydrogen or (1-4C)alkyl; Rw is hydrogen or (1-4C)alkyl].

Preferable optional substituents on Ar2b as 1,3-dioxolan-4-yl, 1,3-dioxan-4-yl, 1,3-dioxan-5-yl or 1,4-dioxan-2-yl are mono- or disubstitution by substituents independently selected from (1-4C)alkyl (including geminal disubstitution), (1-4C)alkoxy, (1-4C)alkylthio, acetamido, (1-4C)alkanoyl, cyano, trifluoromethyl and phenyl].

Preferable optional substituents on CY are mono- or disubstitution by substituents independently selected from (1-4C)alkyl (including geminal disubstitution), hydroxy, (1-4C)alkoxy, (1-4C)alkylthio, acetamido, (1-4C)alkanoyl, cyano, and trifluoromethyl.

Suitable substituents on AR2, AR2a, AR2b, AR3, AR3a, AR3b, AR4 and AR4a are (on an available nitrogen atom, where such substitution does not result in quatemization) (1-4C)alkyl, (1-4C)alkanoyl {wherein the (1-4C)alkyl and (1-4C)alkanoyl groups are optionally substituted by (preferably one) substituents independently selected from cyano, hydroxy, nitro, trifluoromethyl, (1-4C)alkyl S(O)_{q}- (q is 0, 1 or 2), (1-4C)alkoxy, (1-4C)alkoxycarbonyl, (1-4C)alkanoylamino, -CONRvRw or -NRvRw [wherein Rv is hydrogen or (1-4C)alkyl; Rw is hydrogen or (1-4C)alkyl]}, (2-4C)alkenyl, (2-4C)alkynyl, (1-4C)alkoxycarbonyl or oxo (to form an N-oxide).

Suitable pharmaceutically-acceptable salts include acid addition salts such as methanesulfonate, fumarate, hydrochloride, citrate, maleate, tartrate and (less preferably) hydrobromide. Also suitable are salts formed with phosphoric and sulfuric acid. In another aspect suitable salts are base salts such as an alkali metal salt for example sodium, an alkaline earth metal salt for example calcium or magnesium, an organic amine salt for example triethylamine, morpholine, N-methylpiperidine, N-ethylpiperidine, procaine, dibenzylamine, N,N-dibenzylethylamine, tris-(2-hydroxyethyl)amine, N-methyl d-glucamine and amino acids such as lysine. There may be more than one cation or anion depending on the number of charged functions and the valency of the cations or anions. A preferred pharmaceutically-acceptable salt is the sodium salt.

In addition certain salts of the sulfoximine NH residue are envisaged, by way of non - limiting example sulphonic acid derivatives, methane sulfonate, hydrochloride and hydrobromide salts.

However, to facilitate isolation of the salt during preparation, salts which are less soluble in the chosen solvent may be preferred whether pharmaceutically-acceptable or not.

As stated before, we have discovered a range of compounds that have good activity against a broad range of Gram-positive pathogens including organisms known to be resistant to most commonly used antibiotics, together with activity against fastidious Gram negative pathogens such as H.influenzae, M.catarrhalis, Mycoplasma and Chlamydia strains. They have good physical and/or pharmacokinetic properties in general, and favourable toxicological profiles.

Particularly preferred compounds of the invention comprise a compound of formula (IA), or a pharmaceutically-acceptable salt or an in-vivo hydrolysable ester thereof, wherein the substituents HET, T and other substituents mentioned above have values disclosed hereinbefore, or any of the following values (which may be used where appropriate with any of the definitions and embodiments disclosed hereinbefore or hereinafter):

In one embodiment of the invention are provided compounds of formula (IA), in an alternative embodiment are provided pharmaceutically-acceptable salts of compounds of formula (IA), and in a further alternative embodiment are provided in-vivo hydrolysable esters of compounds of formula (IA).

In one embodiment is provided a compound of formula (IA) or a pharmaceutically-acceptable salt or in-vivo hydrolysable ester thereof, as defined herein wherein T is selected from (TA2) and (TB). In another embodiment is provided a compound of formula (IA) as defined herein wherein T is (TA1).

In (TA1), when the ring has an optional double bond between any two ring carbon atoms, the ring is preferably linked via an sp² carbon atom of the double bond.

Preferably (TA1) is (TA1a) or (TA1b), and preferably (TA2) is (TA2a) :- 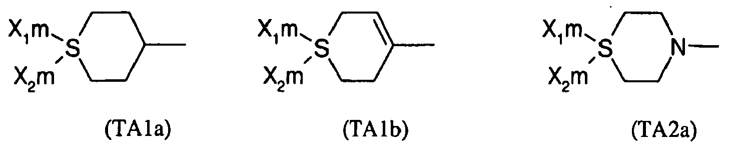 wherein X₁ₘ and X₂ₘ are as defined above, and hereinafter.

5 In (TB1) to (TB3), preferably n₁ =o₁ & n_{1'} = o_{1'} (most preferably all are 1); p₁ = p_{1'} (most preferably both are 0); and further preferred values for the groups defined in (TB) are defined by formulae (TB1a, b), (TB2a) and (TB3a) :- 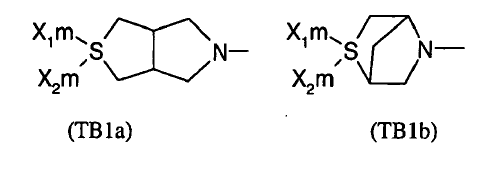 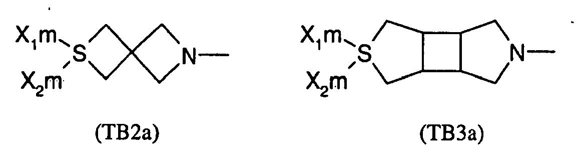 wherein X₁ₘ and X₂ₘ are as defined above, and hereinafter.

Preferably X₁ₘ is O= and X₂ₘ is R₂ₛ-(E)ₘₛ-N-, and vice versa.

When ms is 0, R₂ₛ is preferably selected from :
(i) hydrogen, a (1-6C)alkyl group {optionally monosubstituted by (1-4C)alkanoyl group, cyano, cyano-imino, (1-4C)alkoxy, trifluoromethyl, (1-4C)alkoxycarbonyl, phenyl (optionally substituted as for AR1 defined herein), optionally substituted heteroaryl group of the formula AR2, AR2a, AR2b, AR3, AR3a, AR3b, AR4, AR4a or CY all as defined (and optionally substituted as defined) herein, (1-4C)alkylS(O)_{q}- (q is 0, 1 or 2); or optionally substituted by one or more fluoro groups (including geminal disubstitution); or optionally substituted by one or more hydroxy groups (excluding geminal disubstitution), and/or optionally further substituted, by no more than one of each of, oxo, -NRvRw [wherein Rv is hydrogen or (1-4C)alkyl; Rw is hydrogen or (1-4C)alkyl], (1-6C)alkanoylamino, (1-4C)alkoxycarbonylamino, N-(1-4C)alkyl-N-(1-6C)alkanoylamino, (1-4C)alkylS(O)ₚNH- or (1-4C)alkylS(O)ₚ-((1-4C)alkyl)N- (p is 1 or 2)}; or
(ii) an optionally substituted aryl or optionally substituted heteroaryl group of the formula AR1, AR2, AR2a, AR2b, AR3, AR3a, AR3b, AR4, AR4a or CY all as defined (and optionally substituted as defined) herein; or
(iii) cyano, -CO-NRvRw, -CO-NRv Rw', -SO₂-NRvRw, -SO₂-NRv Rw' [wherein Rv is hydrogen or (1-4C)alkyl; Rw is hydrogen or (1-4C)alkyl; Rw' is phenyl (optionally substituted as for AR1 defined herein), or a heteroaryl group selected from AR2, AR2a, AR2b, AR3, AR3a, AR3b, AR4, AR4a (optionally substituted as defined herein)], (1-4C)alkoxycarbonyl, trifluoromethyl.

When ms is 0, R₂ₛ is most preferably selected from :
(i) hydrogen, (1-6C)alkyl {optionally monosubstituted by (1-4C)alkoxy, trifluoromethyl, (1-4C)alkylS(O)_{q}- (q is 0, 1 or 2); or optionally substituted by one or more fluoro-groups (including geminal disubstitution); or optionally substituted by one or more hydroxy groups (excluding geminal disubstitution)}; or
(iii) -CO-NRvRw [wherein Rv is hydrogen or (1-4C)alkyl; Rw is hydrogen or (1-4C)alkyl], -CO-NRv Rw' [wherein Rv is hydrogen or (1-4C)alkyl; Rw' is phenyl (optionally substituted as for AR1 defined herein)], (1-4C)alkoxycarbonyl.

When ms is 1, E is preferably -CO- or -SO₂- and R₂ₛ is preferably selected from :
(i) (1-6C)alkyl {optionally monosubstituted by cyano, cyano-imino, (1-4C)alkoxy, trifluoromethyl, (1-4C)alkoxycarbonyl, phenyl (optionally substituted as for AR1 defined herein), optionally substituted heteroaryl group of the formula AR2, AR2a, AR2b, AR3, AR3a, AR3b, AR4, AR4a or CY all as defined (and optionally substituted as defined) herein, (1-4C)alkylS(O)_{q}- (q is 0, 1 or 2); and/or (with the proviso that where R₂ₛ is -SO₂- or -O-CO-not on the first carbon atom of the (1-6C) alkyl chain) optionally substituted by one or more groups (including geminal disubstitution) each independently selected from hydroxy and fluoro, and/or optionally monosubstituted by -NRvRw [wherein Rv is hydrogen or (1-4C)alkyl; Rw is hydrogen or (1-4C)alkyl], (1-6C)alkanoylamino, (1-4C)alkoxycarbonylamino, N-(1-4C)alkyl-N-(1-6C)alkanoylamino, (1-4C)alkylS(O)ₚNH- or (1-4C)alkylS(O)ₚ₋((1-4C)alkyl)N- (p is 1 or 2)}; or
(ii) an optionally substituted aryl or heteroaryl group of the formula AR1, AR2, AR2a, AR2b, AR3, AR3a, AR3b, AR4, AR4a or CY all as defined (and optionally substituted as defined) herein.

When ms is 1, E is preferably -CO- or -SO₂- and R₂ₛ is most preferably selected from :
(i) (1-6C)alkyl {optionally monosubstituted by (1-4C)alkoxy, trifluoromethyl, (1-4C)alky]S(O)_{q}- (q is 0, 1 or 2); or optionally substituted by one or more fluoro groups (including geminal disubstitution); or optionally substituted by one or more hydroxy groups (excluding geminal disubstitution)}, (1-6C)alkanoylamino, (1-4C)alkoxycarbonylamino.

In (TB) and (TA2), where ()n₁, ()o₁, ()n_{1'}, ()o_{1'}, ()p₁ and ()p_{1'} represent chains of carbon atoms optionally substituted as defined for AR1 herein, preferable optional substituents are selected from (preferably one of) hydroxy, trifluoromethyl, (1-4C)alkyl S(O)q- (q is 0, 1 or 2), (1-4C)alkoxy, (1-4C)alkoxycarbonyl, cyano, nitro, (1-4C)alkanoylamino, -CONRvRw or-NRvRw [wherein Rv is hydrogen or (1-4C)alkyl; Rw is hydrogen or (1-4C)alkyl]. Most preferably, ()n₁, ()o₁, ()n_{1'}, ()o_{1'}, ()p₁ and ()p_{1'}, represent unsubstituted chains of carbon atoms.

Preferably T is selected from (TA1a & b), (TA2a) and (TB1a & b).

Preferable values for other substituents (which may be used where appropriate with any of the definitions and embodiments disclosed hereinbefore or hereinafter) are :-
(b) preferably HET is 1,2,3-triazole (especially 1,2,3-triazol-1-yl), 1,2,4-triazole (especially 1,2,4-triazol-1-yl) and tetrazole (preferably tetrazol-2-yl).
(c) preferably HET is a di-hydro version of pyrimidine, pyridazine, pyrazine, 1,2,3-triazine, 1,2,4-triazine, 1,3,5-triazine and pyridine.
(d) Preferably HET is unsubstituted.
(f) In one aspect preferably one of R² and R³ is hydrogen and the other fluoro. In another aspect both R² and R³ are fluoro.
(g) Preferably Rc is R¹³CO- and preferably R¹³ is (1-4C)alkoxycarbonyl, hydroxy(1-4C)alkyl, (1-4C)alkyl (optionally substituted by one or two hydroxy groups, or by an (1-4C)alkanoyl group), (1-4C)alkylamino, dimethylamino(1-4C)alkyl, (1-4C)alkoxymethyl, (1-4C)alkanoylmethyl, (1-4C)alkanoyloxy(1-4C)alkyl, (1-5C)alkoxy or 2-cyanoethyl.
(h) More preferably R¹³ is 1,2-dihydroxyethyl, 1,3-dihydroxyprop-2-yl, 1,2,3-trihydroxyprop-1-yl, methoxycarbonyl, hydroxymethyl, methyl, methylamino, dimethylaminomethyl, methoxymethyl, acetoxymethyl, methoxy, methylthio, naphthyl, tert-butoxy or 2-cyanoethyl.
(i) Particularly preferred as R¹³ is 1,2-dihydroxyethyl, 1,3-dihydroxyprop-2-yl or 1,2,3-trihydroxyprop-1-yl.
(j) In another aspect preferably R¹³ is hydrogen, (1-10C)alkyl [optionally substituted by one or more hydroxy] or R¹⁴C(O)O(1-6C)alkyl.

For compounds of formula (I) preferred values for Rc are those in group (Rc2) when present in any of the definitions herein containing Rc.

In the definition of (Rc2c) the AR2a, AR2b, AR3a and AR3b versions of AR2 and AR3 containing groups are preferably excluded.

Further especially preferred compounds of the invention are of the formula (IA) wherein HET is 1,2,3-triazole (especially 1,2,3-triazol-1-yl), 1,2,4-triazole (especially 1,2,4-triazol-1-yl) or tetrazole (preferably tetrazol-2-yl;
R² and R³ are independently hydrogen or fluoro;
T is selected from (TA1a & b), (TA2a) and (TB1a & b); or in-vivo hydrolysable esters or pharmaceutically-acceptable salts thereof.

In the above aspects and preferred compounds of formula (IA), in (TA1), (TA2) and (TB1) to (TB3); and especially in (TA1a & b), (TA2a) and (TB1a & b); preferably X₁ₘ is O= and X₂ₘ is R₂ₛ-(E)ₘₛ-N-, and vice versa; and when ms is 0, R₂ₛ is preferably selected from
(i) hydrogen, a (1-6C)alkyl group {optionally monosubstituted by (1-4C)alkanoyl group, cyano, cyano-imino, (1-4C)alkoxy, trifluoromethyl, (1-4C)alkoxycarbonyl, phenyl (optionally substituted as for AR1 defined herein), optionally substituted heteroaryl group of the formula AR2, AR2a, AR2b, AR3, AR3a, AR3b, AR4, AR4a or CY all as defined (and optionally substituted as defined) herein, (1-4C)alkylS(O)_{q}- (q is 0, 1 or 2); or optionally substituted by one or more fluoro groups (including geminal disubstitution); or optionally substituted by one or more hydroxy groups (excluding geminal disubstitution), and/or optionally further substituted, by no more than one of each of, oxo, -NRvRw [wherein Rv is hydrogen or (1-4C)alkyl; Rw is hydrogen or (1-4C)alkyl], (1-6C)alkanoylamino, (1-4C)alkoxycarbonylamino, N-(1-4C)alkyl-N-(1-6C)alkanoylamino, (1-4C)alkylS(O)ₚNH- or (1-4C)alkylS(O)ₚ-((1-4C)alkyl)N-(p is 1 or 2)}; or
(ii) an optionally substituted aryl or optionally substituted heteroaryl group of the formula AR1, AR2, AR2a, AR2b, AR3, AR3a, AR3b, AR4, AR4a or CY all as defined (and optionally substituted as defined) herein; or (where ms is 0 only),
(iii) cyano, -CO-NRvRw, -CO-NRv Rw', -SO₂-NRvRw, -SO₂-NRv Rw' [wherein Rv is hydrogen or (1-4C)alkyl; Rw is hydrogen or (1-4C)alkyl; Rw' is phenyl (optionally substituted as for AR1 defined herein), or a heteroaryl group selected from AR2, AR2a, AR2b, AR3, AR3a, AR3b, AR4, AR4a (optionally substituted as defined herein)], (1-4C)alkoxycarbonyl, trifluoromethyl;
   and when ms is 1, E is preferably -CO- or -SO₂- and R₂ₛ is preferably selected from :

(i) (1-6C)alkyl {optionally monosubstituted by cyano, cyano-imino, (1-4C)alkoxy, trifluoromethyl, (1-4C)alkoxycarbonyl, phenyl (optionally substituted as for AR1 defined herein), optionally substituted heteroaryl group of the formula AR2, AR2a, AR2b, AR3, AR3a, AR3b, AR4, AR4a or CY all as defined (and optionally substituted as defined) herein, (1-4C)alkylS(O)_{q}- (q is 0, 1 or 2); and/or (with the proviso that where R₂ₛ is -SO₂- or -O-CO-not on the first carbon atom of the (1-6C) alkyl chain) optionally substituted by one or more groups (including geminal disubstitution) each independently selected from hydroxy and fluoro, and/or optionally monosubstituted by -NRvRw [wherein Rv is hydrogen or (1-4C)alkyl; Rw is hydrogen or (1-4C)alkyl], (1-6C)alkanoylamino, (1-4C)alkoxycarbonylamino, N-(1-4C)alkyl-N-(1-6C)alkanoylamino, (1-4C)alkylS(O)ₚNH- or (1-4C)alkylS(O)ₚ-((1-4C)alkyl)N- (p is I or 2)}; or
(ii) an optionally substituted aryl or heteroaryl group of the formula AR1, AR2, AR2a, AR2b, AR3, AR3a, AR3b, AR4, AR4a or CY all as defined (and optionally substituted as defined) herein.

In all of the above aspects and preferred compounds of formula (IA), in-vivo hydrolysable esters are preferred where appropriate, especially phosphoryl esters (as defined by formula (PD3) with npd as 1, or of formula (PS1)).

In all of the above definitions the preferred compounds are as shown in formula (IC) as described hereinafter; i.e. the pharmaceutically active enantiomer.

Particularly preferred compounds of the present invention include the compounds described in the following examples. Therefore the present invention also provides a compound described in any one of the following examples, or a pharmaceutically-acceptable salt or an in-vivo hydrolysable ester thereof (and in particular compounds and salts thereof); and their use as a medicament (as herein described).

The compounds of the formula (IA) may be administered in the form of a pro-drug which is broken down in the human or animal body to give a compound of the formula (IA). A prodrug may be used to alter or improve the physical and/or pharmacokinetic profile of the parent compound and can be formed when the parent compound contains a suitable group or substituent which can be derivatised to form a prodrug. Examples of pro-drugs include in-vivo hydrolysable esters of a compound of the formula (IA) or a pharmaceutically-acceptable salt thereof.

Various forms of prodrugs are known in the art, for examples see:
a) Design of Prodrugs, edited by H. Bundgaard, (Elsevier, 1985) and Methods in Enzymology, Vol. 42, p. 309-396, edited by K. Widder, *et al.* (Academic Press, 1985);
b) A Textbook of Drug Design and Development, edited by Krogsgaard-Larsen and H. Bundgaard, Chapter 5 "Design and Application of Prodrugs", by H. Bundgaard p. 113-191 (1991);
c) H. Bundgaard, Advanced Drug Delivery Reviews, 8, 1-38 (1992);
d) H. Bundgaard, *et al.,* Journal of Pharmaceutical Sciences, 77, 285 (1988); and
e) N. Kakeya, *et al.,* Chem Pharm Bull, 32, 692 (1984).

An in-vivo hydrolysable ester of a compound of the formula (IA) or a pharmaceutically-acceptable salt thereof containing carboxy or hydroxy group is, for example, a pharmaceutically-acceptable ester which is hydrolysed in the human or animal body to produce the parent acid or alcohol. Suitable pharmaceutically-acceptable esters for carboxy include (1-6C)alkoxymethyl esters for example methoxymethyl, (1-6C)alkanoyloxymethyl esters for example pivaloyloxymethyl, phthalidyl esters, (3-8C)cycloalkoxycarbonyloxy(1-6C)alkyl esters for example 1-cyclohexylcarbonyloxyethyl; 1,3-dioxolan-2-onylmethyl esters for example 5-methyl-1,3-dioxolan-2-ylmethyl; and (1-6C)alkoxycarbonyloxyethyl esters for example 1-methoxycarbonyloxyethyl and may be formed at any carboxy group in the compounds of this invention.

An in-vivo hydrolysable ester of a compound of the formula (IA) or a pharmaceutically-acceptable salt thereof containing a hydroxy group or groups includes inorganic esters such as phosphate esters (including phosphoramidic cyclic esters) and α-acyloxyalkyl ethers and related compounds which as a result of the in-vivo hydrolysis of the ester breakdown to give the parent hydroxy group/s. In addition the sulphoximine residue may be derivatised by a convenient biologically labile group to give a derivative suitable for use as a solubilising pro-drug. Examples of α-acyloxyalkyl ethers include acetoxymethoxy and 2,2-dimethylpropionyloxymethoxy. A selection of in-vivo hydrolysable ester forming groups for hydroxy include (1-10C)alkanoyl, benzoyl, phenylacetyl and substituted benzoyl and phenylacetyl, (1-10C)alkoxycarbonyl (to give alkyl carbonate esters), di-(1-4C)alkylcarbamoyl and N-(di-(1-4C)alkylaminoethyl)-N-(1-4C)alkylcarbamoyl (to give carbamates), di-(1-4C)alkylaminoacetyl and carboxyacetyl. Examples of substituents on benzoyl and phenylacetyl include chloromethyl or aminomethyl, (1-4C)alkylaminomethyl and di-((1-4C)alkyl)aminomethyl, and morpholino or piperazino linked from a ring nitrogen atom via a methylene linking group to the 3- or 4-position of the benzoyl ring.

In addition a sulphoximine residue may be derivatised by a convenient biologically labile group to give a derivative suitable for use as a solubilising pro-drug.

Certain suitable in-vivo hydrolysable esters of a compound of the formula (IA) are described within the definitions listed in this specification, for example esters described by the definition (Rc2d), and some groups within (Rc2c). Suitable in-vivo hydrolysable esters of a compound of the formula (IA) are described as follows. For example, a 1,2-diol may be cyclised to form a cyclic ester of formula (PD1) or a pyrophosphate of formula (PD2) :

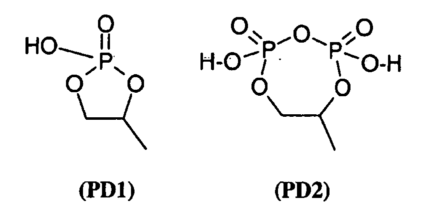

Particularly interesting are such cyclised pro-drugs when the 1,2-diol is on a (1-4C)alkyl chain linked to a carbonyl group in a substituent of formula Rc borne by a nitrogen atom in structures (TA1) or (TA2). Esters of compounds of formula (IA) wherein the HO-function/s in (PD1) and(PD2) are protected by (1-4C)alkyl, phenyl or benzyl are useful intermediates for the preparation of such pro-drugs.

Further in-vivo hydrolysable esters include phosphoramidic esters, and also compounds of formula (IA) in which any free hydroxy group, or sulfoxime group, independently forms a phosphoryl (npd is 1) or phosphiryl (npd is 0) ester of the formula (PD3) or (PS1), wherein npd is independently 0 or 1 for each oxo group :

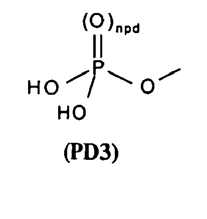

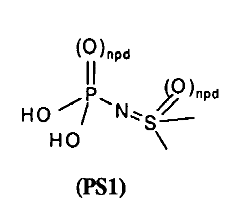

For the avoidance of doubt, phosphono is -P(O)(OH)₂; (1-4C)alkoxy(hydroxy)-phosphoryl is a mono-(1-4C)alkoxy derivative of -O-P(O)(OH)₂; and di-(1-4C)alkoxyphosphoryl is a di-(1-4C)alkoxy derivative of -O-P(O)(OH)₂.

Useful intermediates for the preparation of such esters include compounds containing a group/s of formula (PD3) in which either or both of the -OH groups in (PD3) is independently protected by (1-4C)alkyl (such compounds also being interesting compounds in their own right), phenyl or phenyl-(1-4C)alkyl (such phenyl groups being optionally substituted by 1 or 2 groups independently selected from (1-4C)alkyl, nitro, halo and (1-4C)alkoxy).

Thus, prodrugs containing groups such as (PD1), (PD2) and (PD3) may be prepared by reaction of a compound of formula (IA) containing suitable hydroxy group/s with a suitably protected phosphorylating agent (for example, containing a chloro or dialkylamino leaving group), followed by oxidation (if necessary) and deprotection. Prodrugs containing a group such as (PS1) may be obtained by analagous chemistry.

When a compound of formula (IA) contains a number of free hydroxy group, those groups not being converted into a prodrug functionality may be protected (for example, using a t-butyl-dimethylsilyl group), and later deprotected. Also, enzymatic methods may be used to selectively phosphorylate or dephosphorylate alcohol functionalities.

Other interesting in-vivo hydrolysable esters include, for example, those in which Rc is defined by, for example, R¹⁴C(O)O(1-6C)alkyl-CO- (wherein R¹⁴ is for example, benzyloxy-(1-4C)alkyl, or phenyl). Suitable substituents on a phenyl group in such esters include, for example, 4-(1-4C)piperazino-(1-4C)alkyl, piperazino-(1-4C)alkyl and morpholino-(1-4C)alkyl.

Where pharmaceutically-acceptable salts of an in-vivo hydrolysable ester may be formed this is achieved by conventional techniques. Thus, for example, compounds containing a group of formula (PD1), (PD2) and/or (PD3) may ionise (partially or fully) to form salts with an appropriate number of counter-ions. Thus, by way of example, if an in-vivo hydrolysable ester prodrug of a compound of formula (IA) contains two (PD3) groups, there are four HO-P- functionalities present in the overall molecule, each of which may form an appropriate salt (i.e. the overall molecule may form, for example, a mono-, di-, tri- or tetra-sodium salt).

The compounds of the present invention have a chiral centre at the C-5 position of the oxazolidinone ring. The pharmaceutically active enantiomer is of the formula (IC) wherein Q is

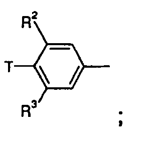

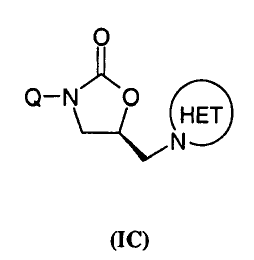

The present invention includes the pure enantiomer depicted above or mixtures of the 5R and 5S enantiomers, for example a racemic mixture. If a mixture of enantiomers is used, a larger amount (depending upon the ratio of the enantiomers) will be required to achieve the same effect as the same weight of the pharmaceutically active enantiomer. For example, the enantiomer depicted above is the 5(R) isomer when HET is 1,2,3- or 1,2,4-triazole or tetrazole.

Furthermore, some compounds of the formula (IA) may have other chiral centres, for example, certain sulfoxime compounds may be chiral at the sulfur atom. It is to be understood that the invention encompasses all such optical and diastereo-isomers, and racemic mixtures, that possess antibacterial activity. It is well known in the art how to prepare optically-active forms (for example by resolution of the racemic form by recrystallisation techniques, by chiral synthesis, by enzymatic resolution, by biotransformation or by chromatographic separation) and how to determine antibacterial activity as described hereinafter.

Furthermore, some compounds of the formula (IA), for example certain sulfoxime compounds may exist as cis- and trans- isomers. It is to be understood that the invention encompasses all such isomers, and mixtures thereof, that possess antibacterial activity.

The invention relates to all tautomeric forms of the compounds of the formula (I) that possess antibacterial activity.

It is also to be understood that certain compounds of the formula (IA) can exist in solvated as well as unsolvated forms such as, for example, hydrated forms. It is to be understood that the invention encompasses all such solvated forms which possess antibacterial activity.

It is also to be understood that certain compounds of the formula (IA) may exhibit polymorphism, and that the invention encompasses all such forms which possess antibacterial activity.

### Process section:

In a further aspect the present invention provides a process for preparing a compound of formula (IA) or a pharmaceutically-acceptable salt or an in-vivo hydrolysable ester thereof. It will be appreciated that during certain of the following processes certain substituents may require protection to prevent their undesired reaction. The skilled chemist will appreciate when such protection is required, and how such protecting groups may be put in place, and later removed.

For examples of protecting groups see one of the many general texts on the subject, for example, 'Protective Groups in Organic Synthesis' by Theodora Green (publisher: John Wiley & Sons).

Protecting groups may be removed by any convenient method as described in the literature or known to the skilled chemist as appropriate for the removal of the protecting group in question, such methods being chosen so as to effect removal of the protecting group with minimum disturbance of groups elsewhere in the molecule.

Thus, if reactants include, for example, groups such as amino, carboxy or hydroxy it may be desirable to protect the group in some of the reactions mentioned herein.

A suitable protecting group for an amino or alkylamino group is, for example, an acyl group, for example an alkanoyl group such as acetyl, an alkoxycarbonyl group, for example a methoxycarbonyl, ethoxycarbonyl or t-butoxycarbonyl group, an arylmethoxycarbonyl group, for example benzyloxycarbonyl, or an aroyl group, for example benzoyl. The deprotection conditions for the above protecting groups necessarily vary with the choice of protecting group. Thus, for example, an acyl group such as an alkanoyl or alkoxycarbonyl group or an aroyl group may be removed for example, by hydrolysis with a suitable base such as an alkali metal hydroxide, for example lithium or sodium hydroxide. Alternatively an acyl group such as a *t*-butoxycarbonyl group may be removed, for example, by treatment with a suitable acid as hydrochloric, sulphuric or phosphoric acid or trifluoroacetic acid and an arylmethoxycarbonyl group such as a benzyloxycarbonyl group may be removed, for example, by hydrogenation over a catalyst such as palladium-on-carbon, or by treatment with a Lewis acid for example boron tris(trifluoroacetate). A suitable alternative protecting group for a primary amino group is, for example, a phthaloyl group which may be removed by treatment with an alkylamine, for example dimethylaminopropylamine, or with hydrazine.

A suitable protecting group for a hydroxy group is, for example, an acyl group, for example an alkanoyl group such as acetyl, an aroyl group, for example benzoyl, or an arylmethyl group, for example benzyl. The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium or sodium hydroxide. Alternatively an arylmethyl group such as a benzyl group may be removed, for example, by hydrogenation over a catalyst such as palladium-on-carbon.

A suitable protecting group for a carboxy group is, for example, an esterifying group, for example a methyl or an ethyl group which may be removed, for example, by hydrolysis with a base such as sodium hydroxide, or for example a *t*-butyl group which may be removed, for example, by treatment with an acid, for example an organic acid such as trifluoroacetic acid, or for example a benzyl group which may be removed, for example, by hydrogenation over a catalyst such as palladium-on-carbon.

Resins may also be used as a protecting group.

The protecting groups may be removed at any convenient stage in the synthesis using conventional techniques well known in the chemical art.

A compound of the formula (IA), or a pharmaceutically-acceptable salt or an *in vivo* hydrolysable ester thereof, may be prepared by any process known to be applicable to the preparation of chemically-related compounds. Such processes, when used to prepare a compound of the formula (IA), or a pharmaceutically-acceptable salt or an *in vivo* hydrolysable ester thereof, are provided as a further feature of the invention and are illustrated by the following representative examples. Necessary starting materials may be obtained by standard procedures of organic chemistry (see, for example, Advanced Organic Chemistry (Wiley-Interscience), Jerry March). The preparation of such starting materials is described within the accompanying non-limiting Examples (in which, for example, 3,5-difluorophenyl, 3-fluorophenyl and (des-fluoro)phenyl containing intermediates may all be prepared by analogous procedures.

Alternatively, necessary starting materials are obtainable by analogous procedures to those illustrated which are within the ordinary skill of an organic chemist. Information on the preparation of necessary starting materials or related compounds (which may be adapted to form necessary starting materials) may also be found in the following Patent and Application Publications, the contents of the relevant process sections of which are hereby incorporated herein by reference :
WO99/02525; WO98/54161; WO97/37980; WO97/30981 (& US5,736,545); WO97/21708 (& US5,719,154); WO97/10223; WO97/09328; WO96/35691; WO96/23788; WO96/15130;
WO96/13502; WO95/25106 (& US5,668,286); WO95/14684 (& US5,652,238);
WO95/07271 (& US5,688,792); WO94/13649; WO94/01 110; WO93/23384 (& US5,547,950-& US 5,700,799); WO93/09103 (& US5,S65,571, US5,654,428, US5,654,435, US5,756,732 & US5,801,246);.US5,231,188; US5,247,090; US5,523,403; WO97/27188; WO97/30995;
WO97/31917; WO98/01447; WO98/01446; WO99/10342; WO99/10343; WO99/11642;
WO99/64416; WO99/64417 and GB99/03299;
European Patent Application Nos. 0,359,418 and 0,609,905; 0,693,491 A1 (& US5,698,574); 0,694,543 A1 (& AU 24985/95); 0,694,544 A1 (& CA 2,154,024); 0,697,412 A1 (& US5,529,998); 0,738,726 A1 (& AU 50735/96); 0,785,201 A1 (& AU 10123/97); German Patent Application Nos. DE 195 14 313 A1 (& US5,529,998); DE 196 01 264 A1 (& AU 10098/97); DE 196 01 265 A1 (& AU 10097/97); DE 196 04 223 A1 (& AU 12516/97); DE 196 49 095 A1 (& AU 12517/97).

The following Patent and Application Publications may also provide useful information and the contents of the relevant process sections are hereby incorporated herein by reference :
FR 2458547; FR 2500450(& GB 2094299, GB 2141716 & US 4,476,136); DE 2923295 (& GB 2028306, GB 2054575, US4,287,351, US4,348,393, US4,413,001, US4,43S,415 & US4,526,786), DE 3017499 (& GB 2053196, US4,346,102 & US4,372,967);
US4,705,799; European Patent Application Nos. 0,312,000; 0,127,902; 0,184,170; 0,352,781; 0,316,594.

Information on the preparation of necessary starting materials or related compounds (which may be adapted to form necessary starting materials) may also be found in WO 01/46185.

The skilled organic chemist will be able to use and adapt the information contained and referenced within the above references to obtain necessary starting materials.

In particular we refer to our PCT patent applications WO-99/64417 and WO-00/21960 wherein detailed guidance is given on convenient methods for preparing oxazolidinone compounds.

The present invention also provides that compounds of the formulae (IA) and pharmaceutically-acceptable salts and *in vivo* hydrolysable esters thereof, can be prepared by a process (a) to (h) as follows (wherein a variable sulfoximine/sulfimine substituent is designated by R and the other variables are as defined above unless otherwise stated); Q is phenylene substituted with R² and R³:
(a) (i) by modifying a substituent in, or introducing a new substituent into, the substituent group RT of HET of another compound of formula (IA) - for instance by (i) displacement of a functional group from a compound of formula (IA) by another functional group, (ii) by oxidation or (iii) reduction of a compound of formula (IA), by (iv) addition of a reagent to or (v) elimination of a reagent from a compound of formula (IA), by (vi) metathesis of a compound of formula (IA) into a modified compound of formula (IA), or by (vii) rearrangement of a compound of formula (IA) to an isomeric compound of formula (IA); or (a) (ii) by modifying a substituent in, or introducing a new substituent into, the group Q of another compound of formula (IA) - for instance by (i) displacement of a functional group from a compound of formula (IA) by another functional group, (ii) by oxidation or (iii) reduction of a compound of formula (IA), by (iv) addition of a reagent to or (v) elimination of a reagent from a compound of formula (IA), by (vi) metathesis of a compound of formula (IA) into a modified compound of formula (IA), or by (vii) rearrangement of a compound of formula (IA) to an isomeric compound of formula (IA) (Scheme 1 shows examples drawn from the range of suitable methods); or
**(b)** by reaction of a compound of formula (II) : 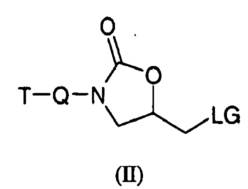 wherein LG is a displaceable group (which may be (i) generated in-situ, for example under Mitsunobu conditions, or (ii) preformed, such as chloro or mesylate) with a compound of the formula (III):

   HET (III)

   wherein HET is HET-H free-base form or HET- anion formed from the free base form (Scheme 2 shows examples drawn from the range of suitable methods); or
**(c)** by reaction of a compound of the formula (IV) :

   T-Q-LG1 (IV)

   wherein LG1 is an isocyanate, amine or urethane group with an epoxide of the formula (V)
   wherein Z is an isocyanate, amine or urethane group with an epoxide of the formula (V)
   wherein the epoxide group serves as a leaving group at the terminal C-atom and as a protected hydroxy group at the internal C-atom; or with a related compound of formula (VA) where the hydroxy group at the internal C-atom is conventionally protected e.g. with an acetyl group and where the leaving group Y at the terminal C-atom is a conventional leaving group e.g. a chloro- or mesyloxy-group (Scheme 3 shows examples drawn from a range of suitable methods); or 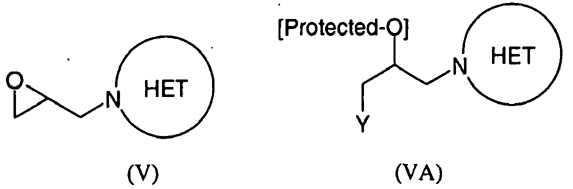
**(d)** by oxidation
   (i) with an aminating agent of a lower valent sulfur compound (VI), or an analogue thereof, which is suitable to give a T substituent as defined by (TA2), or a bi-, or tri-cyclic ring analogue of (VI) which is suitable to give a T substituent as defined by (TB); or
   (ii) with an oxygenating agent of a lower valent sulfur compound (VII), or an analogue thereof, which is suitable to give a T substituent as defined by (TA2), or a bi-, or tri-cyclic ring analogue of (VII) which is suitable to give a T substituent as defined by (TB); 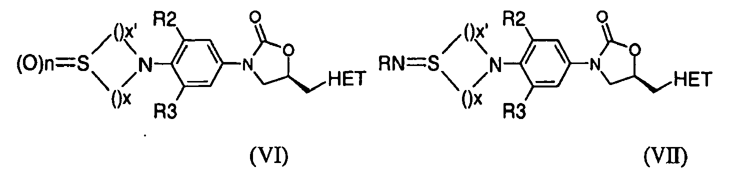
   where n = 0 or 1 and ()x and ()x' are chains of length x and x'. Suitable aminating agents include mesitylenesulfonyl hydroxylamine, sodium azide and polyphosphoric acid, and chloramine-T; suitable oxygenating agents include peracids and osmium tetroxide - amine N-oxide mixtures (Scheme 4 shows examples drawn from a range of suitable methods); or
**(e) (i)** by coupling, using catalysis by transition metals such as palladium(0), of a compound of formula (VIII): 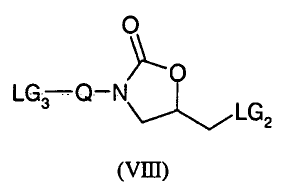 wherein LG2 is a group HET as hereinbefore defined, LG3 is a replaceable substituent - such as chloride, bromide, iodide, or trifluoromethylsulfonyloxy, with a compound of the formula (IX), or an analogue thereof, which is suitable to give a T substituent as defined by (TA1); in which the link is via an sp² carbon atom, or (TA2), or a bi- or tri-cyclic ring analogue of (IX) which is suitable to give a T substituent as defined by (TB); 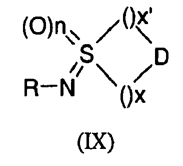 where n = 0 or 1 and ()x and ()x' are chains of length x and x'; D is NH or CH=C-LG4 where LG4 is a replaceable substituent such as chloride, bromide, iodide, or trifluoromethylsulfonyloxy, or (for instance under conditions of the Heck reaction) also hydrogen (Scheme 5 shows examples drawn from the range of suitable methods);
(e) (ii) by coupling, using catalysis by transition metals such as palladium(0), of a compound of formula (X): 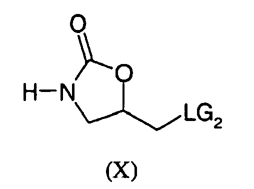 wherein LG2 is a group HET as hereinbefore defined, with a compound [Aryl]-LG4, where LG4 is a replaceable substituent such as chloride, bromide, iodide, or trifluoromethylsulfonyloxy, or an analogue thereof (Scheme 5 shows an example drawn from the range of suitable methods); or
**(f)** Where HET is 1,2,3-triazole there is the additional possibility by cycloaddition via the azide (wherein LG in (II) is azide), with a substituted acetylene or a masked acetylene (such as a vinyl sulfone, a nitroloefin, or an enamine, or a substituted cyclohexa-1,4-diene derivative (Scheme 2 shows examples drawn from the range of suitable methods)
**(g)** Where HET is 4-substituted 1,2,3-triazole there is the additional possibility of synthesis by reaction of a compound of formula (II) where LG = NH₂ (primary amine) with a compound of formula (XI), namely the arenesulfonylhydrazone of a methyl ketone that is further geminally substituted on the methyl group by two substituents (Y' and Y") capable of being eliminated from this initial, and the intermediate, substituted hydrazones as HY' and HY" (or as conjugate bases thereof) (Scheme 6 shows an example drawn from the range of suitable methods); 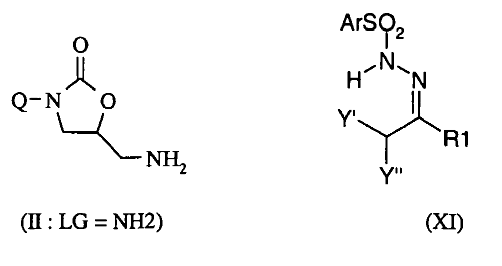
**(h)** by reduction of the carbon-carbon double bond of an unsaturated compound formed for instance by process (e) (i) in which the T substituent (as defined by (TA1)) is linked via an sp² carbon atom, to form the saturated analogue (Scheme 7 shows examples drawn from a range of suitable methods);
**(i)** and thereafter if necessary: (i) removing any protecting groups; (ii) forming a pharmaceutically-acceptable salt; (iii) forming an in-vivo hydrolysable ester.

**(a)** Methods for converting substituents into other substituents are known in the art. For example an alkylthio group may be oxidised to an alkylsulfinyl or alkysulfonyl group, a cyano group reduced to an amino group, a nitro group reduced to an amino group, a hydroxy group alkylated to a methoxy group, a hydroxy group thiomethylated to an arylthiomethyl or a heteroarylthiomethyl group (see, for example, Tet.Lett., 585, 1972), a carbonyl group converted to a thiocarbonyl group (eg. using Lawsson's reagent) or a bromo group converted to an alkylthio group.
**(b)(i)** Reaction (b)(i) (in which LG is initially hydroxy) is performed under Mitsunobu conditions, for example, in the presence of tri-n-butylphosphine and diethyl azodicarboxylate (DEAD) in an organic solvent such as THF, and in the temperature range 0°C - 60°C, but preferably at ambient temperature. Details of Mitsunobu reactions are contained in Tet. Letts., 31, 699, (1990); The Mitsunobu Reaction, D.L.Hughes, Organic Reactions, 1992, Vol.42, 335-656 and Progress in the Mitsunobu Reaction, D.L.Hughes, Organic Preparations and Procedures International, 1996, Vol.28, 127-164. The general method is illustrated in Scheme 2.
**(b)(ii)** Reactions (b)(ii) are performed conveniently in the presence of a suitable base such as, for example, an alkali or alkaline earth metal carbonate, alkoxide or hydroxide, for example sodium carbonate or potassium carbonate, or, for example, an organic amine base such as, for example, pyridine, 2,6-lutidine, collidine, 4-dimethylaminopyridine, triethylamine, morpholine or diazabicyclo-[5.4.0]undec-7-ene, the reaction is also preferably carried out in a suitable inert solvent or diluent, for example methylene chloride, acetonitrile, tetrahydrofuran, 1,2-dimethoxyethane, *N,N*-dimethylformamide, *N,N*-dimethylacetamide, *N*-methylpyrrolidin-2-one or dimethylsulfoxide at and at a temperature in the range 25-60°C.

When LG is chloro, the compound of the formula (II) may be formed by reacting a compound of the formula (II) wherein LG is hydroxy (hydroxy compound) with a chlorinating agent. For example, by reacting the hydroxy compound with thionyl chloride, in a temperature range of ambient temperature to reflux, optionally in a chlorinated solvent such as dichloromethane or by reacting the hydroxy compound with carbon tetrachloride/triphenyl phosphine in dichloromethane, in a temperature range of 0°C to ambient temperature. A compound of the formula (II) wherein LG is chloro or iodo may also be prepared from a compound of the formula (II) wherein LG is mesylate or tosylate, by reacting the latter compound with lithium chloride or lithium iodide and crown ether, in a suitable organic solvent such as THF, in a temperature range of ambient temperature to reflux

When LG is (1-4C)alkanesulfonyloxy or tosylate the compound (II) may be prepared by reacting the hydroxy compound with (1-4C)alkanesulfonyl chloride or tosyl chloride in the presence of a mild base such as triethylamine or pyridine.

When LG is a phosphoryl ester (such as PhO₂-P(O)-O-) or Ph₂-P(O)-O- the compound (II) may be prepared from the hydroxy compound under standard conditions.

If not commercially available, compounds of the formula (III) may be prepared by procedures which are selected from standard chemical techniques, techniques which are analogous to the synthesis of known, structurally similar compounds, or techniques which are analogous to the procedures described in the Examples. For example, standard chemical techniques are as described in Houben Weyl. The general method is illustrated in Scheme 2. **(c)** by reaction of T-Q-LG1 (IV) wherein LG1 is an amine, urethane, or isocyanate with an N-epoxypropyl hetercycle (V). Epoxides of the formula (V) may be prepared from the corresponding N-allylheterocycle of formula (XII): 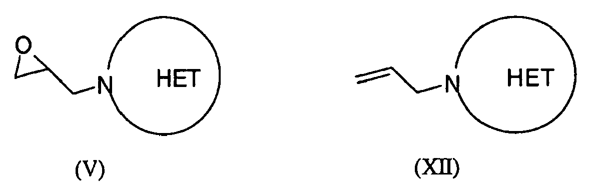 Certain such epoxide and alkene intermediates are novel and are provided as a further feature of the invention. Asymmetric epoxidation may be used to give the desired optical isomer. Compounds of formula (VA) may be obtained from epoxides of formula (V); alternatively compounds of formula (VA) may be used as precursors for epoxides of formula (V) according to the relative ease of synthesis in each case. The skilled chemist will appreciate that the epoxides of formula (V) and the compounds of formula (VA) are structurally equivalent and the choice between them will be made on the grounds of availability, convenience, and cost.

Furthermore, a similar reaction to reaction (c) may be performed in which Q-LG1 wherein LG1 is an amine group is reacted with the epoxide (V) (optionally in the presence of an organic base), and the product is reacted with, for example, phosgene to form the oxazolidinone ring.

Alternatively, a precursor of the group HET may be incorporated in place of the group HET in the epoxide of formula (V).

Such reactions and the preparation of starting materials in within the skill of the ordinary chemist with reference to the above-cited documents disclosing analogous reactions and preparations.

Compounds of the formula (II) wherein LG is hydroxy may be obtained as described in the references cited herein, for example, by reacting a compound T-Q-LG1 (IV) where LG1 is an amine, an isocyanate, or a urethane, especially a compound of the formula (IV, LG1 = NHCO₂R²¹) with a compound of formula (XIII): 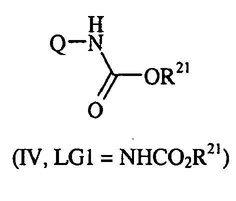 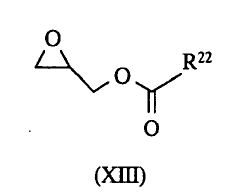 wherein R²¹ is (1-6C)alkyl or benzyl and R²² is (1-4C)alkyl or -S(O)ₙ(1-4C)alkyl where n is 0, 1 or 2. Preferably R²² is (1-4C)alkyl. Compounds of the formula (II), (IV), and (XIII) may be prepared by the skilled man, for example as described in International Patent Application Publication Nos. cited herein, the contents of which are hereby incorporated by reference, and by analogous processes.

Compounds of the formula T-Q-LG1 wherein LG1 is a urethane may be prepared by the skilled chemist, for example by analogous processes to those described in International Patent Application Publication Nos. WO 97/30995 and WO 97/37980. Compounds of the formula Q-LGI wherein LG1 is an isocyanate may be prepared by the skilled chemist, for example by analogous processes to those described in Walter A. Gregory et al in J. Med. Chem. 1990, 33, 2569-2578 and Chung-Ho Park et al in J. Med. Chem. 1992, 35, 1156-1165. The general method is illustrated in Scheme 3.

Compounds of the formula T-Q-LG1 wherein LG1 is an amine may be prepared by arylating an amine of formula (XIV), ()x and ()x' are chains of length x and x', which is suitable to give a T substituent as defined by (TA2), or a bi-, or tri-cyclic ring analogue of (XII) which is suitable to give a T substituent as defined by (TB); with a nitroarylhalide, such as 3,4-difluoronitrobenzene, and reducing the nitro-compound so produced to the corresponding amine. The thioether may be oxidized to a sulfimine or sulfoximine at any convenient stage of the synthesis. Examples of the way that such reactions can be employed in the overall synthesis in different orders according to convenience are shown in Scheme 3A.

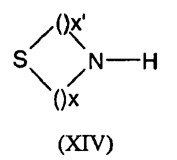

Suitable amine thioethers of the type shown in formula (XIV) may be synthesized by combination of the methods well-known in the art for the separate synthesis of cyclic amines and cyclic thioethers. Cyclic thioethers are readily available by reaction of sulfide anion with bifunctional alkylating agents, such as dibromides or bis-mesylates derived from diols. Certain cyclic thioethers are also available by cycloadditions, such as 1,3-dipolarcycloadditions of thiocarbonyl-ylids to olefins to give tetrahydrothiophenes and 1,4-cycloaddition of thiocarbonyl compounds to 1,3-dienes to give dihydrothiopyrans. Cyclic amines are available by similar reactions of analogous nitrogen compounds. In addition, cyclic amines are available by reduction of a wide range of imides and lactams. It will be apparent to the skilled chemist that the similar functional groups used to prepare the cyclic thioether and cyclic amine functionality may need to be selectively protected by methods known in the art.
**(d)** Convenient methods for aminating thioethers or sulfoxides are indicated in Michael Reggelin and Cornelia Zur in Synthesis, 2000, 1, 1-64. Further references include Reggelin et al, Tetrahedron Letters, 1992, 33 (46), 6959 - 6962; Reggelin et al, Tetrahedron Letters, 1992, 36 (33), 5885 - 5886; and Gage et al, Tetrahedron Letters, 2000, 41, 4301 - 4305.

For substrates containing nucleophilic nitrogen atoms such as tertiary arylamines it is advantageous to use an acidic reaction mixture such as sodium azide in polyphosphoric acid to reduce the amount of amination on nitrogen. Sufoximines may be made either by oxidizing thioethers first to the corresponding sufoxides and then to the sulfoximines or by oxidizing thioethers first to the corresponding sulfilimines (sulfimines) and then to the sulfoximine. The general method for aminating thioethers or sulfoxides and for oxidizing sulfimines is illustrated in Scheme 4. Convenient methods for the preparation of functionalised sulfilimines and sulfoximines include those in which a sulfilimine or sulfoximine is (i) alkylated, for instance by reductive amination using aldehydes, (ii) acylated for instance using acid chlorides in pyridine, or (iii) arylated, for instance by palladium coupling with (hetero)aryl halides or by cyclisation and heteroaromatisation of an acyclic substituent on the sulfoximine N. The general method for refunctionalizing sulfimines or sulfoximines in the final step is also illustrated in Scheme 4.
**(e) (i)** The transition metal catalysed coupling reaction to form a C-C or N-C bond from the corresponding aryl derivatives and the cyclic sulfoximines and sulfimines is performed under conventional conditions (see for instance J.K. Stille, Angew. Chem. Int. Ed. Eng., 1986, 25, 509-524; N. Miyaura and A. Suzuki, Chem. Rev., 1995, 95, 22457-2483; D. Baranano, G. Mann, and J.F. Hartwig, Current Org. Che., 1997,1,287-305; S.P. Stanforth, Tetrahedron, 1998, 54, 263-303). The cyclic sulfoxides and sulfimines used in reaction (e) (i) may be obtained by oxidation of the corresponding cyclic aminothioethers described for (c) according to the methods analogous to those of reaction (d). The general method is illustrated in Scheme 5.
**(e) (ii)** The reaction e (ii) may be conveniently carried out under the conditions described Tetrahedron Letters (2001), 42(22), 3681-3684, or in the analogous conventional conditions described in the above mentioned literature. In such a procedure a preferred variation of LG4 may be bromine.
**(f)** The cycloaddition-cycloreversion reaction to form 1,2,3 triazoles from the corresponding azide is performed under conventional Diels-Alder reaction conditions. The method is illustrated in Scheme 2.
   Compounds of the formula (II) wherein LG is azide may be obtained as described in the references cited herein (particularly in the section proceeding the discussion of protecting groups), for example from the corresponding compounds in which LG is hydroxy or mesylate.
**(g)** The reaction of amines of formula (II, LG = NH2) with arenesulfonyl hydrazones to form 1,2,3 triazoles may be carried out as described in the literature (Sakai, Kunikazu; Hida, Nobuko; Kondo, Kiyosi. Reactions of α-polyhalo ketone tosylhydrazones with sulfide ion and primary amines. Cyclization to 1,2,3-thiadiazoles and 1,2,3-triazoles. Bull. Chem. Soc. Jpn. (1986), 59(1), 179-83; Sakai, Kunikazu; Tsunemoto, Daiei; Kobori, Takeo; Kondo, Kiyoshi; Hida, Nobuko. 1,2,3-Trihetero 5-membered heterocyclic compounds, EP 103840 A2 19840328). The leaving groups Y, Y' may be chloro or any other group capable of being eliminated from the arenesulfonyl hydrazone during the reaction with the amine. The skilled chemist will also appreciate that a similar reaction may be used to produce other substituted triazoles suitable for incorporation into related processes such as reaction with compounds of formula (IV) in process (c).
**(h)** The reduction of a compound formed by process (e) in which the T substituent (as defined by (TA1)) is linked via an sp² carbon atom, to form the saturated analogue, may be performed using methods from the standard range of hydrogenations. For example, a dihydrothiopyran may be reduced to produce the tetrahydrothiopyran analogue.

The following Schemes illustrate process chemistry which allows preparation of compounds of the formula (IA); wherein A and R are values suitable to provide the compounds of formula (IA) defined herein. The Schemes may be genericised by the skilled man to apply to compounds within the present specification which are not specifically illustrated in the Schemes (for example to HET as a 6-membered ring as defined herein).

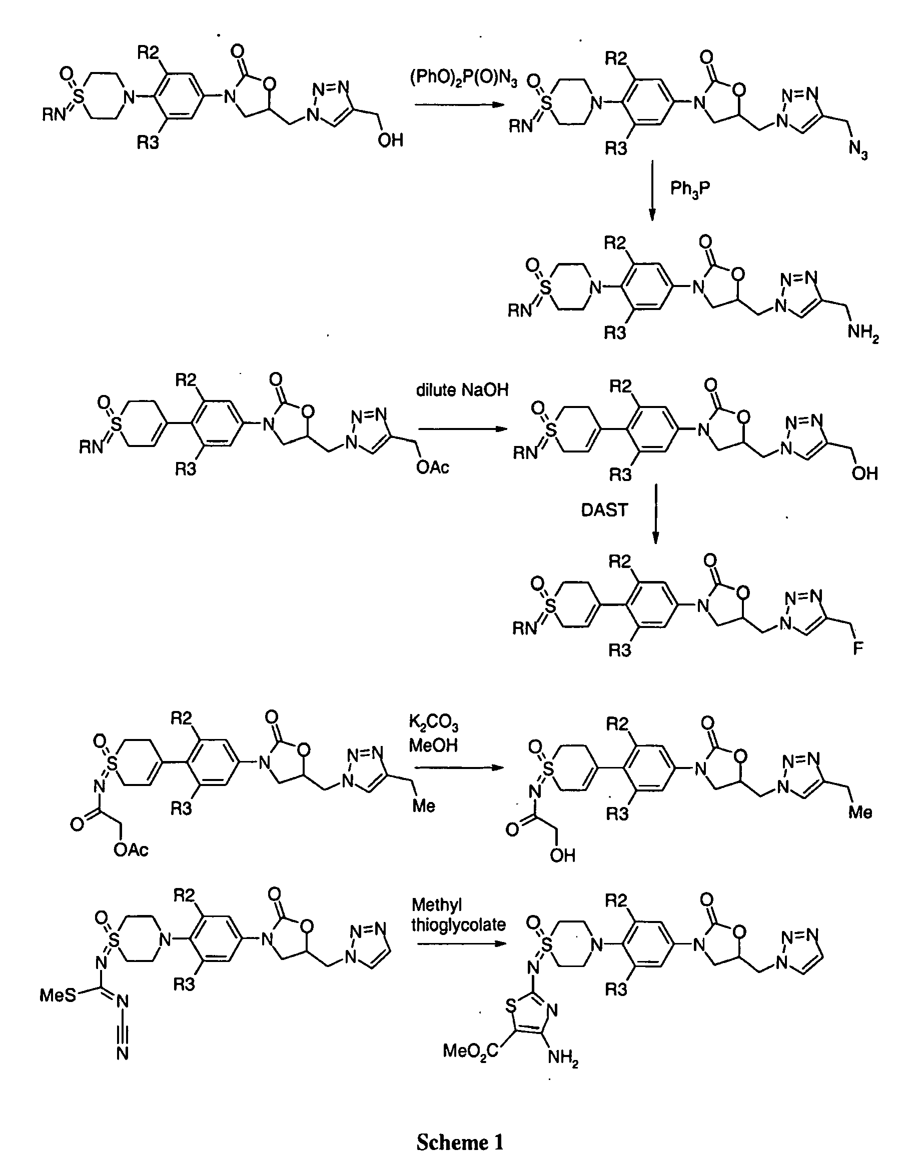

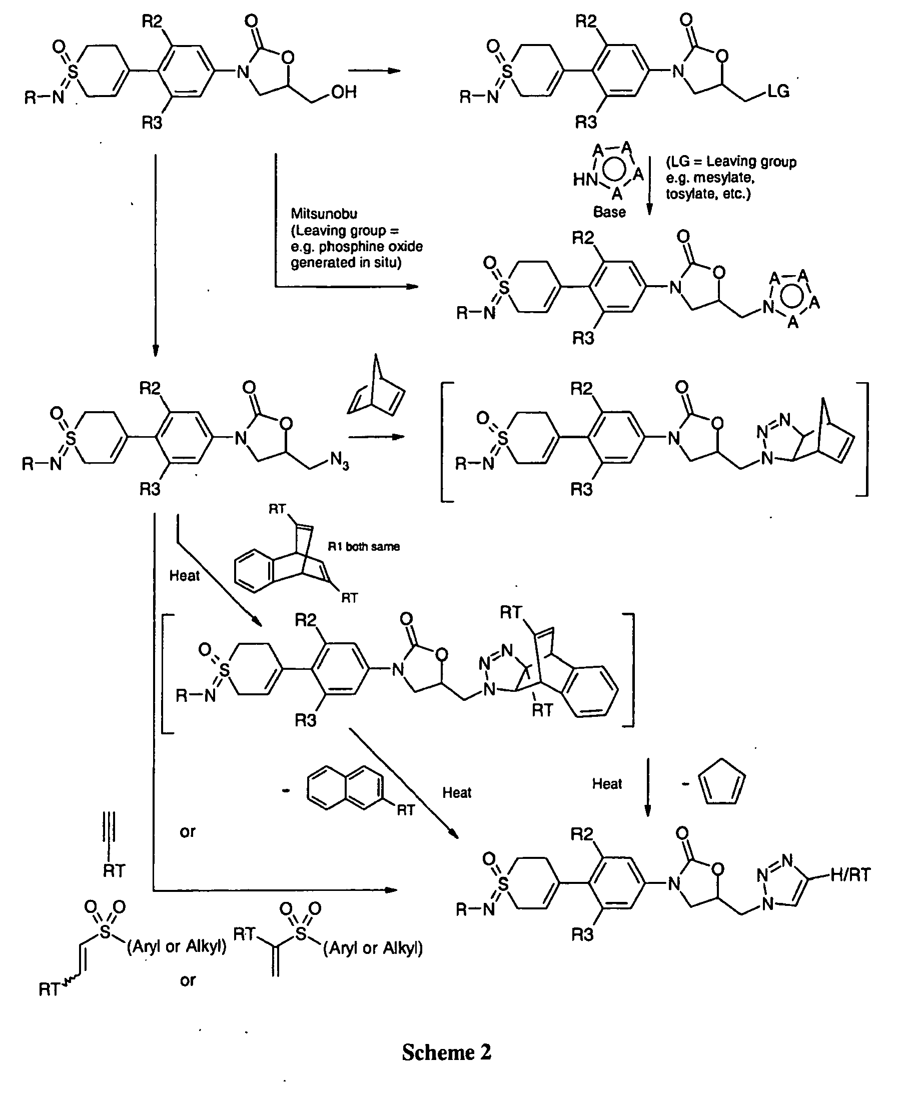

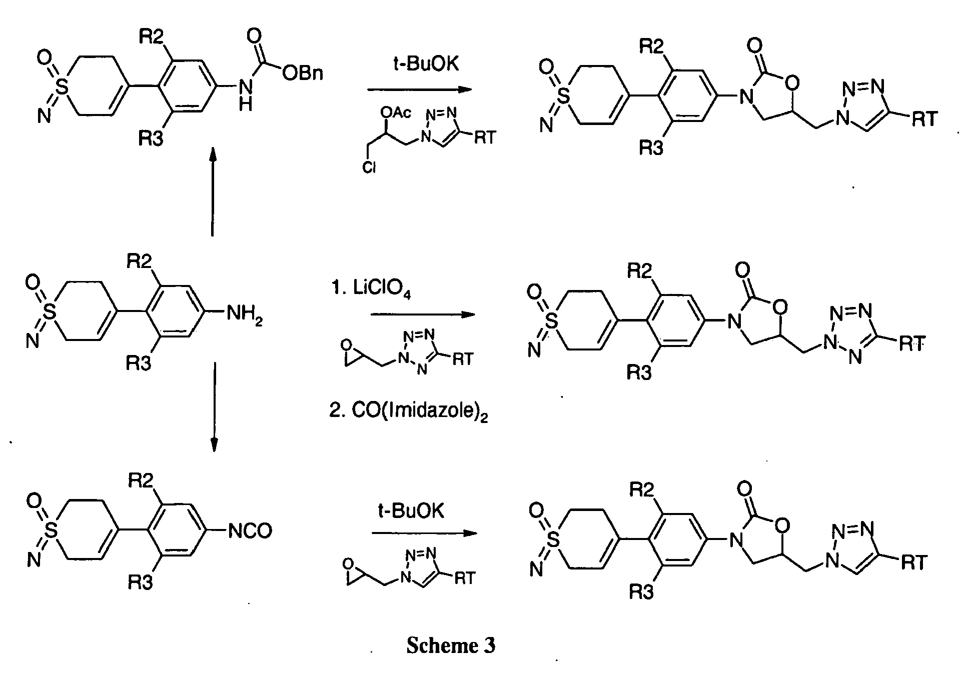

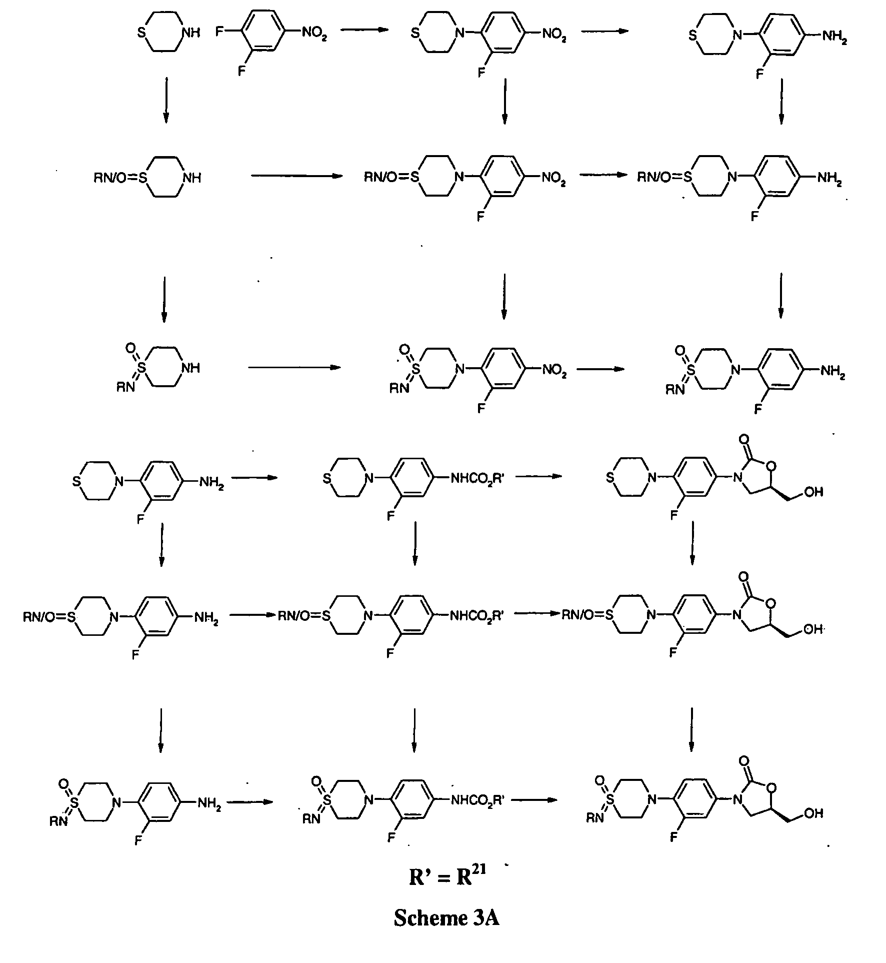

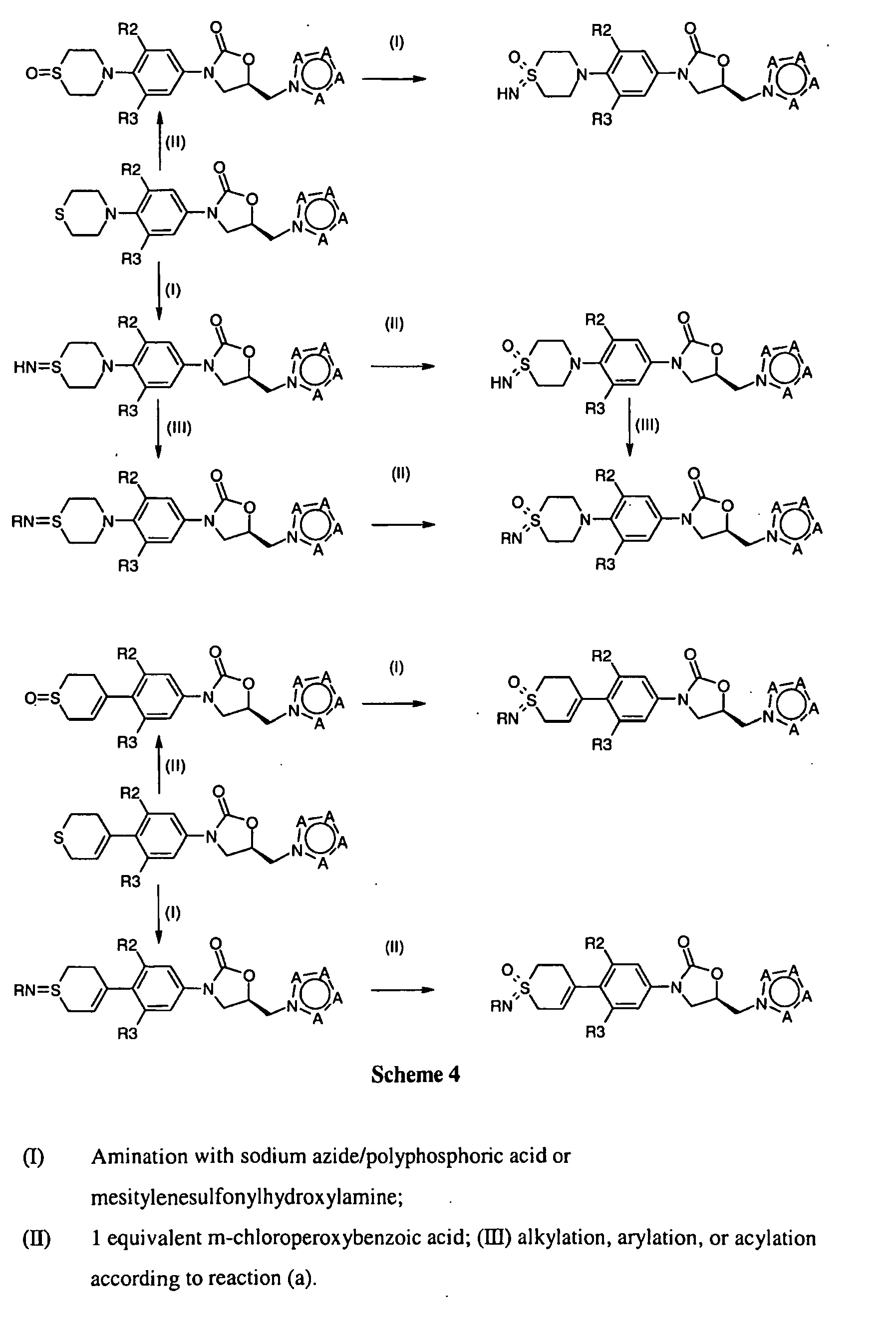

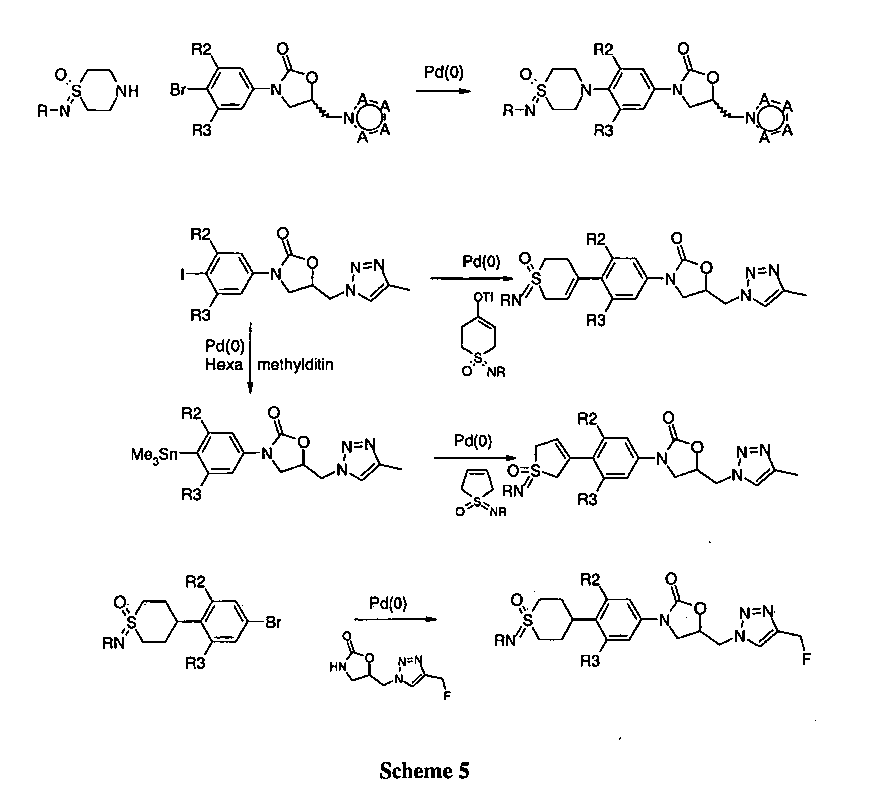

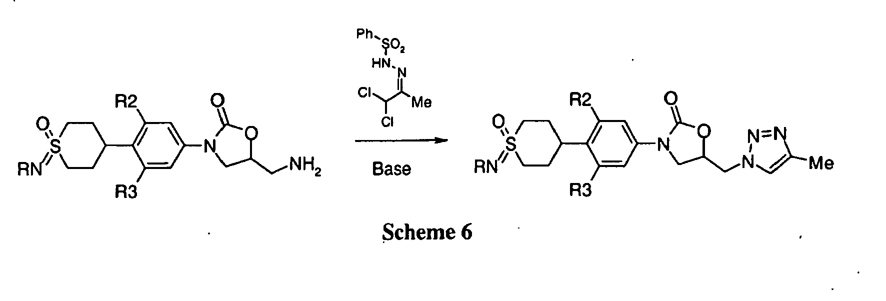

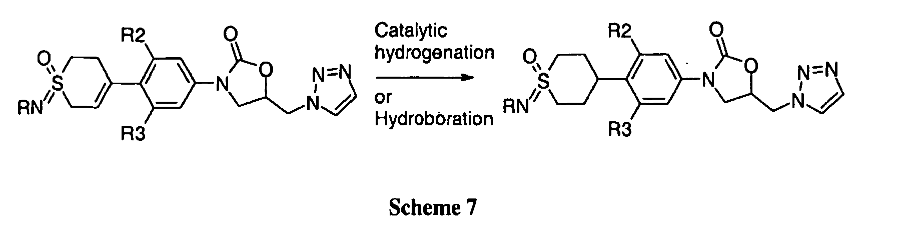

The removal of any protecting groups, the formation of a pharmaceutically-acceptable salt and/or the formation of an *in vivo* hydrolysable ester are within the skill of an ordinary organic chemist using standard techniques. Furthermore, details on the these steps, for example the preparation of in-vivo hydrolysable ester prodrugs has been provided in the section above on such esters, and in certain of the following non-limiting Examples.

Certain novel intermediates utilised in the above processes are provided as a further feature of the invention.

Convenient methods for functionalised sulfilimines and sulfoximines include those in which a sulfilimine or sulfoximine is (i) alkylated, (ii) acylated or (iii) arylated.
A detailed review of sulfoximine chemistry is provided by Michael Reggelin and Cornelia Zur in Synthesis, 2000, 1, 1-64. Further references include Reggelin et al, Tetrahedron Letters, 1992, 33 (46), 6959 - 6962; Reggelin et al, Tetrahedron Letters, 1992, 36 (33), 5885 - 5886; and Gage et al, Tetrahedron Letters, 2000, 41, 4301 - 4305.

General guidance on reaction conditions and reagents may be obtained in Advanced Organic Chemistry, 4^{th} Edition, Jerry March (publisher: J.Wiley & Sons), 1992. Necessary starting materials may be obtained by standard procedures of organic chemistry, such as described in this process section, in the Examples section or by analogous procedures within the ordinary skill of an organic chemist. Certain references are also provided (see above) which describe the preparation of certain suitable starting materials, for particular example see International Patent Application Publication No. WO 97/37980, the contents of which are incorporated here by reference. Processes analogous to those described in the references may also be used by the ordinary organic chemist to obtain necessary starting materials.

Methods for converting substituents into other substituents are known in the art. For example an alkylthio group may be oxidised to an alkylsulfinyl or alkylsulfonyl group, a cyano group reduced to an amino group, a nitro group reduced to an amino group, a hydroxy group alkylated to a methoxy group, a hydroxy group converted to an arylthiomethyl or a heteroarylthiomethyl group (see, for example, Tet.Lett., 585, 1972), a carbonyl group converted to a thiocarbonyl group (eg. using Lawsson's reagent) or a bromo group converted to an alkylthio group.

Further examples of converting substituents into other substituents are contained in the accompanying non-limiting Examples.

Certain compounds may be prepared by the skilled chemist, for example as described in International Patent Application Publication Nos. WO95/07271, WO97/27188, WO 97/30995, WO 98/01446 and WO 98/01447, the contents of which are hereby incorporated by reference, and by analogous processes.

If not commercially available, compounds may be prepared by procedures which are selected from standard chemical techniques, techniques which are analogous to the synthesis of known, structurally similar compounds, or techniques which are analogous to the procedures described in the Examples. For example, standard chemical techniques are as described in Houben Weyl, Methoden der Organische Chemie, E8a, Pt.I (1993), 45-225, B.J.Wakefield (for isoxazoles) and E8c, Pt.I (1994), 409-525, U.Kraatz (for 1,2,4-oxadiazoles). Also, for example, 3-hydroxyisoxazole may be prepared by cyclisation of CH≡ C-CO-NHOH (prepared from CH≡C-CO-O-(1-4C)alkyl) as described in Chem.Pharm.Bull.Japan, 14, 92, (1966).

The removal of any protecting groups, the formation of a pharmaceutically-acceptable salt and/or the formation of an *in vivo* hydrolysable ester are within the skill of an ordinary organic chemist using standard techniques. Furthermore, details on the these steps, for example the preparation of in-vivo hydrolysable ester prodrugs has been provided in the section above on such esters, and in certain of the following non-limiting Examples.

When an optically active form of a compound of the formula (IA) is required, it may be obtained by carrying out one of the above procedures using an optically active starting material (formed, for example, by asymmetric induction of a suitable reaction step), or by resolution of a racemic form of the compound or intermediate using a standard procedure, or by.chromatographic separation of diastereoisomers (when produced). Enzymatic techniques may also be useful for the preparation of optically active compounds and/or intermediates.

Similarly, when a pure regioisomer of a compound of the formula (IA) is required, it may be obtained by carrying out one of the above procedures using a pure regioisomer as a starting material, or by separation of a mixture of the regioisomers or intermediates using a standard procedure.

According to a further feature of the invention there is provided a compound of the formula (IA), or a pharmaceutically-acceptable salt, or in-vivo hydrolysable ester or amide thereof for use in a method of treatment of the human or animal body by therapy.

According to a further feature of the present invention there is provided a method for producing an antibacterial effect in a warm blooded animal, such as man, in need of such treatment, which comprises administering to said animal an effective amount of a compound of the present invention, or a pharmaceutically-acceptable salt, or in-vivo hydrolysable ester thereof.

The invention also provides a compound of the formula (IA), or a pharmaceutically-acceptable salt, or in-vivo hydrolysable ester thereof, for use as a medicament, and for use as an antibacterial agent; and the use of a compound of the formula (IA) of the present invention, or a pharmaceutically-acceptable salt, or in-vivo hydrolysable ester thereof, in the manufacture of a medicament for use in the production of an antibacterial effect in a warm blooded animal, such as man.

In order to use a compound of the formula (IA), an in-vivo hydrolysable ester or a pharmaceutically-acceptable salt thereof, including a pharmaceutically-acceptable salt of an in-vivo hydrolysable ester, (hereinafter in this section relating to pharmaceutical composition "a compound of this invention") for the therapeutic (including prophylactic) treatment of mammals including humans, in particular in treating infection, it is normally formulated in accordance with standard pharmaceutical practice as a pharmaceutical composition.

Therefore in another aspect the present invention provides a pharmaceutical composition which comprises a compound of the formula (IA), an in-vivo hydrolysable ester or a pharmaceutically-acceptable salt thereof, including a pharmaceutically-acceptable salt of an in-vivo hydrolysable ester, and a pharmaceutically-acceptable diluent or carrier.

The pharmaceutical compositions of this invention may be administered in standard manner for the disease condition that it is desired to treat, for example by oral, rectal, topical or parenteral administration. For these purposes the compounds of this invention may be formulated by means known in the art into the form of, for example, tablets, capsules, aqueous or oily solutions or suspensions, (lipid) emulsions, dispersible powders, suppositories, ointments, creams, aerosols (or sprays), drops and sterile injectable aqueous or oily solutions or suspensions.

In addition to the compounds of the present invention the pharmaceutical composition of this invention may also contain or be co-administered (simultaneously, sequentially or separately) with one or more known drugs selected from other clinically useful antibacterial agents (for example, β-lactams or aminoglycosides) and/or other anti-infective agents (for example, an antifungal triazole or amphotericin). These may include carbapenems, for example meropenem or imipenem, to broaden the therapeutic effectiveness. Compounds of this invention may also contain or be co-administered with bactericidal/permeability-increasing protein (BPI) products or efflux pump inhibitors to improve activity against gram negative bacteria and bacteria resistant to antimicrobial agents.

A suitable pharmaceutical composition of this invention is one suitable for oral administration in unit dosage form, for example a tablet or capsule which contains between 1mg and 1g of a compound of this invention, preferably between 100mg and 1g of a compound. Especially preferred is a tablet or capsule which contains between 50mg and 800mg of a compound of this invention, particularly in the range 100mg to 500mg.

In another aspect a pharmaceutical composition of the invention is one suitable for intravenous, subcutaneous or intramuscular injection, for example an injection which contains between 0.1% w/v and 50% w/v (between lmg/ml and 500mg/ml) of a compound of this invention.

Each patient may receive, for example, a daily intravenous, subcutaneous or intramuscular dose of 0.5 mgkg⁻¹ to 20 mgkg⁻¹ of a compound of this invention, the composition being administered 1 to 4 times per day. In another embodiment a daily dose of 5 mgkg⁻¹ to 20 mgkg⁻¹ of a compound of this invention is administered. The intravenous, subcutaneous and intramuscular dose may be given by means of a bolus injection. Alternatively the intravenous dose may be given by continuous infusion over a period of time. Alternatively each patient may receive a daily oral dose which may be approximately equivalent to the daily parenteral dose, the composition being administered 1 to 4 times per day. A pharmaceutical composition to be dosed intravenously may contain advantageously (for example to enhance stability) a suitable bactericide, antioxidant or reducing agent, or a suitable sequestering agent.

In the above other, pharmaceutical composition, process, method, use and medicament manufacture features, the alternative and preferred embodiments of the compounds of the invention described herein also apply.

### Antibacterial Activity :

The pharmaceutically-acceptable compounds of the present invention are useful antibacterial agents having a good spectrum of activity in vitro against standard Gram-positive organisms, which are used to screen for activity against pathogenic bacteria. Notably, the pharmaceutically-acceptable compounds of the present invention show activity against enterococci, pneumococci, methicillin resistant strains of S.aureus and coagulase negative staphylococci, haemophilus and moraxella strains. The antibacterial spectrum and potency of a particular compound may be determined in a standard test system.

The (antibacterial) properties of the compounds of the invention may also be demonstrated and assessed *in-vivo* in conventional tests, for example by oral and/or intravenous dosing of a compound to a warm-blooded mammal using standard techniques.

The following results were obtained on a standard *in-vitro* test system. The activity is described in terms of the minimum inhibitory concentration (MIC) determined by the broth-dilution technique with an inoculum size of 5x10⁴ CFU/spot. Typically, compounds are active in the range 0.01 to 256 µg/ml.

Staphylococci were tested in broth using an inoculum of 5x 10⁴ CFU/spot and an incubation temperature of 37°C for 16-24 hours.

Streptococci were tested in Mueller-Hinton broth supplemented with 2.5% clarified lake horse blood with an innoculum of 10⁴ CFU/well and an incubation temperature of 37°C aerobically for 24 hours.
Fastidious Gram negative organisms were tested in Mueller-Hinton broth supplemented with hemin and NAD, grown aerobically for 24h at 37°C, and with an innoculum of 5x10⁴ CFU/well.

| Organism | | MIC (µg/ml) Example 2 |
|---|---|---|
| Staphylococcus aureus: | | |
| | MSQS | 1 |
| | MRQR | 8 |
| Streptococcus pneumoniae | | 2 |
| Streptococcus pyogenes | | 2 |
| Haemophilus influenzae | | 8 |
| Moraxella catarrhalis | | 8 |

| | | |
|---|---|---|
| MSQS = methicillin sensitive and quinolone sensitive | | |
| MRQR = methincillin resistant and quinolone resistant | | |

Certain intermediates and/or Reference Examples described hereinafter within the scope of the invention may also possess useful activity, and are provided as a further feature of the invention.

The invention is now illustrated but not limited by the following Examples in which unless otherwise stated :-
i) evaporations were carried out by rotary evaporation in vacuo and work-up procedures were carried out after removal of residual solids by filtration;
(ii) operations were carried out at ambient temperature, that is typically in the range 18-26°C and in air unless otherwise stated, or unless the skilled person would otherwise work under an inert atmosphere; where unspecified, temperatures are quoted in °C;
(iii) column chromatography (by the flash procedure) was used to purify compounds and was performed on Merck Kieselgel silica (Art. 9385) unless otherwise stated;
(iv) yields are given for illustration only and are not necessarily the maximum attainable;
(v) the structure of the end-products of the invention were generally confirmed by NMR and mass spectral techniques [proton magnetic resonance spectra were generally determined in DMSO-D6 unless otherwise stated using a Varian Gemini 2000 spectrometer operating at a field strength of 300 MHz, or a Bruker AM250 spectrometer operating at a field strength of 250 MHz; chemical shifts are reported in parts per million downfield from tetramethysilane as an internal standard (δ scale) and peak multiplicities are shown thus: s, singlet; d, doublet; AB or dd, doublet of doublets; t, triplet, m, multiplet; fast-atom bombardment (FAB) mass spectral data were generally obtained using a Platform spectrometer (supplied by Micromass) run in electrospray and, where appropriate, either positive ion data or negative ion data were collected];
(vi) each intermediate was purified to the standard required for the subsequent stage and was characterised in sufficient detail to confirm that the assigned structure was correct; purity was in general assessed by HPLC, TLC, infra-red (IR), MS or NMR analysis; and identity was determined by IR, MS or NMR spectroscopy as appropriate; and
(vii) in which the following abbreviations may be used :-
   ® is a Trademark; DMF is N,N-dimethylformamide; DMA is N,N-dimethylacetamide;
   TLC is thin layer chromatography; HPLC is high pressure liquid chromatography;
   MPLC is medium pressure liquid chromatography; DMSO is dimethylsulfoxide;
   DMSO-d6 is deuterated DMSO;
   CDCl₃ is deuterated chloroform; MS is mass spectroscopy; ESP is electrospray;EI is electron impact; CI is chemical ionisation; APCI is atmospheric pressure chemical ionisation; THF is tetrahydrofuran; TFA is trifluoroacetic acid; NMP is N-methylpyrrolidone;
   HOBT is 1-hydroxy-benzotriazole; EtOAc is ethyl acetate; MeOH is methanol;
   phosphoryl is (HO)₂-P(O)-O-; phosphiryl is (HO)₂-P-O-; EDC is 1-(3-dimethylaminopropyl)-3-ethylcarbodiimide (hydrochloride); PTSA is paratoluenesulfonic acid.

### Examples

### Example 1: (5R)-3-[3-Fluoro-4-(1RS-1-imino-1-oxo-3,6-dihydrothiopyran-4-yl)-phenyl]-5-(1H-1,2,3-triazol-1-ylmethyl)-2-oxazolidinone

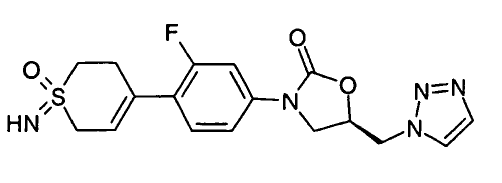

(5*R*)-3-[3-Fluoro-4-(*1RS*-1-oxo-3,6-dihydrothiopyran-4-yl)-phenyl]-5-(1*H*-1,2,3-triazol-1-ylmethyl)-2-oxazolidinone (0.951 g, 2.5 mmol) was dissolved/ suspended in dichloromethane (10 ml) at ambient temperature. *O*-mesitylenesulfonylhydroxylamine (0.68 g, 3.2 mmol, see Synthesis, 1972, 140), in dichloromethane (10 ml) was added dropwise, and the mixture stirred at ambient temperature for 18 hours. The solvent was evaporated in vacuo and the reaction mixture taken up in methane) (5 ml). The resulting precipitate was collected by filtration and subjected to chromatography on silica gel. with a gradient of 2-20% methanol in dichloromethane to give the desired product (220 mg) as free base.
MS (APCI): 392 (MH⁺) for C₁₇H₁₈FN₅O₃S
¹H-NMR (DMSO-d₆) δ: 2.89 (m, 2H); 3.20 (m, 2H); 3.83 (brs, 3H); 3.92 (dd, 1H); 4.26 (dd, 1H); 4.86 (m, 2H); 5.17 (m, 1H); 5.81 (m, 1H); 7.28 (dd, 1H); 7.38 (dd, 1H); 7.45 (dd, 1H); 7.79 (s, 1H); 8.19 (s, 1H).

The intermediates for this compound were prepared as follows:

### (5R)-3-[3-Fluoro-4-(1RS-1-oxo-3,6-dihydrothiopyran-4-yl)-phenyl]-5-(1H-1,2,3-triazol-1-ylmethyl)-2-oxazolidinone

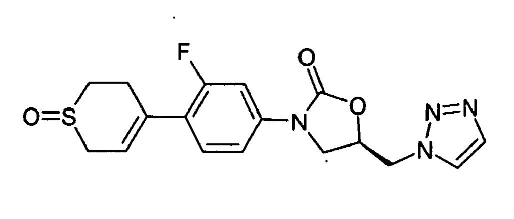

(5*R*)-3-[4-(3,6-dihydro-2*H*-thiopyran-4-yl)-3-fluorophenyl]-5-(1*H*-1,2,3-triazol-1-ylmethyl)-2-oxazolidinone (1.25 g, 3.5 mmol) was stirred in a mixture of methanol and ethyl acetate (1:1, 50 ml) at ambient temperature. Sodium periodate (0.93 g, 4.3 mmol) in water (10 ml) was added dropwise, and it was stirred for 18 hours. Precipitated salts were removed by filtration and solvents were removed under vacuum. The residue was chromatographed on silica gel, washing with 25% acetone in dichloromethane, then eluting with 5 to 7% methanol in dichloromethane to give the title product (1.152 g).
MS (ESP): 377 (MH⁺) for C₁₇H₁₇FN₄O₃S
¹H-NMR (DMSO-d₆) δ: 2.57 (m, 1H); 2.91 (m, 1H); 2.97 (m, 1H); 3.13 (m, 1H); 3.39 (m, 1H); 3.67 (m, 1H); 3.92 (dd, 1H); 4.27 (dd, 1H); 4.86 (m, 2H); 5.17 (m, 1H); 5.84 (m, 1H); 7.28 (dd, 1H); 7.39 (dd, 1H); 7.45 (dd, 1H); 7.79 (d, 1H);. 8.20 (d, 1H).

### (5R)-3-[4-(3,6-dihydro-2H-thiopyran-4-yl)-3-fluorophenyl]-5-(1H-1,2,3-triazol-1-ylmethyl)-2-oxazolidinone

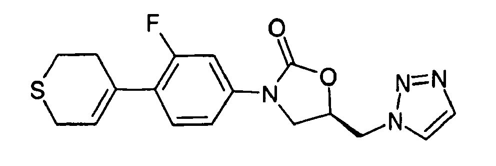

(*5R*)-3-[4-(3,6-dihydro-2*H*-thiopyran-4-yl)-3-fluorophenyl]-5-azidomethyl-2-oxazolidinone (2 g, 5.7 mmol) was dissolved in dioxane (10 ml). Bicyclo[2.2.1]hepta-2,5-diene (3.1 ml, 28.7 mmol) was added and it was refluxed under nitrogen for 18 hours. The solvent was evaporated in vacuo and the residue subjected to chromatography on silica gel eluting with 25% ethylacetate in dichloromethane to give the title compound (1.51 g).
MS (ESP): 361 (MH⁺) for C₁₇H₁₇FN₄O₂S
¹H-NMR (DMSO-d₆) δ: 2.56 (m, 2H); 2.83 (dd, 2H); 3.31 (m, 2H); 3.91 (dd, 1H); 4.26 (dd, 1H); 4.86 (m, 2H); 5.17 (m, 1H); 6.06(m, 1H); 7.25 (dd, 1H); 7.33 (dd, 1H); 7.42 (dd, 1H); 7.78 (d, 1H); 8.19 (d, 1H).

### (5R)-3-[4-(3,6-dihydro-2H-thiopyran-4-yl)-3-fluorophenyl]-5-azidomethyl-2-oxazolidinone

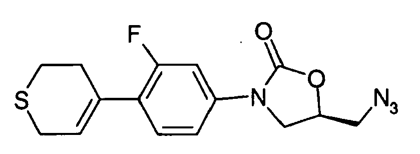

Methanesulfonic acid (*5R*)-3-[4-(3,6-dihydro-2*H*-thiopyran-4-yl)-3-fluorophenyl]-2-oxo-oxazolidin-5-ylmethyl ester (8 g, 19.7 mmol) and sodium azide (4 g, 61.5 mmol) were heated in N,N-dimethylformamide (75 ml) at 80°C for 2 hours. It was cooled to room temperature, diluted with ethyl acetate, washed with potassium phosphate buffer (pH 7) and with water and dried over sodium sulfate. After evaporation of the solvent the title product was obtained as a brown oil (-7 g, crude).
¹H-NMR (DMSO-d₆) δ: 2.56 (m, 2H); 2.83 (dd, 2H); 3.31 (m, 2H); 3.71 (dd, 1H); 3.80 (dd, 1H); 3.81 (dd, 1H); 4.17 (dd, 1H); 4.92 (m, 1H); 6.06(m, 1H); 7.34 (m, 2H); 7.50 (m, 1H). (No MS)

### Methanesulfonic acid (5R)-3-[4-(3,6-dihydro-2H-thiopyran-4-yl)-3-fluorophenyl]-2-oxo-oxazolidin-5-ylmethyl ester

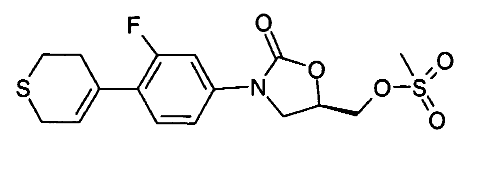

(*5R*)-3-[4-(3,6-dihydro-2*H*-thiopyran-4-yl)-3-fluorophenyl]-5-hydroxymethyl-2-oxazolidinone (14 g, 45.3 mmol) was dissolved in dichloromethane (300 ml) and triethylamine (8.8 ml, 63.3 mmol) was added. It was cooled to -20°C and methanesulfonyl chloride (4.22 ml, 54.4 mmol), dissolved in dichloromethane (50 ml), was added dropwise. The reaction mixture was allowed to warm to room temperature and was quenched with potassium phosphate buffer (pH 7). Dichloromethane was removed under vacuum and it was extracted with ethyl acetate, washed with water and dried over magnesium sulfate. The title compound (16.9 g) was precipitated from dichloromethane by addition of hexane.
¹H-NMR (DMSO-d₆) δ: 2.56 (m, 2H); 2.83 (dd, 2H); 3.28 (s, 3H); 3.32 (m, 2H); 3.85 (dd, 1H); 4.21 (dd, 1H); 4.48 (dd, 1H); 4.53 (dd, 1H); 5.04 (m, 1H); 6.07 (m, 1H); 7.33 (dd, 1H); 7.36 (dd, 1H); 7.50 (dd, 1H). (No MS)

### (5R)-3-[4-(3,6-dihydro-2H-thiopyran-4-yl)-3-fluorophenyl]-5-hydroxymethyl-2-oxazolidinone

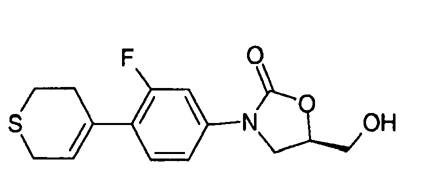

4-(2-Fluoro-4-benzyloxycarbonylaminophenyl)-3,6-dihydro-2*H*-thiopyran (15.3 g, 44.6 mM) was dissolved on dry tetrahydrofuran (175ml) and stirred under nitrogen at -70.
*n*-Butyllithium (1.6M in hexanes, 30ml, 175 mM) was run in over 20 minutes, keeping the temperature below -60°, and the mixture then stirred a further 10 minutes at -70°. A solution of (R)-glycidyl butyrate (6.42 g, 44.62 mM) dissolved in dry tetrahydrofuran (10 ml) was added dropwise over 10 minutes keeping temperature below -60°, and the mixture left to warm to ambient temperature over 18 hours. Methanol (29ml) was added, and the mixture stirred for 10 minutes only. Saturated aqueous sodium bicarbonate (200 ml) was added, and the mixture extracted with ethyl acetate (400 ml). The extract was washed with saturated aqueous sodium bicarbonate (100ml), brine (100ml), dried (magnesium sulfate). Filtered and evaporated.
The crude product was purified on a 300 g silica sinter column , eluting with a gradient from 0% to 100% ethyl acetate in dichloromethane. Relevant fractions were combined, reduced to a small volume, and diluted with an excess of isohexane to precipitate the desired product (11.3 g).
MS (ESP): 310 (MH⁺) for C₁₅H₁₆FNO₃S
NMR (DMSO-d₆) δ: 2.52 (m overlapped by DMSO, ~2H); 2.78 (t, 2H); 3.27 (m, 2H); 3.52 (m, 1H); 3.65 (m, 1H); 3.80 (dd, 1H); 4.06 (dd, 1H); 4.65 (m, 1H); 5.19 (t, 1H); 6.01 (s, 1H); 7.28 (m, 2H); 7.47 (dd, 1H).

### 4-(2-Fluoro-4-benzyloxycarbonylaminophenyl)-3,6-dihydro-2H-thiopyran

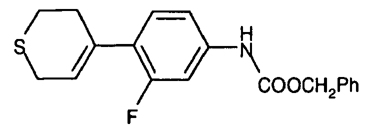

4-(2-Fluoro-4-aminophenyl)-3,6-dihydro-2*H*-thiopyran (9.8 g, 46.8 mM) was dissolved in dry dichloromethane (196ml), pyridine (6.23g, 79.1 mM) added, and the mixture stirred under nitrogen at -20°. A solution of benzyl chloroformate (9.54g, 53.9 mM) dissolved in dry dichloromethane (25 ml) was added dropwise, and the mixture left to warm to ambient temperature over 18 hours. The mixture was washed with 1M hydrochloric acid (200 ml), then brine (100 ml), dried (magnesium sulfate), filtered and evaporated to a small volume. The addition of isohexane (300 ml) precipitated the desired product (15.5 g).
MS (Negative ESP): 342 (M-H⁻) for C₁₉H₁₈FNO₂S
NMR (DMSO-d₆) δ: 2.50 (s, 2H); 2.79 (t, 2H); 3.26 (m, 2H); 5.15 (s, 2H); 5.99 (s, 1H); 7.18 (m, 2H); 7.38 (m, 6H); 10.01 (s, 1H).

### 4-(2-Fluoro-4-aminophenyl)-3,6-dihydro-2H-thiopyran

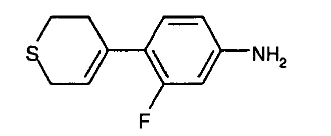

4-Hydroxy-4-(2-fluoro-4-aminophenyl)tetrahydrothiopyran (11.35 g, 50 mM) and butylated hydroxytoluene (50 mg) as antioxidant were suspended in a mixture of concentrated hydrochloric acid (37%, 200 ml) and water (50 ml), and stirred at 80° under nitrogen for 18 hours. Glacial acetic acid (150 ml) was added, and reaction continued at 80° for a further 5 hours. After cooling, the reaction was made basic by the cautious addition of concentrated ammonia and ice. The mixture was extracted with diethyl ether (400 ml), the extract washed with water (100 ml), brine (100 ml), dried(magnesium sulfate), filtered and evaporated to give the title product (10 g) as a dark oil.
NMR (CDCl₃) δ: 2.59 (m, 2H); 2.72 (t, 2H); 3.30 (m, 2H); 3.80 (br, 2H); 5.93 (m, 1H); 6.35 (dd, 1H); 6.39 (dd, 1H); 6.97 (t, 1H).

### Example 2: (5R)-3-[3,5-Difluoro-4-(1RS-1-imino-1-oxo-3,6-dihydrothiopyran-4-yl)-phenyl]-5-(1H-1,2,3-triazol-1-ylmethyl)-2-oxazolidinone

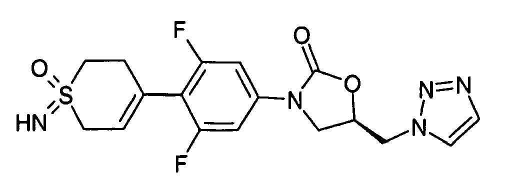

(*5R*)-3-[3,5-Difluoro-4-(1*RS*-1-oxo-3,6-dihydrothiopyran-4-yl)-phenyl]-5-(1*H*-1,2,3-triazol-1-ylmethyl)-2-oxazolidinone (0.54 g, 1.4 mmol) was dissolved/ suspended in dichloromethane (20 ml) at ambient temperature. *O*-Mesitylenesulfonylhydroxylamine (0.3 g, 1.4 mmol, see Synthesis, 1972, 140), in dichloromethane (3 ml) was added dropwise, and the mixture stirred at ambient temperature for 12 hours. The solvent was removed under vacuum and the product was precipitated from methanol by the addition of ethylacetate to give the title compound (0.72 g) as its mesitylene sulfonate salt.
MS (ESP): 410 (MH⁺) for C₁₇H₁₇F₂N₅O₃S
NMR (DMSO-d₆) δ: 2.19 (s, 2x3H); 2.55 (s, 2x6H); 2.92 (br, 2x2H); 3.80-4.06 (m, 2x3H); 4.27 (m, 2x1H); 4.43 (brs, 2x1H); 4.51 (brs, 2x1H); 4.86 (m, 2x1H); 5.03 (m, 2x1H); 5.21 (m, 2x1H); 5.86 (m, 2x1H); 6.76 (s, 2x2H); 7.33 (d, 2H); 7.39 (d, 2H); 7.79 (s, 1H); 8.20 (s, 1H); 8.50 (brs, 1H); 8.71 (s, 1H); 8.89 (s, 1H). 3 exchangeables not detected, complex spectrum resulting from diasteromeric mixture.

The intermediates for this compound were prepared as follows:

### (5R)-3-[3,5-Difluoro-4-(1RS-1-oxo-3.6-dihydrothiopyran-4-yl)-phenyl]-5-(1H-1,2,3-triazol-1-ylmethyl)-2-oxazolidinone

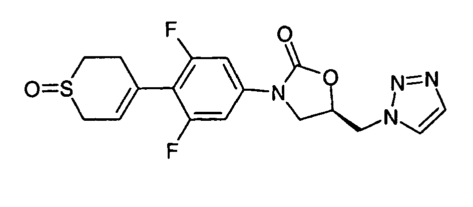

(*R*)-3-[4-(3,6-dihydro-2*H*-thiopyran-4-yl)-3,5-difluorophenyl]-5-(1*H*-1,2,3-triazol-1-ylmethyl)-2-oxazolidinone (0.86 g, 2.3 mmol) was stirred in a mixture of methanol and ethyl acetate (1:1, 20 ml) at ambient temperature. Sodium periodate (0.50 g, 2.4 mmol) in water (10 ml) was added dropwise, and the mixture stirred for 3 hours. Precipitated salts were removed by filtration and washed with ethyl acetate. The filtrate was washed with brine, dried over magnesium sulfate and concentrated to dryness. The residue was chromatographed on silica gel eluting with 5% methanol in dichloromethane to give the title product (0.69 g).
MS (ESP): 395 (MH⁺) for C₁₇H₁₆F₂N₄O₃S
¹H-NMR (DMSO-d₆) δ: 2.41 (brs, 1H); 2.80 (m, 1H); 2.97 (brs, 1H); 3.15 (m, 1H); 3.39 (m, 1H); 3.67 (brs, 1H); 3.94 (m, 1H); 4.25 (dd, 1H); 4.85 (brs, 2H); 5.19 (m, 1H); 5.75 (brs, 1H); 7.33 (d, 2H); 7.79 (brs, 1H); 8.20 (brs, 1H).

### (5R)-3-[4-(3,6-dihydro-2H-thiopyran-4-yl)-3,5-difluorophenyl]-5-(1H-1,2,3-triazol-1-ylmethyl)-2-oxazolidinone

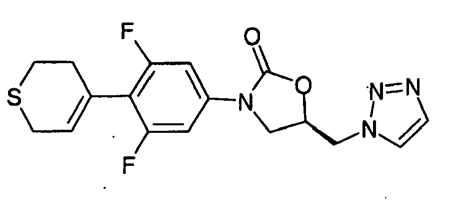

Methanesulfonic acid (*5R*)-3-[4-(3,6-dihydro-2H-thiopyran-4-yl)-3,5-difluorophenyl]-2-oxo-oxazolidin-5-ylmethyl ester (1.1 g, 5.7 mmol) was dissolved in dry N,N-dimethylformamide (5 ml) and sodium azide (0.35 g, 5.43 mmol) was added. It was heated at 60°C for 18 hours. The reaction mixture was cooled to room temperature, diluted with ethylacetate, washed with water and dried over magnesium sulfate. Solvent was removed under vacuum to give an oil. The crude intermediate azide was not characterized. It was taken up in 1,4-dioxane (20 ml), bicyclo[2.2.1]hepta-2,5-diene (1.0 g, 10.9 mmol) was added and it was refluxed for 12 hours. Solvent was removed under vacuum and the residue chromatographed on silica gel with 5% methanol in dichloromethane to give the title compound (0.62g).
MS (ESP): 379 (MH⁺) for C₁₇H₁₆F₂N₄O₂S
NMR (DMSO-d₆) δ: 2.43 (brs, 2H); 2.83 (dd, 2H); 3.31 (brs, 2H); 3.92 (m, 1H); 4.25 (dd, 1H); 4.84 (d, 2H); 5.18 (m, 1H); 5.98 (brs, 1H); 7.28 (d, 2H); 7.79 (brs, 1H); 8.19 (brs, 1H ).

### Methanesulfonic acid (5R)-3-[4-(3,6-dihydro-2H-thiopyran-4-yl)-3,5-difluorophenyl]-2-oxo-oxazolidin-5-ylmethyl ester

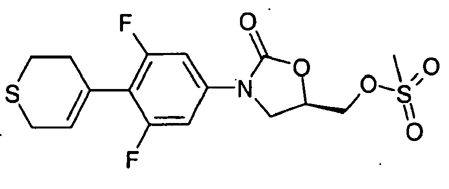

(5*R*)-3-[4-(3,6-dihydro-2H-thiopyran-4-yl)-3,5-difluorophenyl]-5-hydroxymethyl-2-oxazolidin one (4.0 g, 12.2 mmol) was dissolved in dichloromethane (50 ml) and triethylamine (1.85 g, 18.3 mmol) was added. Methanesulfonyl chloride (1.68 g, 14.6 mmol) was added dropwise. The reaction mixture was stirred at room temperature for 1 hour. It was washed with saturated aqueous sodium hydrogencarbonate solution, then with brine and dried over sodium sulfate. The solvent was removed under vacuum and the title compound (5.0 g) was precipitated from dichloromethane by addition of hexanes.
NMR (DMSO-d₆) δ: 2.44 (m, 2H); 2.84 (dd, 2H); 3.28 (s, 3H); 3.31 (m, 2H); 3.86 (dd, 1H); 4.20 (dd, 1H); 4.50 (m, 2H); 5.10 (m, 1H); 5.99 (m, 1H); 7.36 (d, 2H). (No MS)

### (5R)-3-[4-(3,6-dihydro-2H-thiopyran-4-yl)-3,5-difluorophenyl]-5-hydroxymethyl-2-oxazolidinone

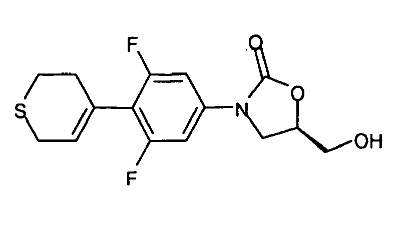

4-(2,6-Difluoro-4-benzyloxycarbonylaminophenyl)-3,6-dihydro-2H-thiopyran (22 g, 61 mM) was reacted with (R)-glycidyl butyrate under essentially the following conditions: material was dissolved in dry tetrahydrofuran (150 ml), and stirred under nitrogen at -70°.
*n*-Butyllithium (1.6M in hexanes, 26 ml, 41.6 mM) was run in over 20 minutes, keeping the temperature below -60°, and the mixture then stirred a further 10 minutes at -70°. A solution of (R)-glycidyl butyrate (5.59 g, 38.8 mM) dissolved in dry tetrahydrofuran (10 ml) was added dropwise over 10 minutes keeping temperature below -60°, and the mixture left to warm to ambient temperature over 18 hours. Methanol (25 ml) was added, and the mixture stirred for 10 minutes only. Saturated aqueous sodium bicarbonate (200 ml) was added, and the mixture extracted with ethyl acetate (400 ml). The extract was washed with saturated aqueous sodium bicarbonate (100 ml), brine (100 ml), dried (magnesium sulfate), filtered and evaporated. Crude product from the final extraction was precipitated from dichloromethane by isohexane, then recrystallised from isopropanol to give the desired product (16.2 g).
MS (ESP): 328 (MH⁺) for C₁₅H₁₅F₂NO₃S
NMR (DMSO-d₆) δ: 2.40 (m, 2H); 2.81 (t, 2H); 3.28 (m, 2H); 3.53 (m, 1H); 3.67 (m, 1H); 3.82 (dd, 1H); 4.08 (t, 1H); 4.70 (m, 1H); 5.21 (t, 1H); 5.95 (s, 1H); 7.33 (d, 2H).

### 4-(2.6-Difluoro-4-benzyloxycarbonylaminophenyl)-3,6-dihydro-2H-thiopyran

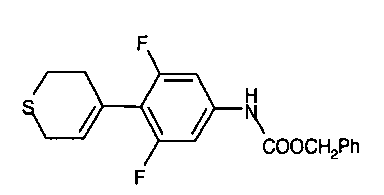

4-(2,6-Difluoro-4-aminophenyl)-3,6-dihydro-2H-thiopyran (15 g, 66 mM) was treated with benzyl chloroformate under essentially the following conditions: material was dissolved in dry dichloromethane (175 ml), pyridine (5.57 g, 70.6 mM) added, and the mixture stirred under nitrogen at -20°. A solution of benzyl chloroformate (8.52 g, 49.9 mM) dissolved in dry dichloromethane (20 ml) was added dropwise, and the mixture left to warm to ambient temperature over 18 hours. The mixture was washed with 1M hydrochloric acid (200 ml), then brine (100 ml), dried (magnesium sulfate), filtered and evaporated to a small volume. The addition of isohexane (300 ml) precipitated the desired product. Similar treatment of the mother liquors from filtration gave more material; total yield (22.5 g).
MS (Negative ESP): 360 (M-H⁻) for C₁₉H₁₇F₂NO₂S
NMR (DMSO-d₆) δ: 2.37 (br, 2H); 2.78 (t, 2H); 3.24 (m, 2H); 5.16 (s, 2H); 5.89 (m, 1H); 7.17 (d, 2H); 7.38 (m, 5H); 10.18 (s, 1H).

### 4-(2,6-Difluoro-4-aminophenyl)-3,6-dihydro-2H-thiopyran

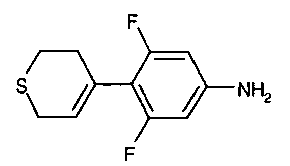

4-Hydroxy-4-(2,6-difluoro-4-aminophenyl)tetrahydrothiopyran (16.7 g, 68 mM) was treated with concentrated hydrochloric acid under essentially the following conditions:
butylated hydroxytoluene (50 mg) used as antioxidant, materials were suspended in a mixture of concentrated hydrochloric acid (37%, 200 ml) and water (50 ml), and stirred at 80° under nitrogen for 18 hours. Glacial acetic acid (150 ml) was added, and reaction continued at 80° for a further 5 hours. After cooling, the reaction was made basic by the cautious addition of concentrated ammonia and ice. The mixture was extracted with diethyl ether (400 ml), the extract washed with water (100 ml), brine (100 ml), dried (magnesium sulfate), filtered and evaporated to give the title product (15.2 g) as a cream solid.
MS (ESP): 228 (MH⁺) for C₁₁H₁₁F₂NS
NMR (CDCl₃) δ: 2.48 (m, 2H); 2.83 (t, 2H); 3.30 (m, 2H); 3.80 (br, 2H); 5.87 (m, 1H); 6.16 (d, 2H).
4-Hydroxy-4-(2,6-difluoro-4-aminophenyl)tetrahydrothiopyran

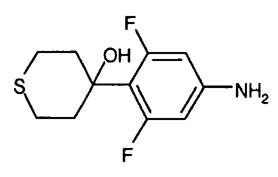

3,5-Difluoroaniline (12.9 g, 0.1 M) was reacted with tetrahydrothiopyran-4-one under essentially the following conditions (except that *n*-butyllithium was used to generate both anions): dissolved in dry tetrahydrofuran (400 ml), stirred under nitrogen, and cooled to -78°. *n*-Butyllithium (1.6M in hexanes, 131 ml, 0.21 M) was run in over 15 minutes, keeping the temperature below -65°, and the mixture then stirred a further 30 minutes at -70°. Chlorotrimethylsilane (22.8 g, 0.21 M) in tetrahydrofuran (100 ml) was added dropwise over 15 minutes, keeping the temperature below -65°, after which the temperature was allowed to rise to ambient, and stirring continued for 40 minutes to complete the silylation. The mixture was then recooled to -78°, and *sec*-butyllithium (1.3M in cyclohexane, 84.3 ml, 0.11 M) added dropwise, and stirring continued at this temperature for 5 hours. A solution of tetrahydrothiopyran-4-one (12.5 g, 0.107 M) in tetrahydrofuran (80 ml) was added dropwise below -70°, and the temperature of the mixture allowed to come to ambient over 18 hours. After cooling in an ice-bath, the reaction was acidified with 1M hydrochloric acid to a pH <1 (-500 ml), stirred 15 minutes, diethyl ether (1 L) added, and the phases separated. The organic layer was washed with 1M hydrochloric acid (200 ml), the combined aqueous layers washed with diethyl ether (200 ml), then made basic with 880 ammonia plus a little ice, then re-extracted with diethyl ether (600 ml). The organic extract was washed with brine (300 ml), dried (magnesium sulfate), filtered and evaporated. Crude product was dissolved in hot dichloromethane (400 ml), evaporated to a low volume, then diluted with isohexane (300 ml). The desired product was precipitated from dichloromethane by isohexane to give a white solid (17.4 g).
MS (Negative ESP): 244 (M-H⁻) for C₁₁H₁₃F₂NOS
NMR (CDQW δ: 2.26 (d, 2H); 2.39 (t, 4H); 2.65 (t, 1H); 3.27 (t, 2H); 3.82 (br, 2H); 6.17 (d, 2H).

### Example 3: (SR)-3-[3-Fluoro-4-(1-imino-1-oxido-4-thiazin-4-yl)phenyl]-5-(1H-1,2,3-triazol-1-ylmethyl)-1,3-oxazolidin-2-one

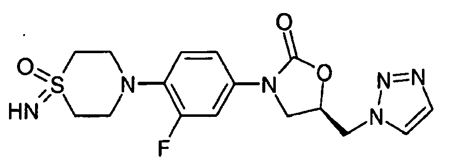

(5*R*)-3-(3-Fluoro-4-(1-oxidothiomorpholin-4-yl)phenyl)-5-(1*H*-1,2,3-triazol-1-ylmethyl)-1,3-oxazolidin-2-one (0.5 g, 1.3 mmol) and sodium azide (0.19 g, 2.9 mmol) were added at ambient temperature under nitrogen to stirred polyphosphoric acid (10 g). The mixture was warmed at 60°C for 12 h, cooled slowly to 0°C and treated dropwise with water (40 ml) and then with enough 50% (w/w) sodium hydroxide to raise the pH to 11.0. This mixture was diluted with water (200 ml) then extracted with a mixture of chloroform and methanol (95:5). The organic extract was dried (Na₂SO₄) and concentrated under reduced pressure to give a residue that was purified by chromatography over silica-gel (elution with 10% methanol in ethyl acetate) to give the desired product (0.34 g) as a free base.
MS (APCI): 395 (M+H)⁺ for C₁₆H₁₉FN₆O₃S
¹H-NMR DMSO-d₆) δ: 3.14 (m, 4H); 3.37 (m, 2H); 3.45 (m, 2H); 3.79 (s, 1H); 3.88 (dd, 1H); 4.23 (t, 1H); 4.84 (d, 2H); 5.14 (m, 1H); 7.14 (dd, 1H);, 7.19 (t, 1H); 7.43 (dd, 1H); 7.78 (d, 1H); 8.18 (d, 1H).

The intermediates for this example were prepared as follows:

### (5R)-3-(3-Fluoro-4-thiomorpholin-4-ylphenyl)-5-(1H-1.2,3-triazol-1-ylmethyl)-1,3-oxazolidin-2-one

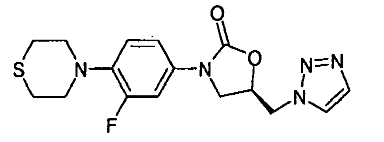

A mixture of (5*R*)-5-(azidomethyl)-3-(3-fluoro-4-thiomorpholin-4-ylphenyl)-1,3-oxazolidin-2-one (20 g, 59 mmol) [Ref: *J. Med. Chem.* 1996, 39, 680-685] and bicyclo[2.2.1]hepta-2,5-diene (20 ml) in dioxane (200 ml) was heated at reflux under nitrogen for 24 hours. The solvent was evaporated under reduced pressure and the involatile residue was purified by chromatography on silica-gel (elution with 10% methanol in dichloromethane) to give the title compound (18 g).
MS (APCI): 364 (M+H)⁺ for C₁₆H₁₈FN₅O₂S
¹H-NMR (DMSO-d₆) δ: 2.75 (t, 4H); 3.21 (t, 4H); 3.87 (dd, 1H); 4.21 (t, 1H); 4.84 (d, 2H); 5.13 (m, 1H); 7.12 (m, 2H); 7.40 (dd, 1H); 7.78 (d, 1H); 8.18 (d, 1H).

### (5R)-3-[3-Fluoro-4-(1-oxidothiomorpholin-4-yl)phenyl]-5-(1H-1,2,3-triazol-1-ylmethyl)-1,3-oxazolidin-2-one

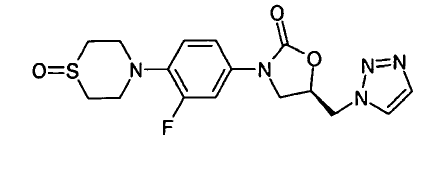

A solution of (5*R*)-3-(3-fluoro-4-thiomorpholin-4-ylphenyl)-5-(1*H*-1,2,3-triazol-1-ylmethyl)-1,3-oxazolidin-2-one (16.0 g, 44.1 mmol) in a mixture of methanol and chloroform (2:1; 300 ml) was treated dropwise with a solution of sodium periodate (11.3 g, 52.9 mmol) in water (200 ml). The mixture was stirred at room temperature for 18 hours and then filtered. The filtrate was concentrated under reduced pressure and the involatile residue was diluted with water (150 ml) and then extracted with chloroform (6 x 250 ml). The combined organic extracts were washed with brine, dried over Na₂SO₄, filtered, and concentrated under reduced pressure to give a foamy solid. The foamy residue was purified by flash chromatography over silica-gel (elution with chloroform, and then with 5% methanol in chloroform) to give the title product (15.7 g).
MS (APCI): 380 (M+H)⁺ for C₁₆H₁₈FN₅O₃S
¹H-NMR (DMSO-d₆) δ: 2.86 (m, 2H); 3.04 (m, 2H); 3.19 (dd, 2H); 3.53 (t, 2H); 3.88 (dd, 1H); 4.22 (t, 1H); 4.84 (d, 2H); 5.15 (m, 1H); 7.15 (dd, 1H); 7.21 (t, 1H); 7.43 (dd, 1H); 7.78 (d, 1H); 8.19 (d, 1H).

### Example 4: (5R)-3-(3-Fluoro-4-[1-(methylimino)-1-oxido-4-thiazin-4-yl]phenyl)-5-(1H-1,2,3-triazol-1-ylmethyl)-1,3-oxazolidin-2-one

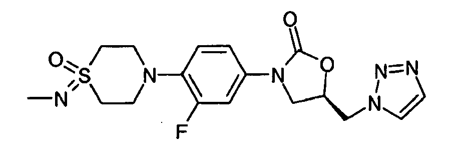

Trifluoroacetic acid (170 µl, 2.3 mmol) was added to a mixture of (5*R*)-3-[3-fluoro-4-(1-imino-1-oxido-4-thiazin-4-yl)phenyl]-5-(1H-1,2,3-triazol-1-ylmethyl)-1,3-oxazolidin-2- one (Example 3) (0.3 g, 0.76 mmol), paraformaldehyde (0.1 g), and triethylsilane (364 µl, 2.3 mmol) in acetonitrile (8 ml) at room temperature. The mixture was stirred for 8 hours at room temperature under nitrogen, then diluted with water (50 ml), neutralized to pH 11, and

extracted with 5% methanol in dichloromethane (4 x 50 ml). The combined organic extracts were dried over Na₂SO₄ and concentrated to give an involatile residue that was purified by flash chromatography over silica-gel (elution with 5% methanol in dichloromethane) to give the title compound (0.34 g).
MS (APCI): 409 (M+H)⁺ for C₁₇H₂₁FN₆O₃S
¹H-NMR (DMSO-d₆) δ: 2.69 (s, 3H); 3.20 (m, 2H); 3.27 (m, 2H); 3.37 (m, 2H); 3.43 (m, 2H); 3.88 (dd, 1H); 4.22 (t, 1H); 4.84 (d, 2H); 5.15 (m, 1H); 7.14 (dd, 1H); 7.19 (t, 1H); 7.43 (dd, 1H); 7.78 (d, 1H); 8.18 (d, 1H).

### Example 5: (5R)-3-(3-Fluoro-4-(1-((1H-imidazol-2-ylmethyl)imino)-1-oxido-4-thiazinan-4-yl)phenyl)-5-(1H-1,2,3-triazol-1-ylmethyl)-1,3-oxazolidin-2-one

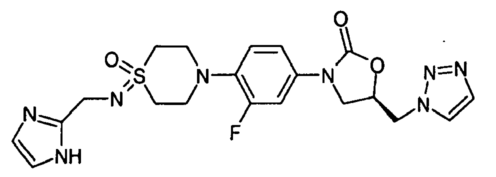

Trifluoroacetic acid (235 µl, 3.04 mmol) was added to a mixture of (5*R*)-3-[3-fluoro-4-(1-imino-1-oxido-4-thiazin-4-yl)phenyl]-5-(1*H*-1,2,3-triazol-1-ylmethyl)-1,3-oxazolidin-2-one (Example 3) (0.3 g, 0.76 mmol), imidazole-2-carboxaldehyde (0.29 g, 3.04 mmol), and triethylsilane (485 µl, 3.04 mmol) in acetonitrile (8 ml) at room temperature. The reaction mixture was stirred under nitrogen for 24 hours at 50°C, allowed to cool to room temperature, diluted with water (50 ml), neutralized to pH 11, and extracted with 5% methanol in dichloromethane (4 x 50 ml). The combined extracts were dried over Na₂SO₄ and concentrated under reduced pressure to give an involatile residue that was purified by flash chromatography over silica-gel (elution with 8% methanol in ethyl acetate) to give the title compound (0.18 g).
MS (APCI): 475 (M+H)⁺ for C₂₀H₂₃FN₈O₃S
¹H-NMR (DMSO-d₆)δ: 3.23-3.42 (m, 8H); 3.87 (dd, 1H); 4.20 (s, 2H); 4.21 (t, 1H); 4.84 (d, 2H); 5.15 (m, 1H); 6.79 (s, 1H); 7.01 (s, 1H); 7.13-7.19 (m, 3H); 7.42 (dd, 1H); 7.78 (d, 1H); 8.18 (d, 1H).

### Example 6: (5R)-3-(3-Fluoro-4-(1-((1-methylthio-1-(N-cyanoimino)methyl)imino)-1-oxido-4-thiazinan-4-yl)phenyl)-5-(1H-1,2,3-triazol-1-ylmethyl)-1,3-oxazolidin-2-one

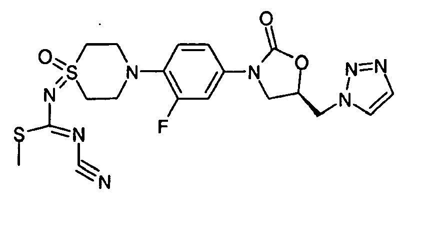

A mixture of (5*R*)-3-(3-fluoro-4-(1-imino-1-oxido-4-thiazinan-4-yl)phenyl)-5-(1*H*-1,2,3-triazol-1-ylmethyl)-1,3-oxazolidin-2-one (Example 3) (1.0 g, 2.53 mmol) and dimethyl N-cyanodithioiminocarbonate was heated in a microwave oven at 140°C for 1.5 hours. The involatile residue was purified by flash chromatography over silica-gel (elution with 7% methanol in ethyl acetate) to give the title compound (0.7 g).
MS (APCI): 493 (M+H)⁺ for C₁₉H₂₁FN₈O₃S₂
¹H-NTMR (DMSO-d₆) δ: 2.58 (s, 3H); 3.47 (m, 2H); 3.65 (m, 2H); 3.81 (m, 2H); 3.88 (dd, 1H); 3.97 (m, 2H); 4.23 (t, 1H); 4.84 (d, 2H); 5.15 (m,1H); 7.16 (dd, 1H); 7.23 (t, 1H); 7.44 (dd, 1H); 7.78 (d, 1H); 8.18 (d, 1H).

### Example 7: (5R)-3-(3-Fluoro-4-(1-((1-dimethylamino-1-(N-cyanoimino)methyl)imino)-1-oxido-4-thiazinan-4-yl)phenyl)-5-(1H-1,2,3-triazol-1-ylmethyl)-1,3-oxazolidin-2-one

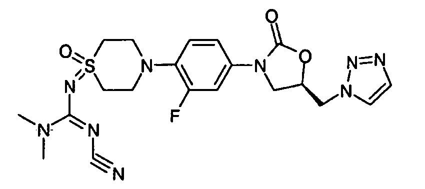

A mixture of (5*R*)-3-(3-fluoro-4-(1-((1-methylthio-1-(N-cyanoimino)methyl)imino)-1-oxido-1λ⁶-4-thiazinan-4-yl)phenyl)-5-(1*H*-1,2,3-triazol-l-ylmethyl)-1,3-oxazolidin-2-one (Example 6) (0.24 g, 0.49 mmol) and dimethylamine (5 ml of a 2M solution in tetrahydrofuran) was heated at 65°C for 2 hours. The reaction mixture was concentrated under reduced pressure and the involatile residue was purified by flash chromatography over silica-gel (elution with 12% methanol in ethyl acetate) to give the title compound (0.18 g).
MS (APCI): 490 (M+H)⁺ for C₂₀H₂₄FN₉O₃S
¹H-NMR (DMSO-d₆) δ: 3.12 (s, 6H); 3.54 (m, 4H); 3.67 (m, 2H); 3.76 (m, 2H); 3.88 (dd, 1H); 4.23 (t, 1H); 4.85 (d, 2H); 5.15 (m, 1H); 7.16 (dd, 1H); 7.23 (t, 1H); 7.44 (dd, 1H); 7.78 (d, 1H); 8.18 (d, 1H).

### Example 8: (5R)-3-(3-Fluoro-4-(1-((4-amino-5-methoxycarbonylthiazol-2-yl)imino)-1-oxido-4-thiazinan-4-yl)phenyl)-5-(1H-1,2,3-triazol-1-ylmethyl)-1,3-oxazolidin-2-one

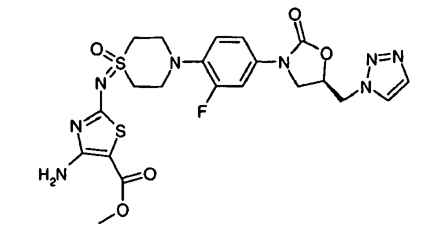

A mixture of (5*R*)-3-(3-fluoro-4-(1-((1-methylthio-1-(N-cyanoimino)methyl)imino)-1-oxido-4-thiazinan-4-yl)phenyl)-5-(1*H*-1,2,3-triazol-1-ylmethyl)-1,3-oxazolidin-2-one (Example 6) (0.4 g, 0.81 mmol) and methylthioglycolate (160 µl, 1.78 mmol) in dry ethanol (25 ml) was treated with triethylamine (2 ml) at room temperature. The reaction mixture was stirred at room temperature for 24 hours then warmed up to 60°C for 15h. The mixture was concentrated under reduced pressure and the involatile residue was purified by chromatography on silica-gel (elution with 5% methanol in ethyl acetate) to give the title compound (180 mg).
MS (APCI): 551 (M+H)⁺ for C₂₁H₂₃FN₈O₅S₂
¹H-NMR (DMSO-d₆) δ: 3.43 (m, 2H); 3.63 (m, 2H); 3.66 (s, 3H); 3.74 (m, 2H); 3.88 (dd, 1H); 3.94 (m, 2H); 4.22 (t, 1H); 4.84 (d, 2H); 5.15 (m, 1H); 6.86 (s, 2H); 7.15 (dd, 1H); 7.24 (t, 1H); 7.44 (dd, 1H); 7.78 (s, 1H); 8.18 (s, 1H).

### Example 9: (5R)-3-[3-Fluoro-4-(1RS-1-(acetylimino)-1-oxo-2,3-dihydro-thiopyran-4-yl)-phenyl]-5-(1,2,3-triazol-1-ylmethyl)-oxazolidin-2-one

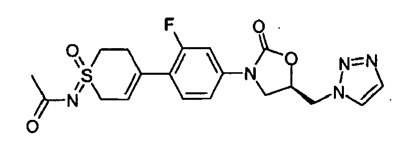

(5*R*)-3-[3-Fluoro-4-(*1RS*-I-imino-1-oxo-3,6-dihydro-thiopyran-4-yl)-phenyl]-5-(1,2,3-triazol-1-ylmethyl)-oxazolidin-2-one (Example 1) as its free base (0.18 g, 0.46 mmol) was dissolved in pyridine (0.3 ml), dichloromethane (2 ml) was added and it was cooled to -20°C. Acetylchloride (66 µl, 0.93 mmol) dissolved in dichloromethane (2 ml) was added dropwise and it was stirred for 1 h. It was quenched with phosphate buffer pH 7, extracted with ethylacetate, washed with brine and dried over sodium sulfate. Chromatography on silicagel with acetone/hexane 2:1 gave 0.161 g of the title compound.
MS (ESP): 433.83 (MH⁺) for C₁₉H₂₀FN₅O₄S
¹H-NMR MMSO-d₆) δ: 8.19 (d, 1H); 7.78 (brs, 1H); 7.47 (dd, 1H); 7.40 (dd, 1H); 7.29 (dd, 1H); 5.87 (m, 1H); 5.18 (m, 1H); 4.86 (d, 2H); 4.40 (m, 1H); 4.26 (dd, 1H); 4.23 (m, 1H); 3.92 (dd, 1H); 3.71 (m, 2H); 2.96 (m, 2H); 1.99 (s, 3H).

### Example 10: (5R)-3-[3,5-Difluoro-4-(1RS-1-(acetylimino)-1-oxo-2,3-dihydrothiopyran-4-yl)-phenyl]-5-(1,2,3-triazol-1-ylmethyl)-oxazolidin-2-one

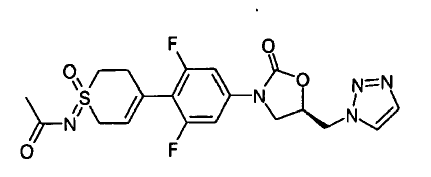

(5*R*)-3-[3,5-Difluoro-4-(1*RS*-1-imino-1-oxo-3,6-dihydrothiopyran-4-yl)-phenyl]-5-(1,2,3-triazol-1-ylmethyl)-oxazolidin-2-one (Example 2) as its mesitylene sulfonate salt (0.1 g, 0.164 mmol) was dissolved in pyridine (0.3 ml), dichloromethane (2 ml) was added and it was cooled to -20°C. Acetylchloride (26mg, 0.33 mmol) dissolved in dichloromethane (2 ml) was added dropwise and it was stirred for 30min. It was quenched with methanol, extracted with ethylacetate, washed with saturated sodium bicarbonate solution and brine and dried over anhydrous magnesium sulfate. Chromatography on silica gel with 5% methanol in dichloromethane gave 50 mg of the title compound.
MS (ESP): 451.75 (MH⁺) for C₁₉H₁₉F₂N₅O₄S
¹H-NMR (CDCl₃) δ: 7.81 (d, 1H); 7.79 (d, 1H); 7.10 (d, 2H); 5.78 (m, 1H); 5.12 (m, 1H); 4.82 (d, 2H); 4.51 (m, 1H); 4.18 (dd, 1H); 4.10 (m, 2H); 3.72 (m, 1H); 3.45 (m, 1H); 3.0 (m, 2H); 2.18 (s, 3H).

### Example 11: (5R)-3-[3,5-Difluoro-4-(1RS-1-(2-hydroxyl-acetylimino)-1-oxo-3.6-dihydrothiopyran-4-yl)-phenyl]-5-(1,2,3-triazol-1-ylmethyl)-oxazolidin-2-one

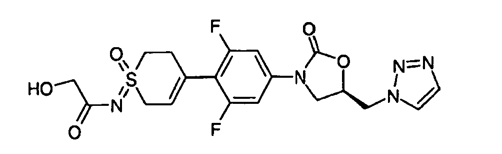

(SR)-3-[3,5-Difluoro-4-(1RS-1-imino-1-oxo-3,6-dihydrothiopyran-4-yl)-phenyl]-5-(1,2,3-triazol-1-ylmethyl)-oxazolidin-2-one (Example 2) as its mesitylene sulfonate salt (0.2 g, 0.33 mmol) was reacted with acetyloxyacetyl chloride (90mg, 0.66 mmol) following the procedure described under example 9. Chromatography on silica gel with 5% methanol in dichloromethane gave 100 mg of pure product. This intermediate was dissolved in 15 ml of methanol, catalytic amount of potassium carbonate was added and the mixture was stirred at room temperature for 3 hours. Ammonium chloride (leq.) was added and the solvent was evaporated. The residue was purified by flash chromatography with acetone to give 50 mg of the title compound as white solid.
MS (ESP): 467.75 (MH⁺) for C₁₉H₁₉F₂N₅O₅S
¹H-NMR (DMSO-d₆) δ: 8.19 (d, 1H); 7.78 (d, 1H); 7.40 (d, 2H); 5.83 (m, 1H); 5.18 (m, 1H); 4.86 (d, 2H); 4.82 (dd, 1H); 4.45(m, 1H); 4.30 (m, 1H); 4.23 (dd, 1H); 3.92 (m, 3H); 3.71 (m, 2H); 2.90 (m, 2H).

## Claims

1. A compound of the formula (IA), or a pharmaceutically-acceptable salt, or in-vivo hydrolysable ester thereof 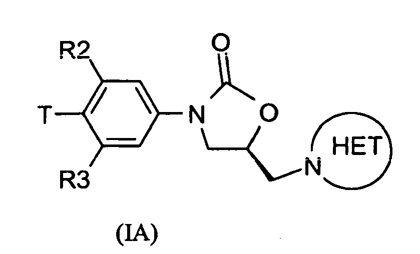 wherein HET is 1,2,3-triazole, 1,2,4-triazole or tetrazole; or HET is a di-hydro version of pyrimidine, pyridazine, pyrazine, 1,2,3-triazine, 1,2,4-triazine, 1,3,5-triazine and pyridine;
wherein HET is optionally substituted on any available C atom, other than a C atom adjacent to the linking N atom, by a substituent selected from (1-4C)alkyl, (2-4C)alkenyl, (3-6C)cycloalkyl, amino, (1-4C)alkylamino, di-(1-4C)alkylamino, (1-4C)alkylthio, (1-4C)alkoxy, (1-4C)alkoxycarbonyl, halogen, cyano and trifluoromethyl and/or on an available nitrogen atom (provided that the ring is not thereby quatemised) by (1-4C)alkyl; and wherein at each occurrence of alkyl, alkenyl and cycloalkyl HET substituents, each is optionally substituted with one or more F, Cl or CN;
R² and R³ are independently hydrogen or fluoro; and
T is selected from (TA1), (TA2) and (TB1) to (TB3), wherein (TA1), (TA2) and (TB1) to (TB3);
(TA) T is selected from the following groups (TA1) and (TA2) :- 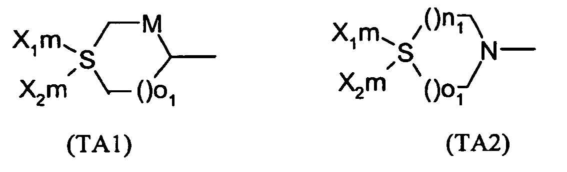 wherein :
in (TA1), ()o₁ is 0 or 1 and represents a chain of carbon atoms (optionally substituted as defined for AR1) of length o₁ and M is a bond joining the adjacent carbon atoms, or M represents one or two carbon atoms, and defines a 4- to 7-membered monocyclic ring, which ring may optionally have one of
(i) one double bond between any two ring carbon atoms; or
(ii) a C1-C3 bridge connecting any two appropriate, non-adjacent ring carbon atoms, which bridge may optionally contain one heteroatom selected from oxygen or >NRc; or
(iii) a C2-C5 cyclic moiety including a ring carbon atom to define a spiro C2-C5 ring system, which ring may optionally contain one heteroatom selected from oxygen or >NRc; or
(iv) a C1-C4 bridge connecting adjacent carbon atoms to define a fused ring, wherein a C2-C4 bridge may optionally contain one heteroatom selected from oxygen or >NRc; wherein Rc is as defined hereinafter;
wherein in (TA2), ()n₁ and ()o₁ are independently 0, 1or 2 and represent chains of carbon atoms (optionally substituted as defined for AR1) of length n₁ and o₁ respectively, and define a 4- to 8-membered monocyclic ring, which ring may optionally have one of
(i) a C1-C3 bridge connecting any two appropriate, non-adjacent ring carbon atoms, which bridge contains one heteroatom selected from oxygen or >NRc; or
(ii) a C2-C5 cyclic moiety including a ring carbon atom to define a spiro C2-C5 ring system, which ring may optionally contain one heteroatom selected from oxygen or >NRc; or
(iii) a C1-C4 bridge connecting adjacent carbon atoms to define a fused ring, wherein a C2-C4 bridge may optionally contain one heteroatom selected from oxygen or >NRc; wherein Rc is as defined hereinafter; and
wherein in (TA1) and (TA2), X₁ₘ and X₂ₘ taken together represent R₂ₛ-(E)ₘₛ-N=; or X₁ₘ is O= and X₂ₘ is R₂ₛ-(E)ₘₛ-N-, and vice versa;
wherein E is an electron withdrawing group selected from -SO₂-, -CO-, -O-CO-, -CO-O-, -CS-, -CON(Rₛ)-, -SO₂N(Rₛ)-, or E may represent a group of the formula R₃ₛ-C(=N-O-R₃ₛ)-C(=O)-, wherein R₃ₛ is H or as defined in R₂ₛ at (i) below;
or, when E is -CON(Rₛ)- or -SO₂N(Rₛ)-, R₂ₛ and Rₛ may link together to form a carbon chain which defines a 5- or 6-membered saturated, unsaturated or partially unsaturated ring linked via the N atom in E, which ring is optionally further substituted by an oxo substituent, and which ring may be optionally fused with a phenyl group to form a benzo-fused system,
wherein the phenyl group is optionally substituted by up to three substituents independently selected from halo, cyano, (1-4C)alkyl and (1-4C)alkoxy;
ms is 0 or 1;
R₂ₛ and Rₛ are independently selected from :
(i) hydrogen (except where E is -SO₂-or -O-CO-), or (1-6C)alkyl {optionally substituted by one or more (1-4C)alkanoyl groups (including geminal disubstitution) and/or optionally monosubstituted by cyano, cyano-imino, (1-4C)alkoxy, trifluoromethyl, (1-4C)alkoxycarbonyl, phenyl (optionally substituted as defined for AR1 herein), optionally substituted heteroaryl group of the formula AR2, AR2a, AR2b, AR3, AR3a, AR3b, AR4, AR4a or CY all as defined (and optionally substituted as defined) herein, (1-4C)alkylS(O)_{q}- (q is 0, 1 or 2); and/or (with the proviso that where R₂ₛ is -SO₂ or -O-CO-not on the first carbon atom of the (1-6C) alkyl chain) optionally substituted by one or more groups (including geminal disubstitution) each independently selected from hydroxy and fluoro, and/or optionally further substituted, by no more than one of each of, oxo, -NRvRw [wherein Rv is hydrogen or (1-4C)alkyl; Rw is hydrogen or (1-4C)alkyl], (1-6C)alkanoylamino, (1-4C)alkoxycarbonylamino, N-(1-4C)alkyl-N-(1-6C)alkanoylamino, (1-4C)alkylS(O)ₚNH- or (1-4C)alkyls(O)ₚ-((1-4C)alkyl)N-(p is 1 or 2)}; or
(ii) an optionally substituted aryl or optionally substituted heteroaryl group of the formula AR1, AR2, AR2a, AR2b, AR3, AR3a, AR3b, AR4, AR4a or CY all as defined (and optionally substituted as defined) herein; or (where ms is 0 only);
(iii) cyano, -CO-NRvRw, -CO-NRv Rw', -SO₂-NRvRw, -SO₂-NRv Rw' [wherein Rv is hydrogen or (1-4C)alkyl; Rw is hydrogen or (1-4C)alkyl; Rw' is phenyl (optionally substituted as defined for AR1 herein), or a heteroaryl group selected from AR2, AR2a, AR2b, AR3, AR3a, AR3b, AR4, AR4a (optionally substituted as defined herein)], (1-4C)alkoxycarbonyl, trifluoromethyl, ethenyl, 2-(1-4C)alkylethenyl, 2-cyanoethenyl, 2-cyano-2-((1-4C)alkyl)ethenyl, 2-nitroethenyl, 2-nitro-2-((1-4C)alkyl)ethenyl, 2-((1-4C)alkylaminocarbonyl)ethenyl, 2-((1-4C)alkoxycarbonyl)ethenyl, 2-(AR1)ethenyl, 2-(AR2)ethenyl, or 2-(AR2a)ethenyl; or
(TB) T is selected from the following groups (TB1) to (TB3):- 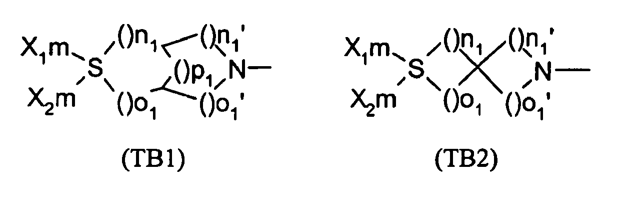 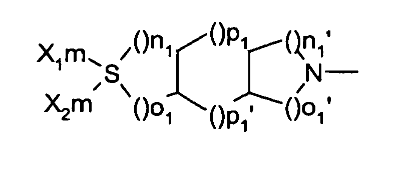 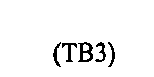 wherein :
X₁ₘ and X₂ₘ taken together represent R₂ₛ-(E)ₘₛ-N=; or
X₁ₘ is O= and X₂ₘ is R₂ₛ-(E)ₘₛ-N-, and vice versa;
wherein E is an electron withdrawing group selected from -SO₂- -CO-, -O-CO-, -CO-O-, -CS-, -CON(Rₛ)-, -SO₂N(Rₛ)-, or E may represent a group of the formula R₃ₛ-C(=N-O-R₃ₛ)-C(=O)-, wherein R₃ₛ is H or as defined in R₂ₛ at (i) below;
or, when E is -CON(Rₛ)- or -SO₂N(Rₛ)-, R₂ₛ and Rₛ may link together to form a carbon chain which defines a 5- or 6-membered saturated, unsaturated or partially unsaturated ring linked via the N atom in E, which ring is optionally further substituted by an oxo substituent, and which ring may be optionally fused with a phenyl group to form a benzo-fused system,
wherein the phenyl group is optionally substituted by up to three substituents independently selected from halo, cyano, (1-4C)alkyl and (1-4C)alkoxy;
ms is 0 or 1;
R₂ₛ and Rₛ are independently selected from :
(i) hydrogen (except where E is -SO₂-or -O-CO-), or (1-6C)alkyl {optionally substituted by one or more (1-4C)alkanoyl groups (including geminal disubstitution) and/or optionally monosubstituted by cyano, cyano-imino, (1-4C)alkoxy, trifluoromethyl, (1-4C)alkoxycarbonyl, phenyl (optionally substituted as for AR1), optionally substituted heteroaryl group of the formula AR2, AR2a, AR2b, AR3, AR3a, AR3b, AR4, AR4a or CY all as defined hereinafter, (1-4C)alkylS(O)_{q}- (q is 0, 1 or 2); and/or (with the proviso that where R₂ₛ is -SO₂ or -O-CO- not on the first carbon atom of the (1-6C) alkyl chain) optionally substituted by one or more groups (including geminal disubstitution) each independently selected from hydroxy and fluoro, and/or optionally further substituted, by no more than one of each of, oxo, -NRvRw [wherein Rv is hydrogen or (1-4C)alkyl; Rw is hydrogen or (1-4C)alkyl], (1-6C)alkanoylamino, (1-4C)alkoxycarbonylamino, N-(1-4C)alkyl-N-(1-6C)alkanoylamino, (1-4C)alkylS(O)ₚNH- or (1-4C)alkylS(O)ₚ-((1-4C)alkyl)N- (p is 1 or 2)}; or
(ii) an optionally substituted aryl or optionally substituted heteroaryl group of the formula AR1, AR2, AR2a, AR2b, AR3, AR3a, AR3b, AR4, AR4a or CY all as defined herein; or (where ms is 0 only);
(iii) cyano, -CO-NRvRw, -CO-NRv Rw', -SO₂-NRvRw, -SO₂-NRv Rw' [wherein Rv is hydrogen or (1-4C)alkyl; Rw is hydrogen or (1-4C)alkyl; Rw' is phenyl (optionally substituted as for AR1), or a heteroaryl group selected from AR2, AR2a, AR2b, AR3, AR3a, AR3b, AR4, AR4a (as defined herein)], (1-4C)alkoxycarbonyl, trifluoromethyl, ethenyl, 2-(1-4C)alkylethenyl, 2-cyanoethenyl, 2-cyano-2-((1-4C)alkyl)ethenyl, 2-nitroethenyl, 2-nitro-2-((1-4C)alkyl)ethenyl, 2-((1-4C)alkylaminocarbonyl)ethenyl, 2-((1-4C)alkoxycarbonyl)ethenyl, 2-(AR1)ethenyl, 2-(AR2)ethenyl, or 2-(AR2a)ethenyl; and
wherein ()n₁, ()o₁, ()n₁ ()o_{1'}, ()p₁ and ()_{p1'} represent chains of carbon atoms (optionally substituted as for AR1) of length n₁, o₁, n_{1'}, o_{1'}, p₁ and p_{1'}, respectively, and are independently 0-2, with the proviso that in (TB1) and (TB2) the sum of n₁, o₁, n_{1'} and o_{1'} does not exceed 8 (giving a maximum ring size of 14 in (TB1) and 11 in (TB2)), and in (TB3) the sum of n₁, o₁, n_{1'}, o_{1'}, p₁ and p_{1'} does not exceed 6 (giving a maximum ring size of 12);
wherein Rc is selected from groups (Rc1) to (Rc5) :-
*(Rc1)* optionally substituted (1-6C)alkyl;
*(Rc2)* R¹³CO- , R¹³SO₂- or R¹³CS-
wherein R¹³ is selected from (Rc2a) to (Rc2e) :-
*(Rc2a)* AR1, AR2, AR2a, AR2b, AR3, AR3a, AR3b, AR4, AR4a, CY;
*(Rc2b)* hydrogen, (1-4C)alkoxycarbonyl, trifluoromethyl, -NRvRw [wherein Rv is hydrogen or (1-4C)alkyl; Rw is hydrogen or (1-4C)alkyl], ethenyl, 2-(1-4C)alkylethenyl, 2-cyanoethenyl, 2-cyano-2-((1-4C)alkyl)ethenyl, 2-nitroethenyl, 2-nitro-2-((1-4C)alkyl)ethenyl, 2-((1-4C)alkylaminocarbonyl)ethenyl, 2-((1-4C)alkoxycarbonyl)ethenyl, 2-(AR1)ethenyl, 2-(AR2)ethenyl, 2-(AR2a)ethenyl;
*(Rc2c)* optionally substituted (1-10C)alkyl;
*(Rc2d)* R¹⁴C(O)O(1-6C)alkyl wherein R¹⁴ is AR1, AR2, (1-4C)alkylamino (the (1-4C)alkyl group being optionally substituted by (1-4C)alkoxycarbonyl or by carboxy), benzyloxy-(1-4C)alkyl or (1-10C)alkyl {optionally substituted as defined for (Rc2c)};
*(Rc2e)* R¹⁵O- wherein R¹⁵ is benzyl, (1-6C)alkyl {optionally substituted as defined for (Rc2c)}, CY, or AR2b;
*(Rc3)* hydrogen, cyano, 2-cyanoethenyl, 2-cyano-2-((1-4C)alkyl)ethenyl, 2-((1-4C)alkylaminocarbonyl)ethenyl, 2-((1-4C)alkoxycarbonyl)ethenyl, 2-nitroethenyl, 2-nitro-2-((1-4C)alkyl)ethenyl, 2-(AR1)ethenyl, 2-(AR2)ethenyl, or of the formula (Rc3a) 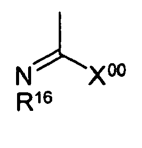 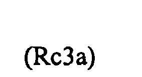 wherein X⁰⁰ is -OR¹⁷, -SR¹⁷, -NHR¹⁷and -N(R¹⁷)₂ ;
wherein R¹⁷ is hydrogen (when X⁰⁰ is -NHR¹⁷ and -N(R¹⁷)₂), and R¹⁷ is (1-4C)alkyl, phenyl or AR2 (when X⁰⁰ is -OR¹⁷, -SR¹⁷ and -NHR¹⁷); and R¹⁶ is cyano, nitro, (1-4C)alkylsulfonyl, (4-7C)cycloalkylsulfonyl, phenylsulfonyl, (1-4C)alkanoyl and (1-4C)alkoxycarbonyl;
*(Rc4)* trityl, AR1, AR2, AR2a, AR2b, AR3, AR3a, AR3b;
*(Rc5)* RdOC(Re)=CH(C=O)-, RfC(=O)C(=O)-, RgN=C(Rh)C(=O)- or RiNHC(Rj)=CHC(=O)- wherein Rd is (1-6C)alkyl; Re is hydrogen or (1-6C)alkyl, or Rd and Re together form a (3-4C)alkylene chain; Rf is hydrogen, (1-6C)alkyl, hydroxy(1-6C)alkyl, (1-6C)alkoxy(1-6C)alkyl, -NRvRw [wherein Rv is hydrogen or (1-4C)alkyl; Rw is hydrogen or (1-4C)alkyl], (1-6C)alkoxy, (1-6C)alkoxy(1-6C)alkoxy, hydroxy(2-6C)alkoxy, (1-4C)alkylamino(2-6C)alkoxy, di-(1-4C)alkylamino(2-6C)alkoxy; Rg is (1-6C)alkyl, hydroxy or (1-6C)alkoxy; Rh is hydrogen or (1-6C)alkyl; Ri is hydrogen, (1-6C)alkyl, AR1, AR2, AR2a, AR2b and Rj is hydrogen or (1-6C)alkyl;
wherein
CY is an optionally substituted cyclobutyl, cyclopentyl, cyclohexyl, cyclopentenyl or cyclohexenyl ring;
**AR1** is an optionally substituted phenyl or optionally substituted naphthyl;
**AR2** is an optionally substituted 5- or 6-membered, fully unsaturated (i.e with the maximum degree of unsaturation) monocyclic heteroaryl ring containing up to four heteroatoms independently selected from O, N and S (but not containing any O-O, O-S or S-S bonds), and linked via a ring carbon atom, or a ring nitrogen atom if the ring is not thereby quatemised;
**AR2a** is a partially hydrogenated version of AR2 (i.e. AR2 systems retaining some, but not the full, degree of unsaturation), linked via a ring carbon atom or linked via a ring nitrogen atom if the ring is not thereby quatemised;
**AR2b** is a fully hydrogenated version of AR2 (i.e. AR2 systems having no unsaturation), linked via a ring carbon atom or linked via a ring nitrogen atom;
**AR3** is an optionally substituted 8-, 9- or 10-membered, fully unsaturated (i.e with the maximum degree of unsaturation) bicyclic heteroaryl ring containing up to four heteroatoms independently selected from O, N and S (but not containing any O-O, O-S or S-S bonds), and linked via a ring carbon atom in either of the rings comprising the bicyclic system;
**AR3a** is a partially hydrogenated version of AR3 (i.e. AR3 systems retaining some, but not the full, degree of unsaturation), linked via a ring carbon atom, or linked via a ring nitrogen atom if the ring is not thereby quatemised, in either of the rings comprising the bicyclic system;
**AR3b** is a fully hydrogenated version of AR3 (i.e. AR3 systems having no unsaturation), linked via a ring carbon atom, or linked via a ring nitrogen atom, in either of the rings comprising the bicyclic system;
**AR4** is an optionally substituted 13- or 14-membered, fully unsaturated (i.e with the maximum degree of unsaturation) tricyclic heteroaryl ring containing up to four heteroatoms independently selected from O, N and S (but not containing any O-O, O-S or S-S bonds), and linked via a ring carbon atom in any of the rings comprising the tricyclic system;
**AR4a** is a partially hydrogenated version of AR4 (i.e. AR4 systems retaining some, but not the full, degree of unsaturation), linked via a ring carbon atom, or linked via a ring nitrogen atom if the ring is not thereby quaternised, in any of the rings comprising the tricyclic system;
wherein substituents on AR1, AR2, AR2a, AR2b, AR3, AR3a, AR3b, AR4, AR4a and CY are (on an available carbon atom) up to three substituents independently selected from (1-4C)alkyl {optionally substituted by (preferably one) substituents selected independently from hydroxy, trifluoromethyl, (1-4C)alkyl S(O)_{q}- (q is 0, 1 or 2) (this last substituent preferably on AR1 only), (1-4C)alkoxy, (1-4C)alkoxycarbonyl, cyano, nitro, (1-4C)alkanoylamino, - CONRvRw or -NRvRw}, trifluoromethyl, hydroxy, halo, nitro, cyano, thiol, (1-4C)alkoxy, (1-4C)alkanoyloxy, dimethylaminomethyleneaminocarbonyl, di(N-(1-4C)alkyl)aminomethylimino, carboxy, (1-4C)alkoxycarbonyl, (1-4C)alkanoyl, (1-4C)alkylSO₂amino, (2-4C)alkenyl {optionally substituted by carboxy or (1-4C)alkoxycarbonyl}, (2-4C)alkynyl, (1-4C)alkanoylamino, oxo (=O), thioxo (=S), (1-4C)alkanoylamino {the (1-4C)alkanoyl group being optionally substituted by hydroxy}, (1-4C)alkyl S(O)_{q}- (q is 0, 1 or 2) {the (1-4C)alkyl group being optionally substituted by one or more groups independently selected from cyano, hydroxy and (1-4C)alkoxy}, -CONRvRw or -NRvRw [wherein Rv is hydrogen or (1-4C)alkyl; Rw is hydrogen or (1-4C)alkyl]; and wherein further suitable substituents on AR1, AR2, AR2a, AR2b, AR3, AR3a, AR3b, AR4, AR4a, and CY (on an available carbon atom), and also on alkyl groups (unless indicated otherwise) are up to three substituents independently selected from trifluoromethoxy, benzoylamino, benzoyl, phenyl {optionally substituted by up to three substituents independently selected from halo, (1-4C)alkoxy or cyano}, furan, pyrrole, pyrazole, imidazole, triazole, pyrimidine, pyridazine, pyridine, isoxazole, oxazole, isothiazole, thiazole, thiophene, hydroxyimino(1-4C)alkyl, (1-4C)alkoxyimino(1-4C)alkyl, halo-(1-4C)alkyl, (1-4C)alkanesulfonamido, -SO₂NRvRw [wherein Rv is hydrogen or (1-4C)alkyl; Rw is hydrogen or (1-4C)alkyl].

2. A compound as claimed in claim 1 of the formula (IA), or a pharmaceutically-acceptable salt, or in-vivo hydrolysable ester thereof, wherein HET is 1,2,3-triazole, 1,2,4-triazole or tetrazole;
R² and R³ are independently hydrogen or fluoro; and
T is selected from (TA1a & b), (TA2a) and (TB1a & b).

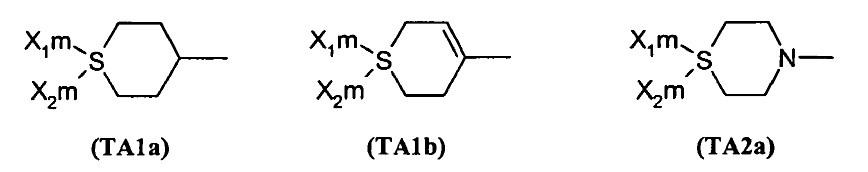

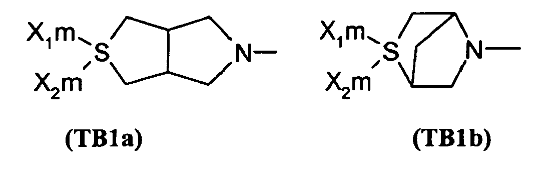

3. A compound of the formula (IA) as claimed in claim 2, or a pharmaceutically-acceptable salt, or in-vivo hydrolysable ester thereof, wherein
T is selected from (TA1a & b) and (TA2a).

4. A compound as claimed in any preceding claim, or a pharmaceutically-acceptable salt, or in-vivo hydrolysable ester thereof, wherein HET is substituted by methyl (optionally substituted with one or more F, Cl or CN);

5. A compound as claimed in any preceding claim, or a pharmaceutically-acceptable salt, or in-vivo hydrolysable ester thereof, wherein HET is unsubstituted.

6. A compound as claimed in any preceding claim or pharmaceutically-acceptable salt or in-vivo hydrolysable ester thereof, wherein X₁ₘ is O= and X₂ₘ is R₂ₛ-(E)ₘₛ-N-, and vice versa; and when ms is 0, R₂ₛ is selected from
(i) hydrogen, a (1-6C)alkyl group {optionally monosubstituted by (1-4C)alkanoyl group, cyano, cyano-imino, (1-4C)alkoxy, trifluoromethyl, (1-4C)alkoxycarbonyl, phenyl (optionally substituted as for AR1 defined herein), optionally substituted heteroaryl group of the formula AR2, AR2a, AR2b, AR3, AR3a, AR3b, AR4, AR4a or CY all as defined (and optionally substituted as defined) herein, (1-4C)alkylS(O)_{q}- (q is 0, 1 or 2); or optionally substituted by one or more fluoro groups (including geminal disubstitution); or optionally substituted by one or more hydroxy groups (excluding geminal disubstitution), and/or optionally further substituted, by no more than one of each of, oxo, -NRvRw [wherein Rv is hydrogen or (1-4C)alkyl; Rw is hydrogen or (1-4C)alkyl], (1-6C)alkanoylamino, (1-4C)alkoxycarbonylamino, N-(1-4C)alkyl-N-(1-6C)alkanoylamino, (1-4C)alkylS(O)ₚNH- or (1-4C)alkylS(O)ₚ-((1-4C)alkyl)N- (p is 1 or 2)} ; or
(ii) an optionally substituted aryl or optionally substituted heteroaryl group of the formula AR1, AR2, AR2a, AR2b, AR3, AR3a, AR3b, AR4, AR4a or CY all as defined (and optionally substituted as defined) herein; or
(iii) cyano, -CO-NRvRw, -CO-NRv Rw', -SO₂-NRvRw, -SO₂-NRv Rw' [wherein Rv is hydrogen or (1-4C)alkyl; Rw is hydrogen or (1-4C)alkyl; Rw' is phenyl (optionally substituted as for AR1 defined herein), or a heteroaryl group selected from AR2, AR2a, AR2b, AR3, AR3a, AR3b, AR4, AR4a (optionally substituted as defined herein)], (1-4C)alkoxycarbonyl, trifluoromethyl;
and when ms is 1, E is -CO- or -SO₂- and R₂ₛ is selected from :
(i) (1-6C)alkyl {optionally monosubstituted by cyano, cyano-imino, (1-4C)alkoxy, trifluoromethyl, (1-4C)alkoxycarbonyl, phenyl (optionally substituted as for AR1 defined herein), optionally substituted heteroaryl group of the formula AR2, AR2a, AR2b, AR3, AR3a, AR3b, AR4, AR4a or CY all as defined (and optionally substituted as defined) herein, (1-4C)alkylS(O)q- (q is 0, 1 or 2); and/or (with the proviso that where R₂ₛ is -SO₂- or -O-CO-not on the first carbon atom of the (1-6C) alkyl chain) optionally substituted by one or more groups (including geminal disubstitution) each independently selected from hydroxy and fluoro, and/or optionally monosubstituted by -NRvRw [wherein Rv is hydrogen or (1-4C)alkyl; Rw is hydrogen or (1-4C)alkyl], (1-6C)alkanoylamino, (1-4C)alkoxycarbonylamino, N-(1-4C)alkyl-N-(1-6C)alkanoylamino, (1-4C)alkylS(O)ₚNH- or (1-4C)alkylS(O)p-((1-4C)alkyl)N- (p is 1 or 2)}; or
(ii) an optionally substituted aryl or heteroaryl group of the formula AR1, AR2, AR2a, AR2b, AR3, AR3a, AR3b, AR4, AR4a or CY all as defined (and optionally substituted as defined) herein.

7. A compound as claimed in any preceding claim or pharmaceutically-acceptable salt or in-vivo hydrolysable ester thereof, wherein X₁ₘ is O= and X₂ₘ is R₂ₛ-(E)ₘₛ-N-, and vice versa; and when ms is 0, R₂ₛ is selected from
(i) hydrogen, (1-6C)alkyl {optionally monosubstituted by (1-4C)alkoxy, trifluoromethyl, (1-4C)alkylS(O)_{q}- (q is 0, 1 or 2); or optionally substituted by one or more fluoro-groups (including geminal disubstitution); or optionally substituted by one or more hydroxy groups (excluding geminal disubstitution)}; or
(iii) -CO-NRvRw [wherein Rv is hydrogen or (1-4C)alkyl; Rw is hydrogen or (1-4C)alkyl], -CO-NRv Rw' [wherein Rv is hydrogen or (1-4C)alkyl; Rw' is phenyl (optionally substituted as for AR1 defined herein)], (1-4C)alkoxycarbonyl; and when ms is 1, E is -CO- or -SO₂- and R₂ₛ is selected from :
(i) (1-6C)alkyl {optionally monosubstituted by (1-4C)alkoxy, trifluoromethyl, (1-4C)alkylS(O)_{q}- (q is 0, 1 or 2); or optionally substituted by one or more fluoro groups (including geminal disubstitution); or optionally substituted by one or more hydroxy groups (excluding geminal disubstitution)}, (1-6C)alkanoylamino, (1-4C)alkoxycarbonylamino.

8. A compound as claimed in any preceding claim or a pharmaceutically-acceptable salt, or in-vivo hydrolysable ester thereof wherein Rc is R¹³CO- and R¹³ is (1-4C)alkoxycarbonyl, hydroxy(1-4C)alkyl, (1-4C)alkyl (optionally substituted by one or two hydroxy groups, or by an (1-4C)alkanoyl group), (1-4C)alkylamino, dimethylamino(1-4C)alkyl, (1-4C)alkoxymethyl, (1-4C)alkanoylmethyl, (1-4C)alkanoyloxy(1-4C)alkyl, (1-5C)alkoxy or 2-cyanoethyl.

9. A compound as claimed in any preceding claim or a pharmaceutically-acceptable salt, or in-vivo hydrolysable ester thereof wherein Rc is R¹³CO- and R¹³ is 1,2-dihydroxyethyl, 1,3-dihydroxyprop-2-yl, 1,2,3-trihydroxyprop-1-yl, methoxycarbonyl, hydroxymethyl, methyl, methylamino, dimethylaminomethyl, methoxymethyl, acetoxymethyl, methoxy, methylthio, naphthyl, tert-butoxy or 2-cyanoethyl.

10. A compound of the formula (IA) as claimed in any preceding claim, or a pharmaceutically-acceptable salt, or in-vivo hydrolysable ester thereof, for use as a medicament.

11. The use of a compound of the formula (IA) as claimed in any preceding claim, or a pharmaceutically-acceptable salt, or in-vivo hydrolysable ester thereof, in the manufacture of a medicament for use in the production of an antibacterial effect in a warm blooded animal.

12. A pharmaceutical composition which comprises a compound of the formula (IA) as claimed in any preceding claim, or a pharmaceutically-acceptable salt or an in-vivo hydrolysable ester thereof, and a pharmaceutically-acceptable diluent or carrier.

13. A method of manufacture of a compound as claimed in any preceding claim and pharmaceutically-acceptable salts and in vivo hydrolysable esters thereof, according to a process (a) to (h) as follows (wherein the variables are as defined above unless otherwise stated); Q is phenylene substituted with R² and R³:
(a) by modifying a substituent in or introducing a substituent into another compound of formula (I): or
(b) by reaction of a compound of formula (II): 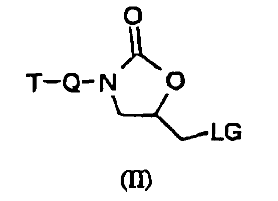 wherein LG is a displaceable group, with a compound of the formula (III):
HET (III)
wherein HET is HET-H free-bass form or HET- anion formed from the free base form, or
(c) by reaction of a compound of the formula (IV) :
T-Q-LG1 (IV)
wherein LG1 is an isocyanate, amine or urethane group with an epoxide of the formula (V); or with a related compound of formula (VA) where the hydroxy group at the internal C-atom is conventionally protected and where the leaving group Y at the terminal C-atom is a conventional leaving group; or 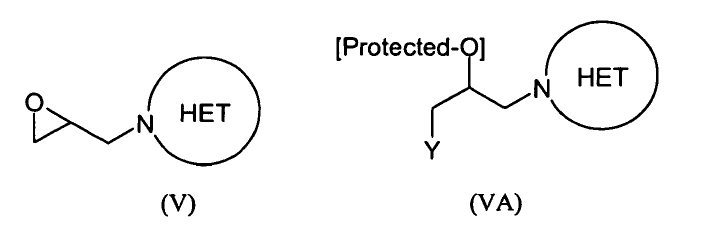
(d) by oxidation
(i) with an aminating agent of a lower valent sulfur compound (VI), or an analogue thereof, which is suitable to give a T substituent as defined by (TA2), or a bi-, or tri-cyclic ring analogue of (VI) which is suitable to give a T substituent as defined by (TB); or
(ii) with an oxygenating agent of a lower valent sulfur compound (VII), or an analogue thereof, which is suitable to give a T substituent as defined by (TA2), or a bi-, or tri-cyclic ring analogue of (VII) which is suitable to give a T substituent as defined by (TB); 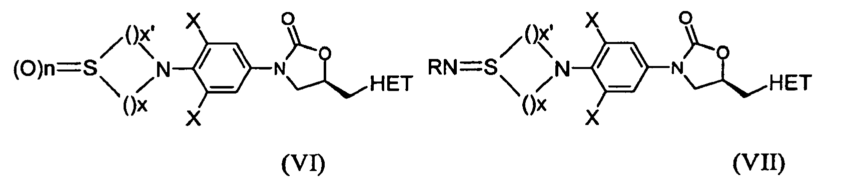
where n = 0 or 1 and ()x and ()x' are chains of length x and x'.; or
(e) (i) by coupling of a compound of formula (VIII) : 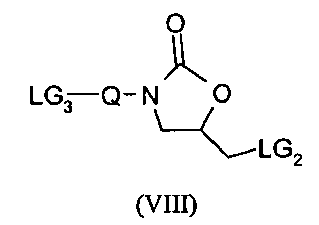 wherein LG2 is a group HET as hereinbefore defined and LG3 is a replaceable substituent, with a compound of the formula (IX), or an analogue thereof, which is suitable to give a T substituent as defined by (TA1), in which the link is via an sp² carbon atom, or (TA2), or a bi-, or tri-cyclic ring analogue of (IX) which is suitable to give a T substituent as defined by (TB); 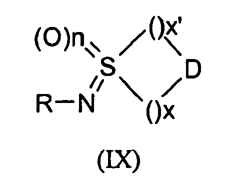 where n = 0 or 1 and ()x and ()x' are chains of length x and x'; D is NH or CH=C-Lg4 where Lg4 is a leaving group ; or
(e) (ii) by coupling, of a compound of formula (X): 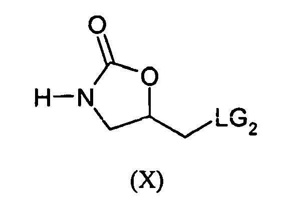 wherein LG2 is a group HET as hereinbefore defined, with a compound [Aryl]-LG4, where LG4 is a replaceable substituent; or
(f) for HET as 1,2,3-triazole by cycloaddition via the azide (wherein e.g. LG in (II) is azide); or
(g) Where HET is 4-substituted 1,2,3-triazole by reaction of a compound of formula (II) where LG = NH₂ (primary amine) with a compound of formula (XI), namely the arenesulfonylhydrazone of a methyl ketone that is further geminally substituted on the methyl group by two substituents (Y' and Y") capable of being eliminated from this initial, and the intermediate, substituted hydrazones as HY' and HY" (or as conjugate bases thereof); 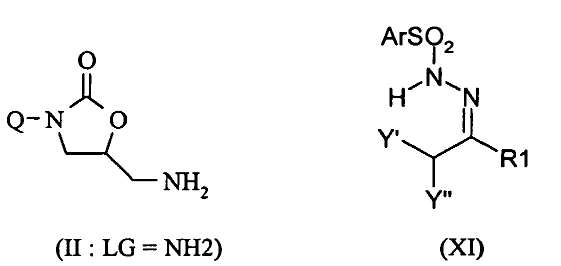
(h) by reduction of a compound formed by process (e) (i) in which the T substituent (as defined by (TA1)) is linked via an sp² carbon atom, to form the saturated analogue;
and thereafter if necessary: (i) removing any protecting groups; (ii) forming a pharmaceutically-acceptable salt; (iii) forming an in-vivo hydrolysable ester.

## Patentansprüche

1. Verbindung der Formel (IA) oder ein pharmazeutisch annehmbares Salz davon oder ein in vivo hydrolysierbarer Ester davon: 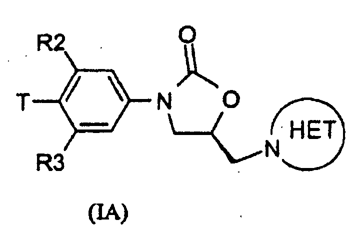 worin HET für 1,2,3-Triazol, 1,2,4-Triazol oder Tetrazol oder eine Dihydro-Version von Pyrimidin, Pyridazin, Pyrazin, 1,2,3-Triazin, 1,2,4-Triazin, 1,3,5-Triazin und Pyridin steht und gegebenenfalls an einem verfügbaren C-Atom außer einem dem verknüpfenden N-Atom benachbarten C-Atom durch einen unter C₁₋₄-Alkyl, C₂₋₄-Alkenyl, C₃₋₆-Cycloalkyl, Amino, C₁₋₄-Alkylamino, Di-C₁₋₄-alkylamino, C₁₋₄-Alkylthio, C₁₋₄-Alkoxy, C₁₋₄-Alkoxycarbonyl, Halogen, Cyano und Trifluormethyl ausgewählten Substituenten substituiert ist und/oder an einem verfügbaren Stickstoffatom (mit der Maßgabe, daß der Ring **dadurch** nicht quaternisiert wird) durch C₁₋₄-Alkyl substituiert ist, wobei Alkyl-, Alkenyl- und Cycloalkyl-HET-Substituenten bei jedem Auftreten jeweils gegebenenfalls ein- oder mehrfach durch F, Cl oder CN substituiert sind;
R² und R³ unabhängig voneinander für Wasserstoff oder Fluor stehen und
T unter (TA1), (TA2) und (TB1) bis (TB3) ausgewählt ist, worin (TA1), (TA2) und (TB1) bis (TB3):
(TA) T unter den folgenden Gruppen (TA1) und (TA2) ausgewählt ist: 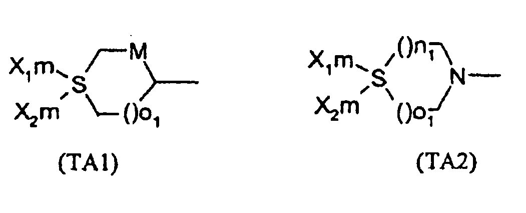 worin:
in (TA1) ()o₁ für 0 oder 1 steht und eine Kette von Kohlenstoffatomen (gegebenenfalls substituiert wie für AR1 definiert) der Länge o₁ darstellt und M für eine die benachbarten Kohlenstoffatome verbindende Gruppe steht oder M ein oder zwei Kohlenstoffatome darstellt und einen 4- bis 7-gliedrigen monocyclischen Ring definiert, welcher gegebenenfalls
(i) eine Doppelbindung zwischen zwei Ringkohlenstoffatomen oder
(ii) eine zwei geeignete, nicht benachbarte Ringkohlenstoffatome verbindende C₁-C₃-Brücke, die gegebenenfalls ein unter Sauerstoff oder >NRc ausgewähltes Heteroatom enthalten kann; oder
(iii) eine cyclische C₂-C₅-Gruppierung, die ein Ringkohlenstoffatom einschließt und so ein C₂-C₅₋Spiroringsystem definiert, wobei der Ring gegebenenfalls ein unter Sauerstoff oder >NRc ausgewähltes Heteroatom enthalten kann; oder
(iv) eine C₁-C₄-Brücke, die benachbarte Kohlenstoffatome verbindet und so einen anellierten Ring definiert, wobei eine C₂-C₄-Brücke gegebenenfalls ein unter Sauerstoff oder >NRc ausgewähltes Heteroatom enthalten kann; wobei Rc die nachstehend aufgeführte Bedeutung besitzt;
aufweisen kann;
worin in (TA2) ()n₁ und ()o₁ unabhängig voneinander für 0, 1 oder 2 stehen und Ketten von Kohlenstoffatomen (gegebenenfalls substituiert wie für AR1 definiert) der Länge n₁ bzw. o₁ darstellen und einen 4- bis 8-gliedrigen monocyclischen Ring definieren, welcher gegebenenfalls
(i) eine zwei geeignete, nicht benachbarte Ringkohlenstoffatome verbindende C₁-C₃-Brücke, die ein unter Sauerstoff oder >NRc ausgewähltes Heteroatom enthält; oder
(ii) eine cyclische C₂-C₅-Gruppierung, die ein Ringkohlenstoffatom einschließt und so ein C₂-C₅₋Spiroringsystem definiert, wobei der Ring gegebenenfalls ein unter Sauerstoff oder >NRc ausgewähltes Heteroatom enthalten kann; oder
(iii) eine C₁-C₄-Brücke, die benachbarte Kohlenstoffatome verbindet und so einen anellierten Ring definiert, wobei eine C₂-C₄-Brücke gegebenenfalls ein unter Sauerstoff oder >NRc ausgewähltes Heteroatom enthalten kann; wobei Rc die nachstehend aufgeführte Bedeutung besitzt; aufweisen kann; und
worin in (TA1) und (TA2) X₁ₘ und X₂ₘ zusammengenommen R₂ₛ- (E)ₘₛ-N= darstellen oder X₁ₘ für O= steht und X₂ₘ für R₂ₛ-(E)ₘₛ-N- steht und umgekehrt;
worin E für eine unter -SO₂-, -CO-, -O-CO-, -CO-O-, -CS-, -CON(Rₛ)-, -SO₂N(Rₛ)- ausgewählte elektronenanziehende Gruppe steht oder eine Gruppe der Formel R₃ₛ-C(=N-O-R₃ₛ)-C(=O)-, worin R₃ₛ für H steht oder die unten bei (i) in R₂ₛ angegebene Bedeutung besitzt, darstellen kann;
oder dann, wenn E für -CON(Rₛ)- oder -SO₂N(Rₛ)-steht, R₂ₛ und Rₛ gemeinsam eine Kohlenstoffkette bilden können, die einen 5- oder 6-gliedrigen gesättigten, ungesättigten oder teilweise ungesättigten Ring definiert, der über das N-Atom in E verknüpft ist, gegebenenfalls weiterhin durch einen Oxosubstituenten substituiert ist und gegebenenfalls mit einer Phenylgruppe unter Bildung eines benzoanellierten Systems anelliert sein kann, wobei die Phenylgruppe gegebenenfalls durch bis zu drei unabhängig voneinander unter Halogen, Cyano, C₁₋₄-Alkyl und C₁₋₄-Alkoxy ausgewählte Substituienten substituiert ist;
ms für 0 oder 1 steht;
R₂ₛ und Rₛ unabhängig voneinander ausgewählt sind unter:
(i) Wasserstoff (außer wenn E für -SO₂- oder -O-COsteht) oder C₁₋₆-Alkyl {gegebenenfalls substituiert durch eine oder mehrere C₁₋₄-Alkanoylgruppen (einschließlich geminaler Disubstitution) und/oder gegebenenfalls einfach substituiert durch Cyano, Cyanoimino, C₁₋₄-Alkoxy, Trifluormethyl, C₁₋₄₋Alkoxycarbonyl, Phenyl (gegebenenfalls substituiert wie hier für AR1 definiert), eine gegebenenfalls substituierte Heteroarylgruppe der Formel AR2, AR2a, AR2b, AR3, AR3a, AR3b, AR4, AR4a oder CY, alle wie definiert (und gegebenenfalls substituiert wie definiert), C₁₋₄-Alkyl-S(O)_{q-}(wobei q 0, 1 oder 2 bedeutet) und/oder (mit der Maßgabe, daß dann, wenn R₂₅ für -SO₂ oder -O-COsteht, nicht am ersten Kohlenstoffatom der C₁₋₆₋Alkylkette) gegebenenfalls substituiert durch eine oder mehrere Gruppen (einschließlich geminaler Disubstitution), die jeweils unabhängig voneinander unter Hydroxy und Fluor ausgewählt sind, und/oder gegebenenfalls weiterhin substituiert durch jeweils höchstens eine der Gruppen Oxo, NRvRw [worin Rv für Wasserstoff oder C₁₋₄₋Alkyl steht und Rw für Wasserstoff oder C₁₋₄-Alkyl steht], C₁₋₆-Alkanoylamino, C₁₋₄-Alkoxycarbonyl-amino, N-C₁₋₄-Alkyl-N-C₁₋₆-alkanoylamino, C₁₋₄-AlkylS(O)ₚNH- oder C₁₋₄-Alkyl-S(O)ₚ-(C₁₋₄-alkyl)N- (wobei p 1 oder 2 bedeutet)}; oder
(ii) einer gegebenenfalls substituierten Arylgruppe oder gegebenenfalls substituierten Heteroarylgruppe der Formel AR1, AR2, AR2a, AR2b, AR3, AR3a, AR3b, AR4, AR4a oder CY, alle wie hier definiert (und gegebenenfalls substituiert wie definiert);
oder (nur wenn ms für 0 steht)
(iii) Cyano, -CO-NRvRw, -CO-NRvRw', -SO₂-NRvRw, -SO₂-NRvRw' [worin Rv für Wasserstoff oder C₁₋₄₋Alkyl steht; Rw für Wasserstoff oder C₁₋₄-Alkyl steht und Rw' für Phenyl (gegebenenfalls substituiert wie hier für AR1 definiert) oder eine unter AR2, AR2a, AR2b, AR3, AR3a, AR3b, AR4, AR4a (gegebenenfalls substituiert wie hier definiert) ausgewählte Heteroarylgruppe steht], C₁₋₄-Alkoxycarbonyl, Trifluormethyl, Ethenyl, 2-C₁₋₄₋Alkylethenyl, 2-Cyanoethenyl, 2-Cyano-2-(C₁₋₄₋alkyl) ethenyl, 2-Nitroethenyl, 2-Nitro-2-(C₁₋₄₋alkyl)ethenyl, 2-(C₁₋₄-Alkylaminocarbonyl)ethenyl, 2-(C₁₋₄-Alkoxycarbonyl)ethenyl, 2-(AR1)-Ethenyl, 2-(AR2)-Ethenyl oder 2-(AR2a)-Ethenyl; oder (TB) T unter den folgenden Gruppen (TB1) bis (TB3) ausgewählt ist: 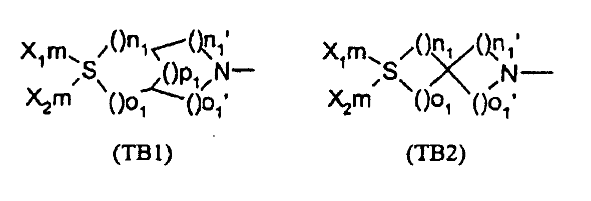 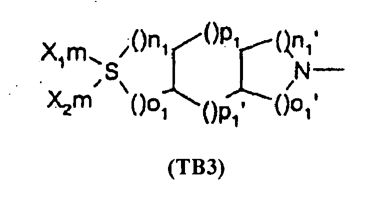
worin:
X₁ₘ und X₂ₘ zusammengenommen R₂ₛ-(E)ₘₛ-N= darstellen oder
X₁ₘ für O= steht und X₂ₘ für R₂ₛ-(E)ₘₛ-N- steht und umgekehrt;
worin E für eine unter -SO₂-, -CO-, -O-CO-, -CO-O-, -CS-, -CON(Rₛ)-, -SO₂N(Rₛ) - ausgewählte elektronenanziehende Gruppe steht oder eine Gruppe der Formel R₃ₛ-C(=N-O-R₃ₛ)-C(=O)-, worin R₃ₛ für H steht oder die unten bei (i) in R₂ₛ angegebene Bedeutung besitzt, darstellen kann;
oder dann, wenn E für -CON(Rₛ)- oder -SO₂N(Rₛ)-steht, R₂ₛ und Rₛ gemeinsam eine Kohlenstoffkette bilden können, die einen 5- oder 6-gliedrigen gesättigten, ungesättigten oder teilweise ungesättigten Ring definiert, der über das N-Atom in E verknüpft ist, gegebenenfalls weiterhin durch einen Oxosubstituenten substituiert ist und gegebenenfalls mit einer Phenylgruppe unter Bildung eines benzoanellierten Systems anelliert sein kann, wobei die Phenylgruppe gegebenenfalls durch bis zu drei unabhängig voneinander unter Halogen, Cyano, C₁₋₄-Alkyl und C₁₋₄-Alkoxy ausgewählte Substituienten substituiert ist;
ms für 0 oder 1 steht;
R₂ₛ und Rₛ unabhängig voneinander ausgewählt sind unter:
(i) Wasserstoff (außer wenn E für -SO₂- oder -O-CO-steht) oder C₁₋₆-Alkyl {gegebenenfalls substituiert durch eine oder mehrere C₁₋₄-Alkanoylgruppen (einschließlich geminaler Disubstitution) und/oder gegebenenfalls einfach substituiert durch Cyano, Cyanoimino, C₁₋₄-Alkoxy, Trifluormethyl, C₁₋₄₋Alkoxycarbonyl, Phenyl (gegebenenfalls substituiert wie für AR1), eine gegebenenfalls substituierte Heteroarylgruppe der Formel AR2, AR2a, AR2b, AR3, AR3a, AR3b, AR4, AR4a oder CY, alle wie hier definiert, C₁₋₄-Alkyl-S(O)_{q}- (wobei q 0, 1 oder 2 bedeutet) und/oder (mit der Maßgabe, daß dann, wenn R₂₅ für -SO₂- oder -O-CO- steht, nicht am ersten Kohlenstoffatom der C₁₋₆-Alkylkette) gegebenenfalls substituiert durch eine oder mehrere Gruppen (einschließlich geminaler Disubstitution), die jeweils unabhängig voneinander unter Hydroxy und Fluor ausgewählt sind, und/oder gegebenenfalls weiterhin substituiert durch jeweils höchstens eine der Gruppen Oxo, NRvRw [worin Rv für Wasserstoff oder C₁₋₄₋Alkyl steht und Rw für Wasserstoff oder C₁₋₄-Alkyl steht], C₁₋₆-Alkanoylamino, C₁₋₄-Alkoxycarbonyl-amino, N-C₁₋₄-Alkyl-N-C₁₋₆-alkanoylamino, C₁₋₄-Alkyl-S(O)ₚNH- oder C₁₋₄-Alkyl-S(O)ₚ-(C₁₋₄-alkyl) N- (wobei p 1 oder 2 bedeutet)}; oder
(ii) einer gegebenenfalls substituierten Aryl-gruppe oder gegebenenfalls substituierten Heteroarylgruppe der Formel AR1, AR2, AR2a, AR2b, AR3, AR3a, AR3b, AR4, AR4a oder CY, alle wie hier definiert;
oder (nur wenn ms für 0 steht)
(iii) Cyano, -CO-NRvRw, -CO-NRvRw', -SO₂-NRvRw, -SO₂-NRvRw' [worin Rv für Wasserstoff oder C₁₋₄₋Alkyl steht; Rw für Wasserstoff oder C₁₋₄-Alkyl steht und Rw' für Phenyl (gegebenenfalls substituiert wie für AR1) oder eine unter AR2, AR2a, AR2b, AR3, AR3a, AR3b, AR4, AR4a (wie hier definiert) ausgewählte Heteroarylgruppe steht], C₁₋₄-Alkoxycarbonyl, Trifluormethyl, Ethenyl, 2-C₁₋₄-Alkylethenyl, 2-Cyanoethenyl, 2-Cyano-2-(C₁₋₄₋alkyl)ethenyl, 2-Nitroethenyl, 2-Nitro-2-(C₁₋₄₋alkyl)ethenyl, 2-(C₁₋₄-Alkylaminocarbonyl)ethenyl, 2-(C₁₋₄-Alkoxycarbonyl)ethenyl, 2-(AR1)-Ethenyl, 2-(AR2)-Ethenyl oder 2-(AR2a)-Ethenyl; und
worin ()n₁, ()o₁, ()n_{1'}, ()o_{1'}, ()p₁ und ()p_{1'} Ketten von Kohlenstoffatomen (gegebenenfalls substituiert wie für AR1) der Länge n₁, o₁, n_{1'}, o_{1'}, p₁ bzw. p_{1'} darstellen und unabhängig voneinander für 0-2 stehen, mit der Maßgabe, daß in (TB1) und (TB2) die Summe von n₁, o₁, n₁, und o₁, höchstens 8 beträgt (was eine maximale Ringgröße von 14 in (TB1) und 11 in (TB2) ergibt) und in (TB3) die Summe von n₁, o₁, n_{1'}, o_{1'}, p₁ und p₁, höchstens 6 beträgt (was eine maximale Ringgröße von 12 ergibt);
worin Rc unter den Gruppen (Rc1) bis (Rc5) ausgewählt ist:
*(Rc1)* gegebenenfalls substituiertes C₁₋₆-Alkyl;
*(Rc2)* R¹³CO-, R¹³SO₂- oder R¹³CS-,
worin R¹³ unter (Rc2a) bis (Rc2e) ausgewählt ist:
*(Rc2a)* AR1, AR2, AR2a, AR2b, AR3, AR3a, AR3b, AR4, AR4a, CY;
*(R2cb)* Wasserstoff, C₁₋₄-Alkoxycarbonyl, Trifluormethyl, -NRvRw [worin Rv für Wasserstoff oder C₁₋₄₋Alkyl steht und Rw für Wasserstoff oder C₁₋₄-Alkyl steht], Ethenyl, 2-C₁₋₄-Alkylethenyl, 2-Cyanoethenyl, 2-Cyano-2-(C₁₋₄-alkyl)ethenyl, 2-Nitroethenyl, 2-Nitro-2-(C₁₋₄-alkyl) ethenyl, 2-(C₁₋₄₋Alkylaminocarbonyl)ethenyl, 2-(C₁₋₄-Alkoxycarbonyl)ethenyl, 2-(AR1)-Ethenyl, 2-(AR2)-Ethenyl, 2-(AR2a)-Ethenyl;
*(Rc2c)* gegebenenfalls substituiertes C₁₋₁₀-Alkyl;
*(Rc2d)* R¹⁴C(O)O-C₁₋₆-alkyl, worin R¹⁴ für AR1, AR2, C₁₋₄-Alkylamino (wobei die C₁₋₄-Alkylgruppe gegebenenfalls durch C₁₋₄-Alkoxycarbonyl oder Carboxy substituiert ist), Benzyloxy-C₁₋₄-alkyl oder C₁₋₁₀-Alkyl {gegebenenfalls substituiert wie für (Rc2c) definiert}, steht;
*(Rc2e)* R¹⁵O-, worin R¹⁵ für Benzyl, C₁₋₆-Alkyl {gegebenenfalls substituiert wie für (Rc2c) definiert}, CY oder AR2b steht;
*(Rc3)* Wasserstoff, Cyano, 2-Cyanoethenyl, 2-Cyano-2- (C₁₋₄-alkyl) ethenyl, 2-(C₁₋₄-Alkylaminocarbonyl)-ethenyl, 2-(C₁₋₄-Alkoxycarbonyl)ethenyl, 2-Nitroethenyl, 2-Nitro-2-(C₁₋₄-alkyl) ethenyl, 2-(AR1) - Ethenyl, 2-(AR2)-Ethenyl oder der Formel (Rc3a) 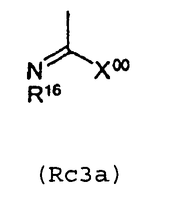 worin X⁰⁰ für -OR¹⁷, -SR¹⁷, -NHR¹⁷ und -N(R¹⁷)₂ steht;
worin R¹⁷ Wasserstoff bedeutet (wenn X⁰⁰ für -NHR¹⁷ und -N(R¹⁷)₂ steht) und R¹⁷ C₁₋₄-Alkyl, Phenyl oder AR2 bedeutet (wenn X⁰⁰ für -OR¹⁷, -SR¹⁷ und -NHR¹⁷ steht) und R¹⁶ Cyano, Nitro, C₁₋₄-Alkylsulfonyl, C₄₋₇-Cycloalkylsulfonyl, Phenylsulfonyl, C₁₋₄₋Alkanoyl und C₁₋₄-Alkoxycarbonyl bedeutet;
(*Rc4*) Trityl, AR1, AR2, AR2a, AR2b, AR3, AR3a, AR3b;
*(Rc5)* RdOC(Re)=CH(C=O)-, RfC(=O)C(=O)-, RgN=C (Rh) C(=O) - oder RiNHC(Rj)=CHC(=O)-, worin Rd für C₁₋₆-Alkyl steht; Re für Wasserstoff oder C₁₋₆₋Alkyl steht; oder Rd und Re gemeinsam eine C₃₋₄₋Alkylenkette bilden; Rf für Wasserstoff, C₁₋₆₋Alkyl, Hydroxy-C₁₋₆-alkyl, C₁₋₆-Alkoxy-C₁₋₆-alkyl, -NRvRw [worin Rv für Wasserstoff oder C₁₋₄-Alkyl steht; Rw für Wasserstoff oder C₁₋₄-Alkyl steht], C₁₋₆-Alkoxy, C₁₋₆-Alkoxy-C₁₋₆-alkoxy, Hydroxy-C₂₋₆₋alkoxy, C₁₋₄-Alkylamino-C₂₋₆-alkoxy, Di-C₁₋₄-alkylamino-C₂₋₆-alkoxy steht; Rg für C₁₋₆-Alkyl, Hydroxy oder C₁₋₆-Alkoxy steht; Rh für Wasserstoff oder C₁₋₆-Alkyl steht; Ri für Wasserstoff, C₁₋₆-Alkyl, AR1, AR2, AR2a, AR2b steht und Rj für Wasserstoff oder C₁₋₆-Alkyl steht;
worin:
CY für einen gegebenenfalls substituierten Cyclobutyl-, Cyclopentyl-, Cyclohexyl-, Cyclopentenyl- oder Cyclohexenylring steht;
AR1 für gegebenenfalls substituiertes Phenyl oder gegebenenfalls substituiertes Naphthyl steht;
AR2 für einen gegebenenfalls substituierten 5-oder 6-gliedrigen, vollständig ungesättigten (d.h. mit dem maximalen Ungesättigtheitsgrad) monocyclischen Heteroarylring, der bis zu vier unabhängig voneinander unter O, N und S ausgewählte Heteroatome (aber keine O-O-, O-S-oder S-S-Bindungen) enthält und über ein Ringkohlenstoffatom oder, sofern der Ring **dadurch** nicht quaternisiert wird, ein Ringstickstoffatom verknüpft ist, steht;
AR2a für eine teilhydrierte Version von AR2 (d.h. AR2-Systeme, die einen bestimmten, aber nicht den vollen Ungesättigtheitsgrad behalten), die über ein Ringkohlenstoffatom oder, sofern der Ring **dadurch** nicht quaternisiert wird, ein Ringstickstoffatom verknüpft ist, steht;
**AR2b** für eine vollhydrierte Version von AR2 (d.h. AR2-Systeme ohne Ungesättigtheit), die über ein Ringkohlenstoffatom oder ein Ringstickstoffatom verknüpft ist, steht;
**AR3** für einen gegebenenfalls substituierten 8-, 9- oder 10-gliedrigen, vollständig ungesättigten (d.h. mit dem maximalen Ungesättigtheitsgrad) bicyclischen Heteroarylring, der bis zu vier unabhängig voneinander unter O, N und S ausgewählte Heteroatome (aber keine O-O-, O-S- oder S-S-Bindungen) enthält und über ein Ringkohlenstoffatom in einem der beiden das bicyclische System bildenden Ringe verknüpft ist, steht;
**AR3a** für eine teilhydrierte Version von AR3 (d.h. AR3-Systeme, die einen bestimmten, aber nicht den vollen Ungesättigtheitsgrad behalten), die über ein Ringkohlenstoffatom oder, sofern der Ring **dadurch** nicht quaternisiert wird, ein Ringstickstoffatom in einem der beiden das bicyclische System bildenden Ringe verknüpft ist, steht;
**AR3b** für eine vollhydrierte Version von AR3 (d.h. AR3-Systeme ohne Ungesättigtheit), die über ein Ringkohlenstoffatom oder ein Ringstickstoffatom in einem der beiden das bicyclische System bildenden Ringe verknüpft ist, steht;
**AR4** für einen gegebenenfalls substituierten 13- oder 14-gliedrigen, vollständig ungesättigten (d.h. mit dem maximalen Ungesättigtheitsgrad) tricyclischen Heteroarylring, der bis zu vier unabhängig voneinander unter O, N und S ausgewählte Heteroatome (aber keine O-O-, O-S- oder S-S-Bindungen) enthält und über ein Ringkohlenstoffatom in einem der das tricyclische System bildenden Ringe verknüpft ist, steht;
**AR4a** für eine teilhydrierte Version von AR4 (d.h. AR4-Systeme, die einen bestimmten, aber nicht den vollen Ungesättigtheitsgrad behalten), die über ein Ringkohlenstoffatom oder, sofern der Ring **dadurch** nicht quaternisiert wird, ein Ringstickstoffatom in einem der das tricyclische System bildenden Ringe verknüpft ist, steht;
worin die Substituenten an AR1, AR2, AR2a, AR2b, AR3, AR3a, AR3b, AR4, AR4a und CY bis zu drei unabhängig voneinander unter C₁₋₄-Alkyl {gegebenenfalls substituiert durch unabhängig voneinander unter Hydroxy, Trifluormethyl, C₁₋₄Alkyl-S(O)_{q-}(wobei q 0, 1 oder 2 bedeutet) (wobei dieser letzte Substituent vorzugsweise nur an AR1 steht), C₁₋₄-Alkoxy, C₁₋₄-Alkoxycarbonyl, Cyano, Nitro, C₁₋₄₋Alkanoylamino, -CONRvRw oder -NRvRw ausgewählte Substituenten (vorzugsweise einen Substituenten)}, Trifluormethyl, Hydroxy, Halogen, Nitro, Cyano, Thiol, C₁₋₄-Alkoxy, C₁₋₄-Alkanoyloxy, Dimethylaminomethylenaminocarbonyl, Di(N-C₁₋₄-alkyl)aminomethyl-imino, Carboxy, C₁₋₄-Alkoxycarbonyl, C₁₋₄-Alkanoyl, C₁₋₄-Alkyl-SO₂-amino, C₂₋₄-Alkenyl {gegebenenfalls substituiert durch Carboxy oder C₁₋₄-Alkoxycarbonyl}, C₂₋₄-Alkinyl, C₁₋₄-Alkanoylamino, Oxo (=O), Thioxo (=S), C₁₋₄-Alkanoylamino {wobei die C₁₋₄-Alkanoylgruppe {egebenenfalls durch Hydroxy substituiert ist}, C₁₋₄-Alkyl-S(O)_{q}- (wobei q 0, 1 oder 2 bedeutet) {wobei die C₁₋₄-Alkylgruppe gegebenenfalls durch eine oder mehrere, unabhängig voneinander unter Cyano, Hydroxy und C₁₋₄-Alkoxy ausgewählte Gruppen substituiert ist}, -CONRvRw oder -NRvRw [worin Rv für Wasserstoff oder C₁₋₄₋Alkyl steht und Rw für Wasserstoff oder C₁₋₄-Alkyl steht] ausgewählte Substituenten (an einem verfügbaren Kohlenstoffatom) sind
und worin weitere geeignete Substituenten an AR1, AR2, AR2a, AR2b, AR3, AR3a, AR3b, AR4, AR4a und CY (an einem verfügbaren Kohlenstoffatom) und auch an Alkylgruppen (sofern nicht anders vermerkt) bis zu drei unabhängig voneinander unter Trifluormethoxy, Benzoylamino, Benzoyl, Phenyl {gegebenenfalls substituiert durch bis zu drei, unabhängig voneinander unter Halogen, C₁₋₄-Alkoxy oder Cyano ausgewählte Substituenten}, Furan, Pyrrol, Pyrazol, Imidazol, Triazol, Pyrimidin, Pyridazin, Pyridin, Isoxazol, Oxazol, Isothiazol, Thiazol, Thiophen, Hydroxyimino-C₁₋₄-alkyl, C₁₋₄-Alkoxyimino-C₁₋₄-alkyl, Halogen-C₁₋₄-alkyl, C₁₋₄₋Alkansulfonamido, -SO₂NRvRw [worin Rv für Wasserstoff oder C₁₋₄-Alkyl steht und Rw für Wasserstoff oder C₁₋₄-Alkyl steht] ausgewählte Substituenten sind.

2. Verbindung der Formel (IA) nach Anspruch 1 oder ein pharmazeutisch annehmbares Salz oder ein in vivo hydrolysierbarer Ester davon, worin HET für 1,2,3-Triazol, 1,2,4-Triazol oder Tetrazol steht; R² und R³ unabhängig voneinander für Wasserstoff oder Fluor stehen und
T unter (TA1a & b), (TA2a) und (TB1a & b) ausgewählt ist.

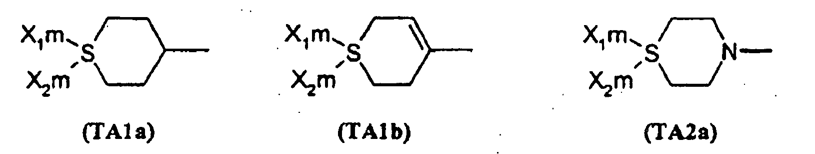

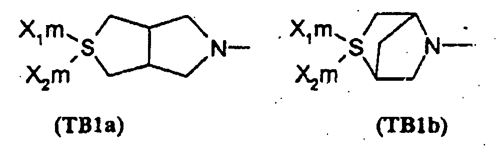

3. Verbindung der Formel (IA) nach Anspruch 2 oder ein pharmazeutisch annehmbares Salz oder ein in vivo hydrolysierbarer Ester davon, worin
T unter (TA1a & b) und (TA2a) ausgewählt ist.

4. Verbindung nach einem der vorhergehenden Ansprüche oder ein pharmazeutisch annehmbares Salz oder ein in vivo hydrolysierbarer Ester davon, worin HET durch Methyl (gegebenenfalls ein- oder mehrfach durch F, Cl oder CN substituiert) substituiert ist.

5. Verbindung nach einem der vorhergehenden Ansprüche oder ein pharmazeutisch annehmbares Salz oder ein in vivo hydrolysierbarer Ester davon, worin HET unsubstituiert ist.

6. Verbindung nach einem der vorhergehenden Ansprüche oder ein pharmazeutisch annehmbares Salz oder ein in vivo hydrolysierbarer Ester davon, worin X₁ₘ für O= steht und X₂ₘ für R₂ₛ-(E)ₘₛ-N- steht und umgekehrt; und dann, wenn ms für 0 steht, R₂ₛ ausgewählt ist unter
(i) Wasserstoff, einer C₁₋₆-Alkylgruppe {gegebenenfalls einfach substituiert durch eine C₁₋₄₋Alkanoylgruppe, Cyano, Cyanoimino, C₁₋₄-Alkoxy, Trifluormethyl, C₁₋₄-Alkoxycarbonyl, Phenyl (gegebenenfalls substituiert wie hier für AR1 definiert), eine gegebenenfalls substituierte Heteroarylgruppe der Formel AR2, AR2a, AR2b, AR3, AR3a, AR3b, AR4, AR4a oder CY, alle wie hier definiert (und gegebenenfalls substituiert wie definiert), C₁₋₄-Alkyl-S(O)_{q}- (wobei q 0, 1 oder 2 bedeutet) und/oder gegebenenfalls substituiert durch eine oder mehrere Fluorgruppen (einschließlich geminaler Disubstitution) und/oder gegebenenfalls substituiert durch eine oder mehrere Hydroxygruppen (einschließlich geminaler Disubstitution) und/oder gegebenenfalls weiterhin substituiert durch jeweils höchstens eine der Gruppen Oxo, NRvRw [worin Rv für Wasserstoff oder C₁₋₄-Alkyl steht und Rw für Wasserstoff oder C₁₋₄₋Alkyl steht], C₁₋₆-Alkanoylamino, C₁₋₄-Alkoxy-carbonylamino, N-C₁₋₄-Alkyl-N-C₁₋₆-alkanoylamino, C₁₋₄-Alkyl-S(O)ₚNH- oder C₁₋₄-Alkyl-S(O)ₚ-(C₁₋₄₋alkyl)N- (wobei p 1 oder 2 bedeutet)}; oder
(ii) einer gegebenenfalls substituierten Arylgruppe oder gegebenenfalls substituierten Heteroarylgruppe der Formel AR1, AR2, AR2a, AR2b, AR3, AR3a, AR3b, AR4, AR4a oder CY, alle wie hier definiert (und gegebenenfalls substituiert wie definiert); oder
(iii) Cyano, -CO-NRvRw, -CO-NRvRw', -SO₂-NRvRw, -SO₂-NRvRw' [worin Rv für Wasserstoff oder C₁₋₄₋Alkyl steht; Rw für Wasserstoff oder C₁₋₄-Alkyl steht und Rw' für Phenyl (gegebenenfalls substituiert wie für hier definiertes AR1) oder eine unter AR2, AR2a, AR2b, AR3, AR3a, AR3b, AR4, AR4a gegebenenfalls substituiert (wie hier definiert) ausgewählte Heteroarylgruppe steht], C₁₋₄-Alkoxycarbonyl, Trifluormethyl;
und dann, wenn ms für 1 steht, E für -CO- oder -SO₂- steht und R₂ₛ ausgewählt ist unter
(i) C₁₋₆-Alkyl {gegebenenfalls einfach substituiert durch Cyano, Cyanoimino, C₁₋₄-Alkoxy, Trifluormethyl, C₁₋₄-Alkoxycarbonyl, Phenyl (gegebenenfalls substituiert wie für hier definiertes AR1), eine gegebenenfalls substituierte Heteroarylgruppe der Formel AR2, AR2a, AR2b, AR3, AR3a, AR3b, AR4, AR4a oder CY, alle wie hier definiert (und gegebenenfalls substituiert wie definiert), C₁₋₄-Alkyl-S(O)_{q-}(wobei q 0, 1 oder 2 bedeutet) und/oder (mit der Maßgabe, daß dann, wenn R₂₅ für -SO₂- oder -O-COsteht, nicht am ersten Kohlenstoffatom der C₁₋₆₋Alkylkette) gegebenenfalls substituiert durch eine oder mehrere Gruppen (einschließlich geminaler Disubstitution), die jeweils unabhängig voneinander unter Hydroxy und Fluor ausgewählt sind, und/oder gegebenenfalls einfach substituiert durch -NRvRw [worin Rv für Wasserstoff oder C₁₋₄-Alkyl steht und Rw für Wasserstoff oder C₁₋₄-Alkyl steht], C₁₋₆₋Alkanoylamino, C₁₋₄-Alkoxycarbonylamino, N-C₁₋₄₋Alkyl-N-C₁₋₆-alkanoylamino, C₁₋₄-Alkyl-S(O)ₚNH- oder C₁₋₄-Alkyl-S(O)ₚ-(C₁₋₄-alkyl)N- (wobei p 1 oder 2 bedeutet)}; oder
(ii) einer gegebenenfalls substituierten Aryl- oder Heteroarylgruppe der Formel AR1, AR2, AR2a, AR2b, AR3, AR3a, AR3b, AR4, AR4a oder CY, alle wie hier definiert (und gegebenenfalls substituiert wie hier definiert).

7. Verbindung nach einem der vorhergehenden Ansprüche oder ein pharmazeutisch annehmbares Salz oder ein in vivo hydrolysierbarer Ester davon, worin X₁ₘ für O= steht und X₂ₘ für R₂ₛ-(E)ₘₛ-N- steht und umgekehrt; und dann, wenn ms für 0 steht, R₂ₛ ausgewählt ist unter
(i) Wasserstoff, C₁₋₆-Alkyl {gegebenenfalls einfach substituiert durch C₁₋₄-Alkoxy, Trifluormethyl, C₁₋₄-Alkyl-S(O)_{q}- (wobei q 0, 1 oder 2 bedeutet) und/oder gegebenenfalls substituiert durch eine oder mehrere Fluorgruppen (einschließlich geminaler Disubstitution) und/oder gegebenenfalls substituiert durch eine oder mehrere Hydroxygruppen (einschließlich geminaler Disubstitution); oder
(iii) -CO-NRvRw [worin Rv für Wasserstoff oder C₁₋₄-Alkyl steht und Rw für Wasserstoff oder C₁₋₄₋Alkyl steht], -CO-NRv Rw' [worin Rv für Wasserstoff oder C₁₋₄-Alkyl steht und Rw' für Phenyl (gegebenenfalls substituiert wie für hier definiertes AR1) steht] C₁₋₄-Alkoxycarbonyl;
und dann, wenn ms für 1 steht, E für -CO- oder -SO₂- steht und R₂ₛ ausgewählt ist unter
(i) C₁₋₆-Alkyl {gegebenenfalls einfach substituiert durch C₁₋₄-Alkoxy, Trifluormethyl C₁₋₄-Alkyl-S(O)_{q-}(wobei q 0, 1 oder 2 bedeutet), oder gegebenenfalls substituiert durch eine oder mehrere Fluorgruppen (ausschließlich geminaler Disubstitution), oder gegebenenfalls substituiert durch eine oder mehrere Hydroxygruppen (ausschließlich geminaler Disubstitution))} C₁₋₆-Alkanoylamino, C₁₋₄-Alkoxy-carbonylamino.

8. Verbindung nach einem der vorhergehenden Ansprüche oder ein pharmazeutisch annehmbares Salz oder ein in vivo hydrolysierbarer Ester davon, worin Rc für R¹³CO- steht und R¹³ für C₁₋₄-Alkoxycarbonyl, Hydroxy-C₁₋₄-alkyl, C₁₋₄-Alkyl (gegebenenfalls substituiert durch eine oder zwei Hydroxygruppen oder eine C₁₋₄-Alkanoylgruppe), C₁₋₄-Alkylamino, Dimethylamino-C₁₋₄-alkyl, C₁₋₄-Alkoxymethyl, C₁₋₄₋Alkanoylmethyl, C₁₋₄-Alkanoylmethyl-C₁₋₄-alkyl, C₁₋₅₋Alkoxy oder 2-Cyanoethyl steht.

9. Verbindung nach einem der vorhergehenden Ansprüche oder ein pharmazeutisch annehmbares Salz oder ein in vivo hydrolysierbarer Ester davon, worin Rc für R¹³ CO- steht und R¹³ für 1,2-Dihydroxyethyl, 1,3-Dihydroxyprop-2-yl, 1,2,3-Trihydroxyprop-1-yl, Methoxycarbonyl, Hydroxymethyl, Methyl, Methylamino, Dimethylaminomethyl, Methoxymethyl, Acetoxymethyl, Methoxy, Methylthio, Naphthyl, tert.-Butoxy oder 2-Cyanoethyl steht.

10. Verbindung der Formel (IA) nach einem der vorhergehenden Ansprüche oder ein pharmazeutisch annehmbares Salz oder ein in vivo hydrolysierbarer Ester davon zur Verwendung als Arzneimittel.

11. Verwendung einer Verbindung der Formel (IA) nach einem der vorhergehenden Ansprüche oder eines pharmazeutisch annehmbaren Salzes oder eines in vivo hydrolysierbaren Esters davon bei der Herstellung eines Arzneimittels zur Verwendung bei der Hervorrufung einer antibakteriellen Wirkung bei einem Warmblüter.

12. Pharmazeutische Zusammensetzung, die eine Verbindung der Formel (IA) nach einem der vorhergehenden Ansprüche oder ein pharmazeutisch annehmbares Salz oder einen in vivo hydrolysierbaren Ester davon und ein pharmazeutisch annehmbares Verdünnungsmittel oder einen pharmazeutisch annehmbaren Träger enthält.

13. Verfahren zur Herstellung einer Verbindung der Formel (IA) nach einem der vorhergehenden Ansprüche oder pharmazeutisch annehmbarer Salze oder in vivo hydrolysierbarer Ester davon gemäß einem der folgenden Verfahren (a) bis (h) (worin die Variablen wie oben definiert sind, sofern nicht anders vermerkt, und Q für durch R² und R³ substituiertes Phenylen steht) **dadurch**, daß man:
(a) in einer anderen Verbindung der Formel (I) einen Substituenten modifiziert oder in eine andere Verbindung der Formel (I) einen Substituenten einführt; oder
(b) eine Verbindung der Formel (II) 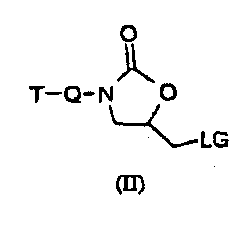 worin LG für eine austauschbare Gruppe steht, mit einer Verbindung der Formel (III):
HET (III)
worin HET für die Form der freien Base HET-H oder aus der Form der freien Base gebildetes HET-Anion steht, umsetzt; oder
(c) eine Verbindung der Formel (IV):
T-Q-LG1 (IV)
worin LG1 für eine Isocyanat-, Amino- oder Urethangruppe steht, mit einem Epoxid der Formel (V) oder mit einer verwandten Verbindung der Formel (VA), in der die Hydroxygruppe am internen C-Atom auf herkömmliche Art und Weise geschützt ist und die Abgangsgruppe Y am terminalen C-Atom für eine herkömmliche Abgangsgruppe steht; oder 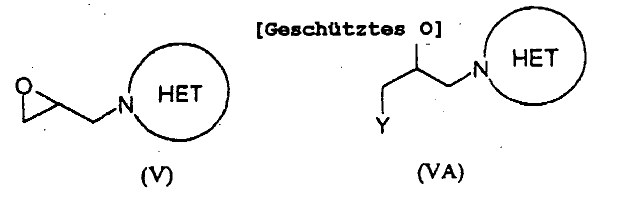 umsetzt;
(d)
(i) eine niederwertige Schwefelverbindung (VI) oder ein Analogon davon, das zur Bildung eines Substituenten T gemäß (TA2) geeignet ist, oder ein bi- oder tricyclisches Analogon von (VI), das zur Bildung eines Substituenten T gemäß (TB) geeignet ist, mit einem Aminierungsmittel oxidiert oder
(ii) eine niederwertige Schwefelverbindung (VII) oder ein Analogon davon, das zur Bildung eines Substituenten T gemäß (TA2) geeignet ist, oder ein bi- oder tricyclisches Analogon von (VII), das zur Bildung eines Substituenten T gemäß (TB) geeignet ist, mit einem Oxygenierungsmittel oxidiert; 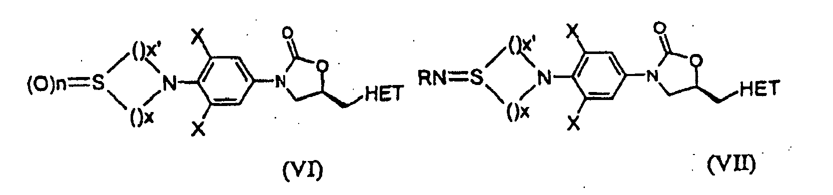
wobei n = 0 oder 1 und ()x und ()x' für Ketten der Länge x und x' stehen; oder
(e)(i) eine Verbindung der Formel (VIII): 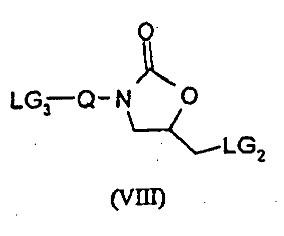 worin LG2 für eine Gruppe HET gemäß obiger Definition steht und LG3 für einen austauschbaren Substituenten steht, mit einer Verbindung der Formel (IX) oder einem Analogon davon, das zur Bildung eines Substituenten T gemäß (TA1) mit Verknüpfung über ein sp²-Kohlenstoffatom oder (TA2) geeignet ist, oder einem bi- oder tricyclischen Analogon von (IX), das zur Bildung eines Substituenten T gemäß (TB) geeignet ist, kuppelt; 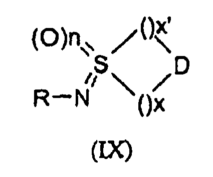 wobei n = 0 oder 1 und ()x und ()x' für Ketten der Länge x und x' stehen; D für NH oder CH=C-Lg4, worin Lg4 eine Abgangsgruppe bedeutet, steht; oder
(e)(ii) eine Verbindung der Formel (x): 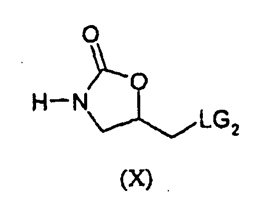 worin LG2 für eine Gruppe HET gemäß obiger Definition steht, mit einer Verbindung [Aryl]-LG4, worin LG4 für einen austauschbaren Substituenten steht, kuppelt; oder
(f) für HET als 1,2,3-Triazol eine Cycloaddition über das Azid (worin z.B. LG in (II) für Azid steht) durchführt; oder
(g) wenn HET für 4-substituiertes 1,2,3-Triazol steht, eine Verbindung der Formel (II) mit LG = NH₂ (primäres Amin) mit einer Verbindung der Formel (XI), nämlich dem Arensulfonylhydrazon eines Methylketons, das weiterhin an der Methylgruppe durch zwei Substituenten (Y' und Y''), die aus diesem als Ausgangsstoff verwendeten und als Zwischenprodukt gebildeten substituierten Hydrazon als HY' und HY" (oder als konjugierte Basen davon) eliminiert werden können, geminal substituiert ist, umsetzt; 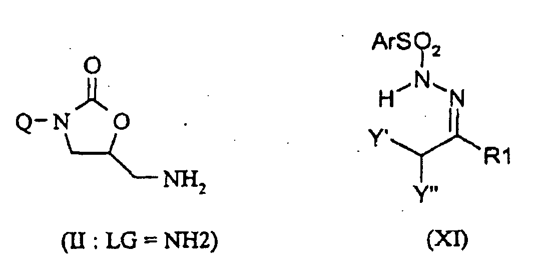
(h) eine nach Verfahren (e)(i) gebildete Verbindung, in der der Substituent T (gemäß (TA1)) über ein sp²-Kohlenstoffatom verknüpft ist, zu einem gesättigten Analogon reduziert;
und danach gegebenenfalls: (i) Schutzgruppen abspaltet; (ii) ein pharmazeutisch annehmbares Salz bildet; (iii) einen in vivo hydrolysierbaren Ester bildet.

## Revendications

1. Composé de formule (IA), ou un sel pharmaceutiquement acceptable, ou un ester hydrolysable in vivo de celui-ci 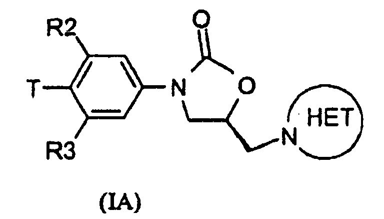 dans laquelle HET est le 1,2,3-triazole, le 1,2,4-triazole ou le tétrazole ; ou HET est une version di-hydro de la pyrimidine, de la pyridazine, de la pyrazine, de la 1,2,3-triazine, de la 1,2,4-triazine, de la 1,3,5-triazine et de la pyridine ;
dans laquelle HET est éventuellement substitué sur tout atome de C disponible, autre qu'un atome de C adjacent à l'atome de N liant, par un substituant choisi parmi un (1-4C)alkyle, un (2-4C)alcényle, un (3-6C)cycloalkyle, un amino, un (1-4C)-alkylamino, un di-(1-4C)alkylamino, un (1-4C)-alkylthio, un (1-4C)alcoxy, un (1-4C)-alcoxycarbonyle, un halogène, un cyano et un trifluorométhyle et/ou sur un atome d'azote disponible (à condition que le cycle ne soit pas ainsi quaternisé) par un (1-4C)alkyle; et dans laquelle à chaque apparition de substituants alkyle, alcényle et cycloalkyle de HET, chacun est éventuellement substitué par un ou plusieurs F, C1
ou CN ;
R² et R³ sont indépendamment l'hydrogène ou un fluoro ; et
T est choisi parmi (TA1), (TA2) et (TB1) à (TB3),
(TA) T est choisi parmi les groupes suivants (TA1) et (TA2) :- 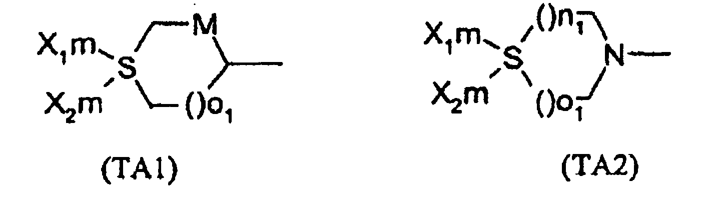 où :
dans (TA1), ()o₁ est 0 ou 1 et représente une chaîne d'atomes de carbone (éventuellement substitués comme défini pour AR1) O₁ de long et M est une liaison reliant les atomes de carbone adjacents, ou M représente un ou deux atomes de carbone, et définit un cycle monocyclique à 4 à 7 chaînons, lequel cycle peut éventuellement avoir l'un de
(i) une double liaison entre deux atomes de carbone du cycle quelconques ; ou
(ii) un pont C1-C3 reliant deux atomes de carbone du cycle quelconques non adjacents appropriés, lequel pont peut éventuellement contenir un hétéroatome choisi parmi l'oxygène ou >NRc ; ou
(iii) un motif cyclique C2-C5 comportant un atome de carbone du cycle pour définir un système de cycle spiro C2-C5, lequel cycle peut éventuellement contenir un hétéroatome choisi parmi l'oxygène ou >NRc ; ou
(iv) un pont C1-C4 reliant des atomes de carbone adjacents pour définir un cycle condensé, où un pont C2-C4 peut éventuellement contenir un hétéroatome choisi parmi l'oxygène ou >NRc ; où Rc est tel que défini ci-après ;
où dans (TA2), ()n₁ et ()o₁ sont indépendamment 0, 1 ou 2 et représentent des chaînes d'atomes de carbone (éventuellement substitués comme défini pour AR1) n₁ et o₁ de long respectivement, et définissent un cycle monocyclique à 4 à 8 chaînons, lequel cycle peut éventuellement avoir l' un de
(i) un pont C1-C3 reliant deux atomes de carbone du cycle quelconques non adjacents appropriés, lequel pont contient un hétéroatome choisi parmi l'oxygène ou >NRc ; ou
(ii) un motif cyclique C2-C5 comportant un atome de carbone du cycle pour définir un système de cycle spiro C2-C5, lequel cycle peut éventuellement contenir un hétéroatome choisi parmi l'oxygène ou >NRc ; ou
(iii) un pont C1-C4 reliant des atomes de carbone adjacents pour définir un cycle condensé, où un cycle C2-C4 peut éventuellement contenir un hétéroatome choisi parmi l'oxygène ou >NRc ; où Rc est tel que défini ci-après ; et
où dans (TA1) et (TA2), X₁ₘ et X₂ₘ pris ensemble représentent R₂ₛ-(E)ₘₛ-N=; ou X₁ₘ est O= et X₂ₘ est R₂ₛ-(E)ₘₛ-N-, et vice versa ;
où E est un groupe électroattracteur choisi parmi -SO₂-, -CO-, -O-CO-, -CO-O-, -CS-, -CON(Rₛ)-, -SO₂N(Rₛ)-, ou E peut représenter un groupe de formule R₃ₛ-C(=N-O-R₃ₛ)-C(=O)-, où R₃ₛ est H ou tel que défini dans R₂ₛ en (i) ci-dessous ;
ou lorsque E est -CON(Rₛ)- ou -SO₂N(Rₛ)-, R₂ₛ et Rₛ peuvent se lier ensemble pour former une chaîne carbonée qui définit un cycle saturé, insaturé ou partiellement insaturé à 5 ou 6 chaînons lié par l'intermédiaire de l'atome de N dans E, le cycle est éventuellement encore substitué par un substituant oxo, et lequel cycle peut être éventuellement condensé avec un groupe phényle pour former un système benzo- condensé, où le groupe phényle est éventuellement substitué par jusqu'à trois substituants choisis indépendamment parmi un halogéno, un cyano, un (1-4C) alkyle et un (1-4C)alcoxy ;
ms est 0 ou 1 ;
R₂ₛ et Rₛ sont choisis indépendamment parmi :
(i) l'hydrogène (sauf où E est -SO₂- ou -O-CO-), ou un (1-6C)alkyle {éventuellement substitué par un ou plusieurs groupes (1-4C)alcanoyle (dont une disubstitution géminée) et/ou éventuellement monosubstitué par un cyano, un cyano-imino, un (1-4C)alcoxy, un trifluorométhyle, un (1-4C)alcoxycarbonyle, un phényle (éventuellement substitué comme défini pour AR1 dans la présente), un groupe hétéroaryle éventuellement substitué de formule AR2, AR2a, AR2b, AR3, AR3a, AR3b, AR4, AR4a ou CY tous comme définis (et éventuellement substitués comme défini) dans la présente, un (1-4C)alkyl-S(O)_{q}- (q est 0, 1 ou 2) ; et/ou (à condition que lorsque R₂ₛ est -SO₂- ou -O-CO- pas sur le premier atome de carbone de la chaîne (1-6C)alkyle) éventuellement substitué par un ou plusieurs groupes (dont une disubstitution géminée) choisi chacun indépendamment parmi un hydroxy et un fluoro et/ou éventuellement encore substitué par pas plus de un de chacun parmi un oxo, NRvRw- [où Rv est l'hydrogène ou un (1-4C)alkyle ; Rw est l'hydrogène ou un (1-4C)alkyle], un (1-6C)alcanoylamino, un (1-4C)alcoxycarbonylamino, un N-(1-4C)alkyl-N-(1-6C)alcanoylamino, un (1-4C)alkylS(O)ₚNH- ou un (1-4C)alkylS(O)ₚ-((1-4C)alkyl)N- (p est 1 ou 2)}; ou
(ii) un groupe aryle éventuellement substitué ou hétéroaryle éventuellement substitué de formule AR1, AR2, AR2a, AR2b, AR3, AR3a, AR3b, AR4, AR4a ou CY tous comme défini (et éventuellement substitués comme défini) dans la présente ;
ou (où ms est 0 uniquement) ;
(iii) un cyano, -CO-NRvRw, -CO-NRvRw', -SO₂-NRvRw, -SO₂-NRvRw' [où Rv est l'hydrogène ou un (1-4C)alkyle ; Rw est l'hydrogène ou un (1-4C)alkyle ; Rw' est un phényle (éventuellement substitué comme défini pour AR1 dans la présente), ou un groupe hétéroaryle choisi parmi AR2, AR2a, AR2b, AR3, AR3a, AR3b, AR4, AR4a (éventuellement substitués comme défini dans la présente)], un (1-4C)alcoxycarbonyle, un trifluorométhyle, un éthényle, un 2-(1-4C)alkyléthényle, un 2-cyanoéthényle, un 2-cyano-2-((1-4C)alkyl)éthényl, un 2-nitroéthényle, un 2-nitro-2-((1-4C)alkyl)éthényle, un 2-((1-4C)alkylaminocarbonyl)éthényle, un 2-((1-4C)-alcoxycarbonyl)éthényle, un 2-(AR1)éthényle, un 2-(AR2)éthényle, ou un 2-(AR2a)éthényle ; ou
(TB) T est choisi parmi les groupes suivants (TB1) à (TB3) :- 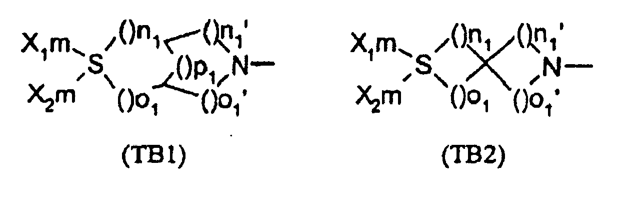 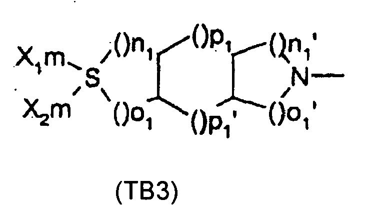 où :
X₁ₘ et X₂ₘ pris ensemble représentent R₂ₛ-(E)ₘₛ-N= ; ou X₁ₘ est O= et X₂ₘ est R₂ₛ-(E)ₘₛ-N-, et vice versa ;
où E est un groupe électroattracteur choisi parmi -SO₂-, -CO-, -O-CO-, -CO-O-, -CS-, -CON(Rₛ)-, -SO₂N(Rₛ)-, ou E peut représenter un groupe de formule R₃ₛ-C(=N-O-R₃ₛ)-C(=O)-, où R₃ₛ est H ou tel que défini dans R₂ₛ en (i) ci-dessous ;
ou lorsque E est -CON(Rₛ)- ou -SO₂N(Rₛ)-, R₂ₛ et Rₛ peuvent se lier ensemble pour former une chaîne carbonée qui définit un cycle saturé, insaturé ou partiellement insaturé à 5 ou 6 chaînons lié par l'intermédiaire de l'atome de N dans E, lequel cycle est éventuellement encore substitué par un substituant oxo, et lequel cycle peut être éventuellement condensé avec un groupe phényle pour former un système benzo- condensé, où le groupe phényle est éventuellement substitué par jusqu'à trois substituants choisis indépendamment parmi un halogéno, un cyano, un (1-4C) alkyle et un (1-4C)alcoxy ;
ms est 0 ou 1 ;
R₂ₛ et Rₛ sont choisis indépendamment parmi :
(i) l'hydrogène (sauf où E est -SO₂- ou -O-CO-), ou un (1-6C)alkyle {éventuellement substitué par un ou plusieurs groupes (1-4C)alcanoyle (dont une disubstitution géminée) et/ou éventuellement monosubstitué par un cyano, un cyano-imino, un (1-4C)alcoxy, un trifluorométhyle, un (1-4C)alcoxycarbonyle, un phényle (éventuellement substitué comme défini pour AR1), un groupe hétéroaryle éventuellement substitué de formule AR2, AR2a, AR2b, AR3, AR3a, AR3b, AR4, AR4a ou CY tous comme définis ci-après, un (1-4C)alkyl-S(O)_{q-}(q est 0, 1 ou 2) ; et/ou (à condition que lorsque R₂ₛ est -SO₂- ou -O-CO- pas sur le premier atome de carbone de la chaîne (1-6C)alkyle) éventuellement substitué par un ou plusieurs groupes (dont une disubstitution géminée) choisi chacun indépendamment parmi un hydroxy et un fluoro et/ou éventuellement encore substitué par pas plus de un de chacun parmi un oxo, NRvRw- [où Rv est l'hydrogène ou un (1-4C)alkyle ; Rw est l'hydrogène ou un (1-4C)alkyle], un (1-6C)alcanoylamino, un (1-4C)alcoxycarbonylamino, un N-(1-4C)alkyl-N-(1-6C)alcanoylamino, un (1-4C)alkylS(O)ₚNH- ou un (1-4C)alkylS(O)ₚ-((1-4C)alkyl)N- (p est 1 ou 2)} ; ou
(ii) un groupe aryle éventuellement substitué ou hétéroaryle éventuellement substitué de formule AR1, AR2, AR2a, AR2b, AR3, AR3a, AR3b, AR4, AR4a ou CY tous comme défini dans la présente ;
ou (où ms est 0 uniquement) ;
(iii) un cyano, -CO-NRvRw, -CO-NRvRw', -SO₂-NRvRw, -SO₂-NRvRw'[où Rv est l'hydrogène ou un (1-4C)alkyle ; Rw est l'hydrogène ou un (1-4C)alkyle ; Rw' est un phényle (éventuellement substitué comme défini pour AR1), ou un groupe hétéroaryle choisi parmi AR2, AR2a, AR2b, AR3, AR3a, AR3b, AR4, AR4a (tels que définis dans la présente)], un (1-4C)alcoxycarbonyle, un trifluorométhyle, un éthényle, un 2-(1-4C)alkyléthényle, un 2-cyanoéthényle, un 2-cyano-2-((1-4C)alkyl)éthényl, un 2-nitroéthényle, un 2-nitro-2-((1-4C)alkyl)éthényle, un 2-((1-4C)alkylaminocarbonyl)éthényle, un 2-((1-4C)-alcoxycarbonyl)éthényle, un 2-(AR1)éthényle, un 2-(AR2)éthényle, ou un 2-(AR2a)éthényle ; et
où ()n₁, ()o₁, () n_{1'}, ()o_{1'}, ()p₁ et ()p_{1'} représentent des chaînes d'atomes de carbone (éventuellement substituées comme pour AR1) n₁, o₁, n_{1'} o_{1'} p₁ et p_{1'} de long respectivement, et sont indépendamment 0-2, à condition que dans (TB1) et (TB2) la somme de n₁, o₁, n_{1'} et o_{1'} ne dépasse pas 8 (donnant une taille maximum de cycle de 14 dans (TB1) et 11 dans (TB2)), et dans (TB3) la somme de n₁, o₁, n_{1'}, o_{1'}, p₁ et p_{1'} ne dépasse pas 6 (donnant une taille maximum de cycle de 12) ;
où Rc est choisi parmi les groupes (Rc1) à (Rc5) :-
*(Rc1)* (1-6C)alkyle éventuellement substitué ;
*(Rc2)* R¹³CO-, R¹³SO₂- ou R¹³CS-,
où R¹³ est choisi parmi (Rc2a) à (Rc2e) :-
*(Rc2a)* AR1, AR2, AR2a, AR2b, AR3, AR3a, AR3b, AR4, AR4a, CY ;
*(Rc2b)* l'hydrogène, un (1-4C)alcoxycarbonyle, un trifluorométhyle, -NrvRw [où Rv est l'hydrogène ou un (1-4C)alkyle ; Rw est l'hydrogène ou un (1-4C)alkyle], un éthényle, un 2-(1-4C)-alkyléthényle, un 2-cyanoéthényle, un 2-cyano-2-((1-4C)alkyl)éthényle, un 2-nitroéthényle, un 2-nitro-2-((1-4C)alkyl)éthényle, un 2-((1-4C)alkylaminocarbonyl)éthényle,
un 2-((1-4C)alcoxycarbonyl)éthényle, un 2-(AR1)-éthényle, un 2-(AR2)éthényle, un 2-(AR2a)-éthényle ;
*(Rc2c)* un (1-10C)alkyle éventuellement substitué ;
*(Rc2d)* un R¹⁴C(O)O(1-6C)alkyle où R¹⁴ est AR1, AR2, un (1-4C)alkylamino (le groupe (1-4C)alkyle étant éventuellement substitué par un (1-4C)-alcoxycarbonyle ou par un carboxy), un benzyloxy-(1-4C)alkyle ou un (1-10C)alkyle {éventuellement substitué comme défini pour (Rc2c)} ;
*(Rc2e)* R¹⁵O- où R¹⁵ est un benzyle, un (1-6C) alkyle {éventuellement substitué comme défini pour (Rc2c)}, CY, ou AR2b ;
(*Rc3*) l'hydrogène, un cyano, un 2-cyanoéthényle, un 2-cyano-2-((1-4C)alkyl)éthényle, un 2-((1-4C)-alkylaminocarbonyl)éthényle, un 2-((1-4C)alcoxy-carbonyl)éthényle, un 2-nitroéthényle, un 2-nitro-2-((1-4C)alkyl)éthényle, un 2-(AR1)éthényle, un 2-(AR2)éthényle, ou de formule (Rc3a) 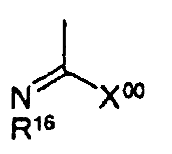 dans laquelle X⁰⁰ est -OR¹⁷, -SR¹⁷, -NHR¹⁷ et -N(R¹⁷)₂ ;
où R¹⁷ est l'hydrogène (lorsque X⁰⁰ est -NHR¹⁷ et -N(R¹⁷)₂), et R¹⁷ est un (1-4C) alkyle, un phényle ou AR2 (lorsque X⁰⁰ est -OR¹⁷, -SR¹⁷ et -NHR¹⁷) ; et R¹⁶ est un cyano, un nitro, un (1-4C)alkylsulfonyle, un (4-7C)cycloalkylsulfonyle, un phénylsulfonyle, un (1-4C)alcanoyle et un (1-4C)alcoxycarbonyle ;
(Rc4) un trityle, AR1, AR2, AR2a, AR2b, AR3, AR3a, AR3b ;
*(Rc5)* RdOC (Re) =CH (C=O) -, RfC (=O) C (=O) -, RgN=C(Rh)C=O- ou RiNHC (Rj)=CHC(=O)- où Rd est un (1-6C)alkyle ; Re est l'hydrogène ou un (1-6C)alkyle, ou Rd et Re forment ensemble une chaîne (3-4C)alkylène ; Rf est l'hydrogène, un (1-6C)alkyle, un hydroxy(1-6C)alkyle, un (1-6C)alcoxy(1-6C)alkyle ; -NRvRw [où Rv est l'hydrogène ou un (1-4C)alkyle ; Rw est l'hydrogène ou un (1-4C)alkyle], un (1-6C)alcoxy, un (1-6C)alcoxy(1-6C)alcoxy, un hydroxy-(2-6C)alcoxy, un (1-4C) alkylamino (2-6C) alcoxy, un di- (1-4C) alkylamino (2-6C) alcoxy ; Rg est un (1-6C)alkyle, un hydroxy ou un (1-6C)alcoxy ; Rh est l'hydrogène ou un (1-6C)alkyle ; Ri est l'hydrogène, un (1-6C)alkyle, AR1, AR2, AR2a, AR2b et Rj est l'hydrogène ou un (1-6C)alkyle ;
où
CY est un cycle cyclobutyle, cyclopentyle, cyclohexyle, cyclopentényle ou cyclohexényle éventuellement substitué ;
**AR1** est un phényle éventuellement substitué ou un naphtyle éventuellement substitué ;
**AR2** est un cycle hétéroaryle monocyclique à 5 ou 6 chaînons totalement insaturé (c'est-à-dire avec le degré d'insaturation maximum) éventuellement substitué, contenant jusqu'à quatre hétéroatomes choisis indépendamment parmi O, N et S (mais ne contenant pas de liaisons O-O, O-S ou S-S) et lié par un atome de carbone du cycle, ou un atome d'azote du cycle si le cycle n'est pas ainsi quaternisé ;
**AR2a** est une version partiellement hydrogénée d'AR2 (c'est-à-dire les systèmes AR2 conservant un certain degré, mais pas la totalité du degré d'insaturation), liée par un atome de carbone du cycle ou liée par un atome d'azote du cycle si le cycle n'est pas ainsi quaternisé ;
**AR2b** est une version totalement hydrogénée d'AR2 (c'est-à-dire des systèmes AR2 n'ayant pas d'insaturation), lié par un atome de carbone du cycle ou lié par un atome d'azote du cycle ;
**AR3** est un cycle hétéroaryle bicyclique à 8, 9 ou 10 chaînons totalement insaturé (c'est-à-dire avec le degré d'insaturation maximum) éventuellement substitué, contenant jusqu'à quatre hétéroatomes choisis indépendamment parmi O, N et S (mais ne contenant pas de liaisons O-O, O-S ou S-S), et lié par un atome de carbone du cycle dans l'un ou l'autre des cycles comprenant le système bicyclique ;
**AR3a** est une version partiellement hydrogénée d'AR3 (c'est-à-dire les systèmes AR3 conservant un certain degré, mais pas la totalité du degré d'insaturation) lié par un atome de carbone du cycle ou lié par un atome d'azote du cycle si le cycle n'est pas ainsi quaternisé, dans l'un ou l'autre des cycles comprenant le système bicyclique ;
**AR3b** est une version totalement hydrogénée d'AR3 (c'est-à-dire des systèmes AR3 n'ayant pas d'insaturation), lié par un atome de carbone du cycle ou lié par un atome d'azote du cycle, dans l'un ou l'autre des cycles comprenant le système bicyclique ;
**AR4** est un cycle hétéroaryle tricyclique à 13 ou 14 chaînons totalement insaturé (c'est-à-dire avec le degré d'insaturation maximum) éventuellement substitué, contenant jusqu'à quatre hétéroatomes choisis indépendamment parmi O, N et S (mais ne contenant pas de liaisons O-O, O-S ou S-S quelconques), et lié par un atome de carbone du cycle dans l'un quelconque des cycles comprenant le système tricycligue ;
**AR4a** est une version partiellement hydrogénée d'AR4 (c'est-à-dire les systèmes AR4a conservant un certain degré mais pas la totalité du degré d'insaturation), lié par un atome de carbone du cycle ou lié par un atome d'azote du cycle si le cycle n'est pas ainsi quaternisé, dans l'un quelconque des cycles comprenant le système tricyclique ;
où les substituants sur AR1, AR2, AR2a, AR2b, AR3, AR3a, AR3b, AR4, AR4a et CY sont (sur un atome de carbone disponible) jusqu'à trois substituants choisis indépendamment parmi un (1-4C)alkyle {éventuellement substitué par des (de préférence un) substituants choisis indépendamment parmi un hydroxy, un trifluorométhyle, un (1-4C)alkylS(O)_{q-}(q est 0, 1 ou 2) (ce dernier substituant de préférence sur AR1 uniquement), un (1-4C)alcoxy, un (1-4C)alcoxycarbonyle, un cyano, un nitro, un (1-4C)alcanoylamino, -CONRvRw ou -NRvRw}, un trifluorométhyle, un hydroxy, un halogéno, un nitro, un cyano, un thiol, un (1-4C)alcoxy, (1-4C)alcanoyloxy, un diméthyl-aminométhylèneaminocarbonyle, un di(N-(1-4C)-alkyl)aminométhylimino, un carboxy, un (1-4C)-alcoxycarbonyle, un (1-4C) alcanoyle, un (1-4C)-alkylSO₂amino, un (2-4C)alcénylé {éventuellement substitué par un carboxy ou un (1-4C)alcoxycarbonyle}, un (2-4C)alcynyle, un (1-4C)alcanoylamino, un oxo(=O), un thioxo(=S), un (1-4C)alcanoylamino {le groupe (1-4C)alcanoyle étant éventuellement substitué par un hydroxy}, un (1-4C)alkylS(O)_{q-} (q est 0, 1 ou 2) {le groupe (1-4C)alkyle étant éventuellement substitué par un ou plusieurs groupes choisis indépendamment parmi un cyano, un hydroxy et un (1-4C) alcoxy} , -CONRvRw ou -NRvRw [où Rv est l'hydrogène ou un (1-4C)alkyle ; Rw est l'hydrogène ou un (1-4C)alkyle] ; et où d'autres substituants convenables sur AR1, AR2, AR2a, AR2b, AR3, AR3a, AR3b, AR4, AR4a, et CY (sur un atome de carbone disponible), et également sur des groupes alkyle (sauf indication contraire) sont jusqu'à trois substituants choisis indépendamment parmi un trifluorométhoxy, un benzoylamino, un benzoyle, un phényle {éventuellement substitué par jusqu'à trois substituants choisis indépendamment parmi un halogéno, un (1-4C)alcoxy ou un cyano} , le furane, le pyrrole, le pyrazole, l'imidazole, le triazole, la pyrimidine, la pyridazine, la pyridine, l'isoxazole, l'oxazole, l'isothiazole, le thiazole, le thiophène, un hydroxyimino-(1-4C)alkyle, un (1-4C)alcoxyimino(1-4C)alkyle, un halogéno-(1-4C)alkyle, un (1-4C)alcanesulfonamido, -SO₂NRvRw [où Rv est l'hydrogène ou un (1-4C)alkyle ; Rw est l'hydrogène ou un (1-4C)alkyle] .

2. Composé selon la revendication 1 de formule (IA), ou un sel pharmaceutiquement acceptable, ou un ester hydrolysable in vivo de celui-ci, dans laquelle HET est le 1,2,3-triazole, le 1,2,4-triazole ou le tétrazole ;
R² et R³ sont indépendamment l'hydrogène ou un fluoro ; et
T est choisi parmi (TA1a & b), (TA2a) et (TB1a & b).

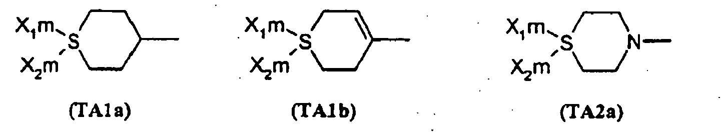

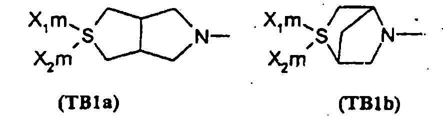

3. Composé de formule (IA) selon la revendication 2, ou un sel pharmaceutiquement acceptable, ou un ester hydrolysable in vivo de celui-ci, dans laquelle T est choisi parmi (TA1a & b) et (TA2a).

4. Composé selon l'une quelconque des revendications précédentes, ou un sel pharmaceutiquement acceptable, ou un ester hydrolysable in vivo de celui-ci, **caractérisé en ce que** HET est substitué par un méthyle (éventuellement substitué par un ou plusieurs F, C1 ou CN) ;

5. Composé selon l'une quelconque des revendications précédentes, ou un sel pharmaceutiquement acceptable, ou un ester hydrolysable in vivo de celui-ci, **caractérisé en ce que** HET est non substitué.

6. Composé selon l'une quelconque des revendications précédentes, ou un sel pharmaceutiquement acceptable ou un ester hydrolysable in vivo de celui-ci, **caractérisé en ce que** X₁ₘ est O= et X₂ₘ est R₂ₛ-(E)ₘₛ-N-, et vice versa;
et lorsque ms est 0, R₂ₛ est choisi parmi
(i) l'hydrogène, un groupe (1-6C)alkyle {éventuellement monosubstitué par un groupe (1-4C)alcanoyle, un cyano, un cyano-imino, un (1-4C)alcoxy, un trifluorométhyle, un (1-4C)alcoxycarbonyle, un phényle (éventuellement substitué comme pour AR1 défini dans la présente), un groupe hétéroaryle éventuellement substitué de formule AR2, AR2a, AR2b, AR3, AR3a, AR3b, AR4, AR4a ou CY tous comme définis (et éventuellement substitués comme défini) dans la présente, un (1-4C)alkylS(O)_{q}- (q est 0, 1 ou 2) ; ou éventuellement substitué par un ou plusieurs groupes fluoro (dont une disubstitution géminée) ; ou éventuellement substitué par un ou plusieurs groupes hydroxy (à l'exclusion d'une disubstitution géminée), et/ou éventuellement encore substitué par pas plus de un de chacun parmi un oxo, -NRvRw [où Rv est l'hydrogène ou un (1-4C)alkyle ; Rw est l'hydrogène ou un (1-4C)alkyle], un (1-6C) alcanoylamino, un (1-4C)alcoxycarbonylamino, un N-(1-4C)alkyl-N-(1-6C)alcanoylamino, un (1-4C)alkylS(O)ₚNH- ou un (1-4C)alkylS(O)ₚ₋((1-4C)alkyl)N- (p est 1 ou 2)} ; ou
(ii) un groupe aryle éventuellement substitué ou hétéroaryle éventuellement substitué de formule AR1, AR2, AR2a, AR2b, AR3, AR3a, AR3b, AR4, AR4a ou CY tous comme définis (et éventuellement substitués comme défini) dans la présente ; ou
(iii) un cyano, -CO-NRvRw, -CO-NRv Rw', -SO₂NRvRw, -SO₂-NRv Rw' [où Rv est l'hydrogène ou un (1-4C)alkyle ; Rw est l'hydrogène ou un (1-4C)alkyle ; Rw' est un phényle (éventuellement substitué comme pour AR1 défini dans la présente), ou un groupe hétéroaryle choisi parmi AR2, AR2a, AR2b, AR3, AR3a, AR3b, AR4, AR4a (éventuellement substitué comme défini dans la présente)], un (1-4C)alcoxycarbonyle, un trifluorométhyle ;
et lorsque ms est 1, E est -CO- ou -SO₂- et R₂ₛ est choisi parmi :
(i) un (1-6C)alkyle {éventuellement monosubstitué par un cyano, un cyano-imino, un (1-4C)alcoxy, un trifluorométhyle, un (1-4C)alcoxycarbonyle, un phényle (éventuellement substitué comme pour AR1 défini dans la présente), un groupe hétéroaryle éventuellement substitué de formule AR2, AR2a, AR2b, AR3, AR3a, AR3b, AR4, AR4a ou CY tous comme définis (et éventuellement substitués comme défini) dans la présente, un (1-4C)alkylS(O)_{q}-(q est 0, 1 ou 2) ; et/ou (à condition que lorsque R₂ₛ est -SO₂- ou -O-CO- pas sur le premier atome de carbone de la chaîne (1-6C)alkyle) éventuellement substitué par un ou plusieurs groupes (dont une disubstitution géminée) choisis chacun indépendamment parmi un hydroxy et un fluoro, et/ou éventuellement monosubstitué par -NRvRw [où Rv est l'hydrogène ou un (1-4C)alkyle ; Rw est l'hydrogène ou un (1-4C) alkyle], un (1-6C)alcanoylamino, un (1-4C)alcoxycarbonylamino, un N-(1-4C)alkyl-N-(1-6C)alcanoylamino, un (1-4C)alkylS(O)pNH- ou un (1-4C)alkylS(O)ₚ₋((1-4C)alkyl)N- (p est 1 ou 2)} ; ou
(ii) un groupe aryle ou hétéroaryle éventuellement substitué de formule AR1, AR2, AR2a, AR2b, AR3, AR3a, AR3b, AR4, AR4a ou CY tous comme définis (et éventuellement substitués comme défini) dans la présente.

7. Composé selon l'une quelconque des revendications précédentes ou un sel pharmaceutiquement acceptable ou un ester hydrolysable in vivo de celui-ci, **caractérisé en ce que** X₁ₘ est O= et X₂ₘ est R₂ₛ-(E)ₘₛ-N-, et vice versa ; et lorsque ms est 0, R₂ₛ est choisi parmi
(i) l'hydrogène, un (1-6C)alkyle {éventuellement monosubstitué par un (1-4C)alcoxy, un trifluorométhyle, un (1-4C) alkylS (O) q- (q est 0, 1 ou 2) ; ou éventuellement substitué par un ou plusieurs groupes fluoro (dont une disubstitution géminée) ; ou éventuellement substitué par un ou plusieurs groupes hydroxy (à l'exclusion d'une disubstitution géminée)} ; ou
(ii) -CO-NRvRw [où Rv est l'hydrogène ou un (1-4C)alkyle ; Rw est l'hydrogène ou un (1-4C)alkyle], -CO-NRv Rw' [où Rv est l'hydrogène ou un (1-4C)alkyle ; Rw' est un phényle (éventuellement substitué comme pour AR1 défini dans la présente)], un (1-4C)alcoxycarbonyle ;
et lorsque ms est 1, E est -CO- ou -SO₂- et R₂ₛ est choisi parmi :
(i) un (1-6C)alkyle {éventuellement monosubstitué par un (1-4C)alcoxy, un trifluorométhyle, un (1-4C)alkylS(O)_{q}- (q est 0, 1 ou 2) ; ou éventuellement substitué par un ou plusieurs groupes fluoro (dont une disubstitution géminée) ; ou éventuellement substitué par un ou plusieurs groupes hydroxy (à l'exclusion d'une disubstitution géminée)}, un (1-6C)alcanoylamino, un (1-4C)alcoxycarbonylamino.

8. Composé selon l'une quelconque des revendications précédentes ou un sel pharmaceutiquement acceptable ou un ester hydrolysable in vivo de celui-ci, **caractérisé en ce que** Rc est R¹³CO- et R¹³ est un (1-4C)alcoxycarbonyle, un hydroxy-(1-4C)alkyle, un (1-4C)alkyle (éventuellement substitué par un ou deux groupes hydroxy, ou par un groupe (1-4C)alcanoyle), un (1-4C)alkylamino, un diméthylamino(1-4C)alkyle, un (1-4C)alcoxyméthyle, un (1-4C)alcanoylméthyle, un (1-4C) alcanoyloxy (1-4C) alkyle, un (1-5C)alcoxy ou un 2-cyanoéthyle.

9. Composé selon l'une quelconque des revendications précédentes ou un sel pharmaceutiquement acceptable ou un ester hydrolysable in vivo de celui-ci, **caractérisé en ce que** Rc est R¹³CO- et R¹³ est un 1,2-dihydroxyéthyle, un 1,3-dihydroxyprop-2-yle, un 1,2,3-trihydroxyprop-1-yle, un méthoxycarbonyle, un hydroxyméthyle, un méthyle, un méthylamino, un diméthylaminométhyle, un méthoxyméthyle, un acétoxyméthyle, un méthoxy, un méthylthio, un naphtyle, un tert-butoxy ou un 2-cyanoéthyle.

10. Composé de formule (IA) selon l'une quelconque des revendications précédentes, ou un sel pharmaceutiquement acceptable ou un ester hydrolysable in vivo de celui-ci, destiné à être utilisé comme médicament.

11. Utilisation d'un composé de formule (IA) selon l'une quelconque des revendications précédentes, ou d'un sel pharmaceutiquement acceptable, ou d'un ester hydrolysable in vivo de celui-ci, dans la fabrication d'un médicament destiné à être utilisé dans la production d'un effet antibactérien chez un animal à sang chaud.

12. Composition pharmaceutique comprenant un composé de formule (IA) selon l'une quelconque des revendications précédentes, ou un sel pharmaceutiquement acceptable ou un ester hydrolysable in vivo de celui-ci, et un diluant ou un support pharmaceutiquement acceptable.

13. Procédé de fabrication d'un composé selon l'une quelconque des revendications précédentes, et des sels pharmaceutiquement acceptables et des esters hydrolysables in vivo de celui-ci, selon un procédé (a) à (h) comme suit (où les variables sont telles que définies ci-dessus sauf indication contraire) ; Q est le phénylène substitué par R² et R³ :
(a) par modification d'un substituant dans ou introduction d'un substituant dans un autre composé de formule (I) ; ou
(b) par réaction d'un composé de formule (II) : 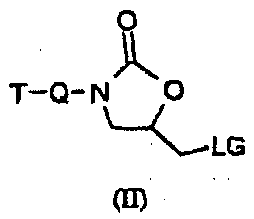 dans laquelle LG est un groupe déplaçable, avec un composé de formule (III):
**HET (III)**
dans laquelle HET est la forme base libre HET-H ou l'anion HET- formé à partir de la forme base libre ; ou
(c) par réaction d'un composé de formule (IV):
**T-Q-LG1 (IV)**
dans laquelle LG1 est un groupe isocyanate, amine
ou uréthane avec un époxyde de formule (V) ; ou avec un composé apparenté de formule (VA) où le groupe hydroxy sur l'atome de C interne est protégé de manière classique et où le groupe partant Y sur l'atome de C terminal est un groupe partant classique ; ou 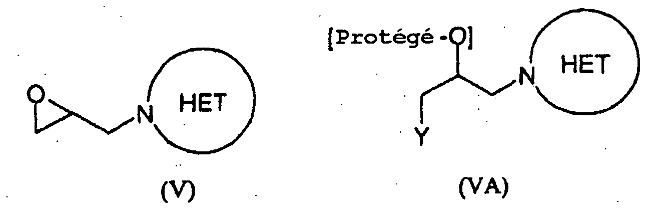
(d) par oxydation
(i) avec un agent d'amination d'un composé de soufre de plus faible valence (VI), ou un analogue de celui-ci, qui est convenable pour donner un substituant T comme défini par (TA2), ou un analogue de cycle tricyclique de (VI) qui est convenable pour donner un substituant T comme défini par (TB) ; ou
(ii) avec un agent d'oxygénation d'un composé de soufre de plus faible valence (VII) ou un analogue de celui-ci, qui est convenable pour donner un substituant T comme défini par (TA2), ou un analogue de cycle bi- ou tricyclique de (VII) qui est convenable pour donner un substituant T comme défini par (TB) ; 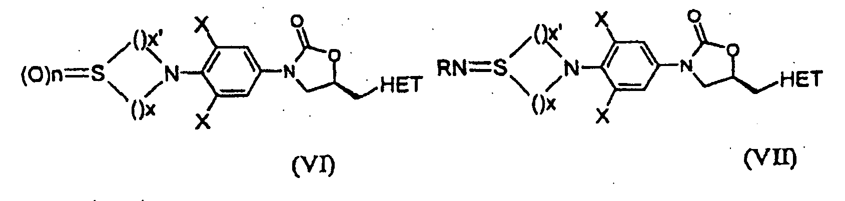
où n = 0 ou 1 est ()x et () x' sont des chaînes x et x' de long ; ou
(e) (i) par couplage d'un composé de formule (VIII) : 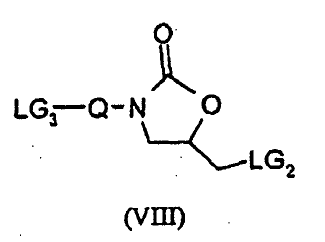 dans laquelle LG2 est un groupe HET tel que défini précédemment et LG3 est un substituant remplaçable, avec un composé de formule (IX), ou un analogue de celui-ci, qui est convenable pour donner un substituant T tel que défini par (TA1),
dans lequel le lien est par l'intermédiaire d'un atome de carbone sp², ou (TA2), ou un analogue de cycle bi- ou tricyclique de (IX) qui est convenable pour donner un substituant T tel que défini par (TB) ; 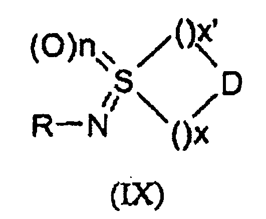 où n = 0 ou 1 et ()x et ()x' sont des chaînes x et x' de long ; D est NH ou CH=C-Lg4 où Lg4 est un groupe partant ; ou
(e) (ii) par couplage d'un composé de formule (X) : 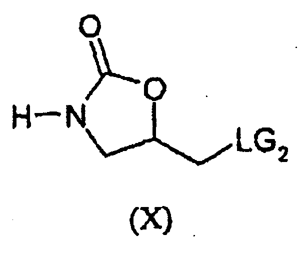 dans laquelle LG2 est un groupe HET tel que défini précédemment, avec un composé [aryl]-LG4, où LG4 est un substituant remplaçable ; ou
(f) pour HET en tant que 1,2,3-triazole par cycloaddition par l'intermédiaire de l'azide (où par exemple LG dans (II) est un azide) ; ou
(g) Lorsque HET le 1,2,3-triazole substitué en position 4, par réaction d'un composé de formule (II) où LG=NH₂ (amine primaire) avec un composé de formule (XI), à savoir l'arènesulfonylhydrazone d'une méthylcétone qui est encore substituée géminalement sur le groupe méthyle par deux substituants (Y' et Y'') susceptibles d'être éliminés de ces hydrazones substituées de départ et intermédiaires sous forme de HY' et de HY'' (ou sous forme de ses bases conjuguées) ; 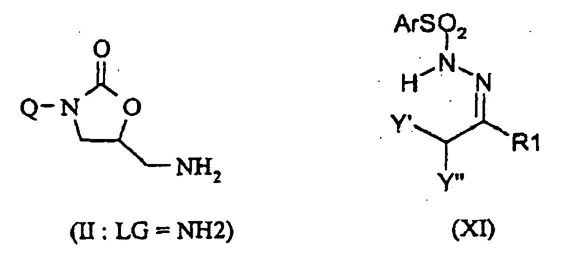
(h) par réduction d'un composé formé par le procédé (e) (i) dans lequel le substituant T (tel que défini par (TA1)) est lié par un atome de carbone sp², pour former l'analogue saturé ;
puis, le cas échéant : (i) l'élimination de tout groupe protecteur ; (ii) la formation d'un sel pharmaceutiquement acceptable ; (iii) la formation d'un ester hydrolysable in vivo.
